# EUROPEAN PATENT APPLICATION

(11) **EP 1 348 443 A1**
(43) Date of publication of application: **01.10.2003**
(21) Application number: 01999263.5
(22) Date of filing: 07.12.2001
(51) Int. Cl.: A61K 45/06, A61K 31/18, A61K 31/216, A61K 31/275, A61K 31/428, A61K 31/35, A61P 31/04, A61P 29/00, C07D 309/28

(54) **COMBINATION DRUGS**

(30) Priority: 08.12.2000 JP 2000379787
(71) Applicant: Takeda Chemical Industries, Ltd., Osaka-shi, Osaka 541-0045 (JP)
(72) Inventor: IIZAWA, Yuji, Muko-shi, Kyoto 617-0002 (JP); II, Masayuki, Minoh-shi, Osaka 562-0005 (JP); HASHIGUCHI, Shohei, Toyonaka-shi, Osaka 561-0881 (JP); KITAZAKI, Tomoyuki, Kobe-shi, Hyogo 651-1221 (JP)
(74) Representative: Rickard, Timothy Mark Adrian
(86) International application number: JP0110773
(87) International publication number: WO02045750

(57) **Abstract**

The present invention relates to a pharmaceutical agent containing an anti-sepsis drug (e.g., cycloalkene compound), and at least one kind of drug selected from the group consisting of an antibacterial agent, an antifungal agent, a non-steroidal antiinflammatory drug, a steroid and an anticoagulant in combination.

## Description

### Technical Field

The present invention relates to a combination drug comprising a cycloalkene compound useful as an anti-sepsis drug and the like and a treatment method of sepsis and the like.

### Background Art

WO 99/46242 describes that (i) a compound represented by the formula: 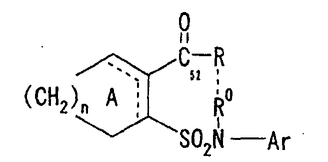 wherein R represents an aliphatic hydrocarbon group optionally having substituents, an aromatic hydrocarbon group optionally having substituents, a heterocyclic group optionally having substituents, a group represented by the formula: -OR¹ (wherein R¹ represents a hydrogen atom or an aliphatic hydrocarbon group optionally having substituents) or a group represented by the formula: 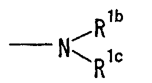 (wherein R^{1b} represents a hydrogen atom or an aliphatic hydrocarbon group optionally having substituents, and R^{1c} is, the same as or different from R^{1b}, a hydrogen atom or an aliphatic hydrocarbon group optionally having substituents), R⁰ represents a hydrogen atom or an aliphatic hydrocarbon group, or R¹ and R⁰ represent a bond with each other, ring A is a cycloalkene substituted by 1 to 4 substituents selected from (1) an aliphatic hydrocarbon group optionally having substituents, (2) an aromatic hydrocarbon group optionally having substituents, (3) a group represented by the formula: -OR¹¹ (wherein R¹¹ represents a hydrogen atom or an aliphatic hydrocarbon group optionally having substituents and (4) a halogen atom,
Ar represents an aromatic hydrocarbon group optionally having substituents,
a group represented by the formula: 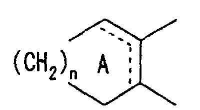 represents a group represented by the formula: 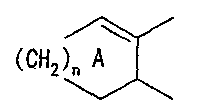 or 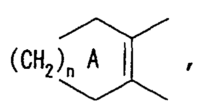 and n is an integer of 1 to 4, and
(ii) a compound represented by the formula: 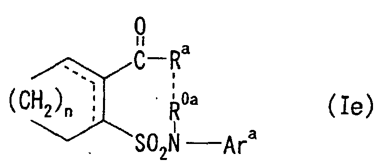 wherein R^{a} represents an aliphatic hydrocarbon group optionally having substituents, an aromatic hydrocarbon group optionally having substituents, a heterocyclic group optionally having substituents, a group represented by the formula: -OR^{1a} (wherein R^{1a} represents a hydrogen atom or an aliphatic hydrocarbon group optionally having substituents) or a group represented by the formula: 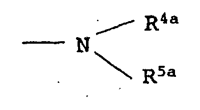 (wherein R^{4a} and R^{5a} are the same or different and each is a hydrogen atom or an aliphatic hydrocarbon group optionally having substituents,
R^{0a} represents a hydrogen atom or an aliphatic hydrocarbon group, or R^{a} and R^{0a} represent a bond with each other,
Ar^{a} represents an aromatic hydrocarbon group optionally having substituents,
a group represented by the formula: 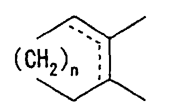 represents a group represented by the formula: 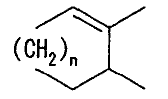 or 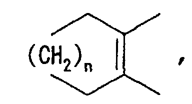 and n represents an integer of 1 to 4, a salt thereof and a prodrug thereof have a nitric oxide (NO) production-inhibiting effect and an inhibitory effect on the production of inflammatory cytokines, such as TNF-α, IL-1, IL-6 and the like, and are useful as a prophylactic and therapeutic agent against the diseases including cardiac diseases, autoimmune diseases, inflammatory diseases, central nervous system diseases, infectious diseases, sepsis, septic shock and the like.

### Brief Description of the Drawings

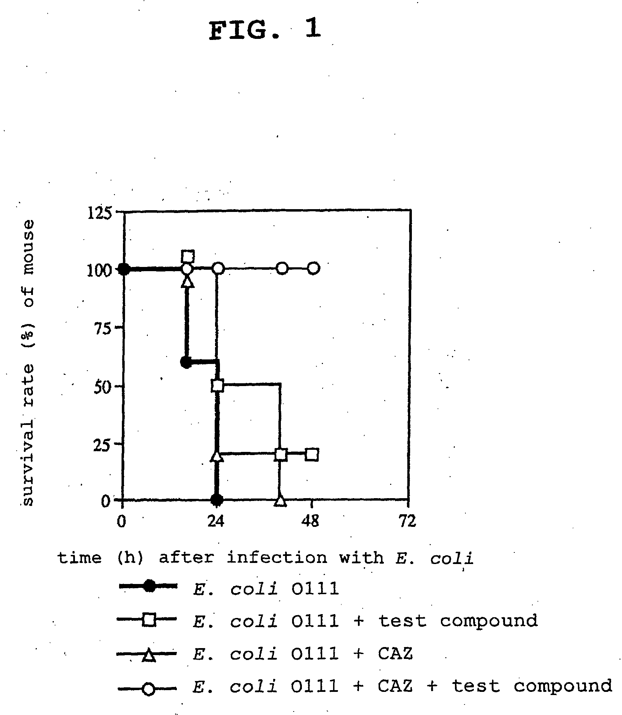

Fig. 1: the transverse axis shows the time after infection with *E. coli* 0111, the vertical axis shows the survival rate (Survival (%)) of mice, CAZ shows ceftazidime, the test compound is compound 29 of Reference Example B65, ● indicates the results of no administration to mouse infected with *E. coli* 0111, □ indicates the results of administration of compound 29 of Reference Example B65 to mouse infected with *E. coli* 0111, Δ indicates the results of administration of CAZ to mouse infected with *E. coli* 0111, and . indicates the results of administration of compound 29 of Reference Example B65 and CAZ to mouse infected with *E. coli* 0111.

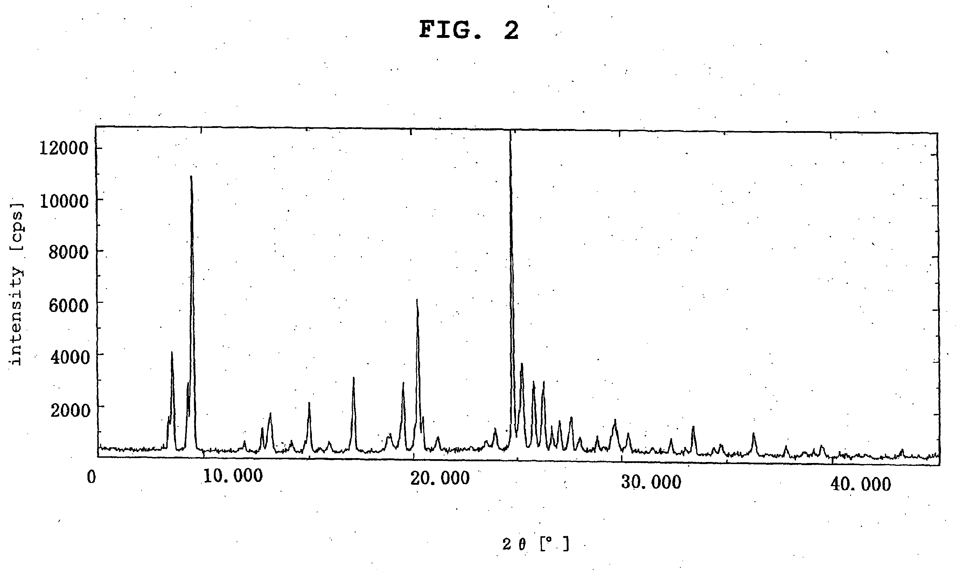

Fig. 2 shows a powder X-ray diffraction pattern of the crystal obtained in Reference Example F2.

### Disclosure of the Invention

The present invention aims at provision of a combination drug of a cycloalkene compound useful as an anti-sepsis drug and the like, and a treatment method of sepsis and the like.

As a result of the intensive studies done by the present inventors in view of the above-mentioned problems, it was found that a combined use of the above-mentioned cycloalkene compound with an antibacterial agent, an antifungal agent, a non-steroidal antiinflammatory drug, a steroid, an anticoagulant and the like affords effective treatment of the diseases such as sepsis and the like. Further studies made by the present inventors based on said finding resulted in the completion of the present invention.

Accordingly, the present invention relates to
[1] a pharmaceutical agent comprising an anti-sepsis drug and at least one kind of drug selected from the group consisting of an antibacterial agent, an antifungal agent, a non-steroidal antiinflammatory drug, a steroid and an anticoagulant in combination,
[2] the pharmaceutical agent of the above-mentioned [1], wherein the anti-sepsis drug is a non-peptidic compound,
[3] the pharmaceutical agent of the above-mentioned [1], wherein the anti-sepsis drug is a cycloalkene compound,
[4] the pharmaceutical agent of the above-mentioned [1], wherein the anti-sepsis drug is a compound represented by the formula (I): 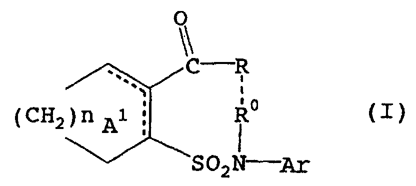 wherein
   - R: is an aliphatic hydrocarbon group optionally having substituents, an aromatic hydrocarbon group optionally having substituents, a heterocyclic group optionally having substituents, a group represented by the formula: -OR¹ wherein R¹ is a hydrogen atom or an aliphatic hydrocarbon group optionally having substituents, or a group represented by the formula: 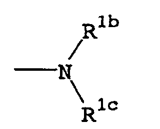 wherein
   R^{1b} and R^{1c} are the same or different and each is a hydrogen atom or an aliphatic hydrocarbon group optionally having substituents,
   - R⁰: is a hydrogen atom or an aliphatic hydrocarbon group, or R and R⁰ are linked to each other to form a bond,
   - ring A¹: is a cycloalkene optionally substituted by 1 to 4 substituents selected from the group consisting of
   (1) an aliphatic hydrocarbon group optionally having substituents,
   (2) an aromatic hydrocarbon group optionally having substituents,
   (3) a group represented by the formula: -OR¹¹ wherein R¹¹ is a hydrogen atom or an aliphatic hydrocarbon group optionally having substituents and
   (4) a halogen atom,
   - Ar: is an aromatic hydrocarbon group optionally having substituents,
   a group represented by the formula: 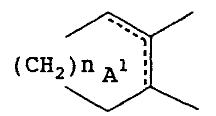 is a group represented by the formula: 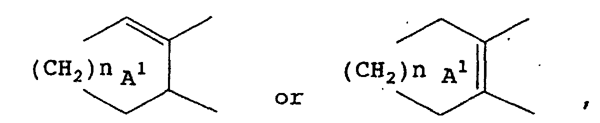 and
   - n: is an integer of 1 to 4,
   or a salt thereof or a prodrug thereof,
[5] the pharmaceutical agent of the above-mentioned [4], wherein, in the formula (I),
   R is a group represented by the formula: -OR¹ wherein R¹ is as defined in the above-mentioned [4],
   R⁰ is a hydrogen atom or an aliphatic hydrocarbon group, ring A¹ is an unsubstituted cyclohexene,
   Ar is an aromatic hydrocarbon group optionally having substituents, and
   n is 2,
[6] the pharmaceutical agent of the above-mentioned [1], wherein the anti-sepsis drug is a compound represented by the formula (II): 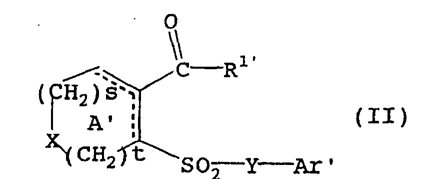 wherein R^{1'} is an aliphatic hydrocarbon group optionally having substituents, an aromatic hydrocarbon group optionally having substituents, a heterocyclic group optionally having substituents, a group represented by the formula: -OR^{1a'} wherein R^{1a'} is a hydrogen atom or an aliphatic hydrocarbon group optionally having substituents, or a group represented by the formula: 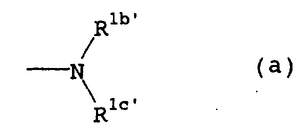 wherein R^{1b'} and R^{1c'} are the same or different and each is a hydrogen atom or an aliphatic hydrocarbon group optionally having substituents,
   X is a methylene group, NH, a sulfur atom or an oxygen atom,
   Y is a methylene group optionally having substituents or NH optionally having substituents,
   ring A' is a 5- to 8-membered ring optionally having 1 to 4 substituents selected from the group consisting of (1) an aliphatic hydrocarbon group optionally having substituents, (2) an aromatic hydrocarbon group optionally having substituents, (3) a group represented by the formula: -OR^{2'} wherein R^{2'} is a hydrogen atom or an aliphatic hydrocarbon group optionally having substituents and (4) a halogen atom,
   Ar' is an aromatic hydrocarbon group optionally having substituents,
   a group represented by the formula: 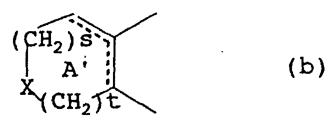 is a group represented by the formula: 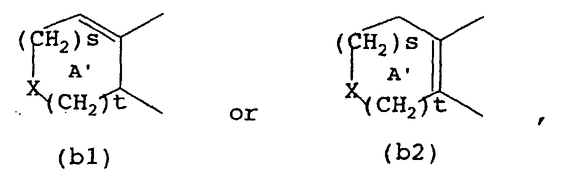 s is an integer of 0 to 2,
   t is an integer of 1 to 3, and
   the total of s and t is not more than 4;
   provided that when X is a methylene group, Y is a methylene group optionally having substituents, or a salt thereof or a prodrug thereof,
[7] the pharmaceutical agent of the above-mentioned [6], wherein, in the formula (II),
   R^{1'} is a group represented by the formula: -OR^{1a'} wherein R^{1a'} is C₁₋₆ alkyl,
   X is a methylene group or an oxygen atom,
   Y is a methylene group or NH,
   Ar' is a phenyl group optionally having 1 or 2 substituents selected from the group consisting of halogen atoms and a C₁₋₆ alkoxy group,
   a group represented by the formula: 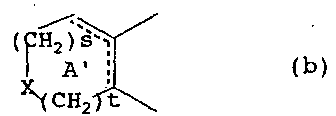 is a group represented by the formula: 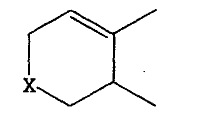 s is 1, and
   t is 1,
[8] the pharmaceutical agent of the above-mentioned [1], comprising an anti-sepsis drug and one or more kinds of drugs selected from an antibacterial agent and an antifungal agent in combination,
[9] the pharmaceutical agent of the above-mentioned [1], comprising an anti-sepsis drug and one or more kinds of drugs selected from a non-steroidal antiinflammatory drug and steroid in combination,
[10] the pharmaceutical agent of the above-mentioned [1], comprising an anticoagulant and an anti-sepsis drug in combination,
[11] the pharmaceutical agent of the above-mentioned [1] or [4], which is a prophylactic or therapeutic agent of sepsis,
[12] the pharmaceutical agent of the above-mentioned [1] or [4], which is a prophylactic or therapeutic agent of septic shock,
[13] the pharmaceutical agent of the above-mentioned [1] or [4], which is a prophylactic or therapeutic agent of an inflammatory disease or an infectious disease,
[14] a method for the prophylaxis or treatment of sepsis, which comprises administration of an effective amount of an anti-sepsis drug and an effective amount of at least one kind of drug selected from an antibacterial agent, an antifungal agent, a non-steroidal antiinflammatory drug, a steroid and an anticoagulant in combination to a mammal,
[15] a method for the prophylaxis or treatment of an inflammatory disease or an infectious disease, which comprises administration of an effective amount of an anti-sepsis drug and an effective amount of at least one kind of drug selected from the group consisting of an antibacterial agent, an antifungal agent, a non-steroidal antiinflammatory drug, a steroid and an anticoagulant in combination to a mammal,
[16] use of an anti-sepsis drug and at least one kind of drug selected from the group consisting of an antibacterial agent, an antifungal agent, a non-steroidal antiinflammatory drug, a steroid and an anticoagulant for the production of a prophylactic or therapeutic agent of sepsis, and
[17] use of an anti-sepsis drug and at least one kind of drug selected from the group consisting of an antibacterial agent, an antifungal agent, a non-steroidal antiinflammatory drug, a steroid and an anticoagulant for the production of a prophylactic or therapeutic agent of an inflammatory disease or an infectious disease.

Moreover, the present invention provides
[1] a pharmaceutical agent comprising compound (I) represented by the formula (I); 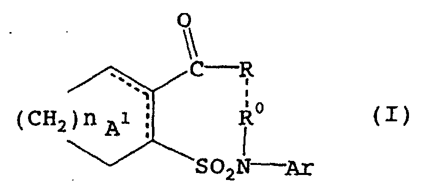 wherein
   - R: is an aliphatic hydrocarbon group optionally having substituents, an aromatic hydrocarbon group optionally having substituents, a heterocyclic group optionally having substituents, a group represented by the formula: -OR¹ wherein R¹ is a hydrogen atom or an aliphatic hydrocarbon group optionally having substituents, or a group represented by the formula: 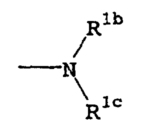 wherein
   R^{1b} and R^{1c} are the same or different and each is a hydrogen atom or an aliphatic hydrocarbon group optionally having substituents,
   - R⁰: is a hydrogen atom or an aliphatic hydrocarbon group, or R and R⁰ in combination form a bond,
   - ring A¹: is a cycloalkene optionally substituted by 1 to 4 substituents selected from the group consisting of
   (1) an aliphatic hydrocarbon group optionally having substituents,
   (2) an aromatic hydrocarbon group optionally having substituents,
   (3) a group represented by the formula: -OR¹¹ wherein R¹¹ is a hydrogen atom or an aliphatic hydrocarbon group optionally having substituents and
   (4) a halogen atom,
   - Ar: is an aromatic hydrocarbon group optionally having substituents,
   a group represented by the formula: 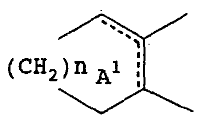 is a group represented by the formula: 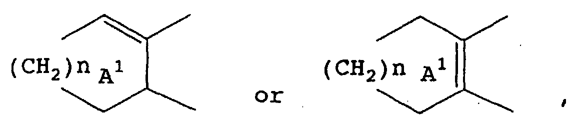 and
   - n: is an integer of 1 to 4,
   or a salt thereof or a prodrug thereof, and one or more kinds of drugs selected from an antibacterial agent, an antifungal agent, a non-steroidal antiinflammatory drug, a steroid and an anticoagulant in combination,

[2] a pharmaceutical agent comprising the above-mentioned compound (I) or a salt thereof or a prodrug thereof, and one or more kinds of drugs selected from an antibacterial agent and an antifungal agent in combination,
[3] a pharmaceutical agent comprising the above-mentioned compound (I) or a salt thereof or a prodrug thereof, and one or more kinds of drugs selected from a non-steroidal antiinflammatory drug and a steroid in combination,
[4] the pharmaceutical agent of [1], which is a prophylactic or therapeutic agent of sepsis or septic shock,
[5] the pharmaceutical agent of [1], which is a prophylactic or therapeutic agent of an inflammatory disease or an infectious disease,
[6] a method for the prophylaxis or treatment of sepsis or septic shock, which comprises administration of an effective amount of the above-mentioned compound (I) or a salt thereof or a prodrug thereof, and an effective amount of one or more kinds of drugs selected from an antibacterial agent, an antifungal agent, a non-steroidal antiinflammatory drug, a steroid and an anticoagulant in combination to a mammal,
[7] a method for the prophylaxis or treatment of an inflammatory disease or an infectious disease, which comprises administration of an effective amount of the above-mentioned compound (I) or a salt thereof or a prodrug thereof and an effective amount of one or more kinds of drugs selected from an antibacterial agent, an antifungal agent, a non-steroidal antiinflammatory drug, a steroid and an anticoagulant in combination to a mammal,
[8] the pharmaceutical agent of [1], wherein the compound represented by the formula (I) is a compound represented by (i) the formula: 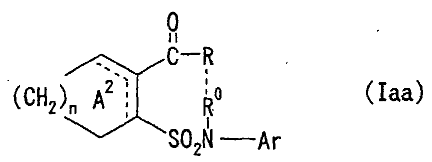 wherein
   - R: is an aliphatic hydrocarbon group optionally having substituents, an aromatic hydrocarbon group optionally having substituents, a heterocyclic group optionally having substituents, a group represented by the formula: -OR¹ wherein R¹ is a hydrogen atom or an aliphatic hydrocarbon group optionally having substituents, or a group represented by the formula: 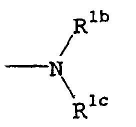 wherein
   R^{1b} is a hydrogen atom or an aliphatic hydrocarbon group optionally having substituents,
   R^{1c} is the same as or different from R^{1b} and is a hydrogen atom or an aliphatic hydrocarbon group optionally having substituents,
   - R⁰: is a hydrogen atom or an aliphatic hydrocarbon group, or R and R⁰ are linked to each other to form a bond,
   - ring A²: is a cycloalkene substituted by 1 to 4 substituents selected from
   (1) an aliphatic hydrocarbon group optionally having substituents,
   (2) an aromatic hydrocarbon group optionally having substituents,
   (3) a group represented by the formula: -OR¹¹ wherein R¹¹ is a hydrogen atom or an aliphatic hydrocarbon group optionally having substituents and
   (4) a halogen atom,
   - Ar: is an aromatic hydrocarbon group optionally having substituents,
   a group represented by the formula: 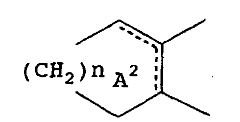 is a group represented by the formula: 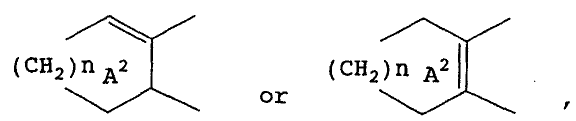 and
   - n: is an integer of 1 to 4, or
   (ii) the formula: 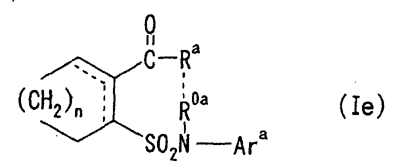 wherein
   - R^{a}: is an aliphatic hydrocarbon group optionally having substituents, an aromatic hydrocarbon group optionally having substituents, a heterocyclic group optionally having substituents, a group represented by the formula: -OR^{1a} wherein R^{1a} is a hydrogen atom or an aliphatic hydrocarbon group optionally having substituents, or a group represented by the formula: 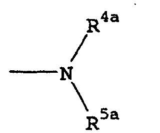 wherein
   R^{4b} and R^{5a} are the same or different and each is a hydrogen atom or an aliphatic hydrocarbon group optionally having substituents,
   - R^{0a}: is a hydrogen atom or an aliphatic hydrocarbon group, or R^{a} and R^{0a} in combination form a bond,
   - Ar^{a}: is an aromatic hydrocarbon group optionally having substituents,
   a group represented by the formula: 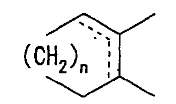 is a group represented by the formula: 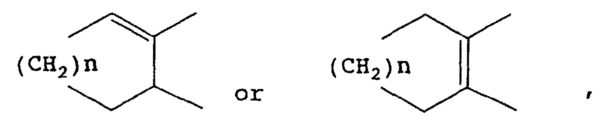 and
   - n: is an integer of 1 to 4,
[9] the pharmaceutical agent of [8], wherein the compound represented by the formula (Iaa) is a compound of the formula: 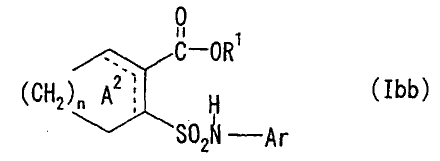 wherein each symbol is as defined in [8],
[10] the pharmaceutical agent of [8], wherein
   ring A² is cycloalkene substituted by a lower alkyl, a phenyl or a halogen,
   R¹ is a lower alkyl group,
   Ar is a phenyl group optionally having substituents, and
   n is 2,
[11] the pharmaceutical agent of [8], wherein the compound represented by the formula (Ie) is a compound represented by the formula: 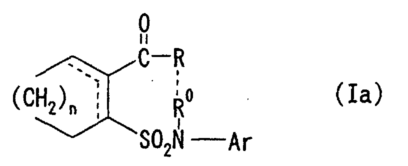 wherein
   - R: is an aliphatic hydrocarbon group optionally having substituents, an aromatic hydrocarbon group optionally having substituents, a heterocyclic group optionally having substituents, a group represented by the formula: -OR¹ wherein R¹ is a hydrogen atom or an aliphatic hydrocarbon group optionally having substituents, or a group represented by the formula: 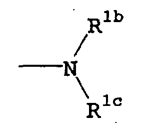 wherein
   R^{1b} is a hydrogen atom or an aliphatic hydrocarbon group optionally having substituents, and
   R^{1c} is the same as or different from R^{1b} and is a hydrogen atom or an aliphatic hydrocarbon group optionally having substituents,
   - R⁰: is a hydrogen atom or an aliphatic hydrocarbon group, or R and R⁰ in combination form a bond,
   - Ar: is an aromatic hydrocarbon group optionally having substituents,
   a group represented by the formula: 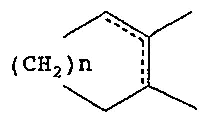 is a group represented by the formula: 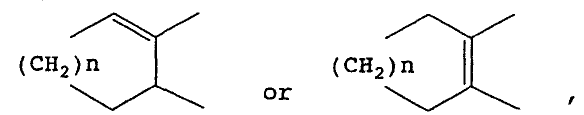 and
   - n: is an integer of 1 to 4,
[12] the pharmaceutical agent of [11], wherein the compound represented by the formula (Ia) is a compound represented by the formula: 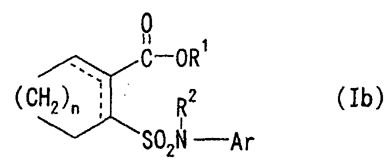 wherein R² is a hydrogen atom or an aliphatic hydrocarbon group, and R¹, Ar, n and a group represented by the formula: 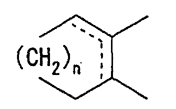 are as defined in [11],
[13] the pharmaceutical agent of [12], wherein R¹ is a lower alkyl group optionally having substituents,
[14] the pharmaceutical agent of [12], wherein R¹ is an ethyl group,
[15] the pharmaceutical agent of [12], wherein R² is a hydrogen atom or a lower alkyl group,
[16] the pharmaceutical agent of [12], wherein R² is a hydrogen atom,
[17] the pharmaceutical agent of [12], wherein Ar is a phenyl group optionally having substituents,
[18] the pharmaceutical agent of [12], wherein Ar is a phenyl group substituted by a halogen and/or a lower alkyl,
[19] the pharmaceutical agent of [12], wherein Ar is a group represented by the formula: 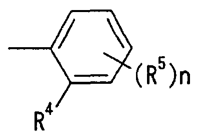 wherein R⁴ and R⁵ are the same or different and each is a halogen atom or a lower alkyl group and n is an integer of 0-2,
[20] the pharmaceutical agent of [18] or [19], wherein the halogen atom is a fluorine atom or a chlorine atom,
[21] the pharmaceutical agent of [12], wherein the group represented by the formula: 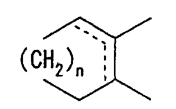 is a group represented by the formula: 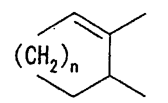 wherein n is as defined in [12],
[22] the pharmaceutical agent of [12], wherein n is 1-3,
[23] the pharmaceutical agent of [12], wherein R¹ is a lower alkyl group optionally having substituents, R² is a hydrogen atom or a lower alkyl group, Ar is a phenyl group optionally having substituents, and n is 1, 2 or 3,
[24] the pharmaceutical agent of [12], wherein R¹ is a lower alkyl group optionally having substituents, R² is a hydrogen atom, Ar is a phenyl group substituted by a halogen atom, and n is 2,
[25] the pharmaceutical agent of [11], wherein the compound represented by the formula (Ia) is a compound represented by the formula: 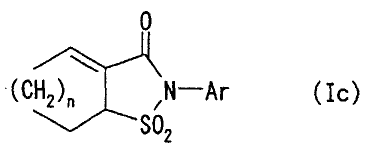 wherein Ar and n are as defined in [11].
[26] the pharmaceutical agent of [25], wherein Ar is a phenyl group optionally having substituents, and n is 2,
[27] the pharmaceutical agent of [11], wherein the compound represented by the formula (Ia) is a compound represented by the formula: 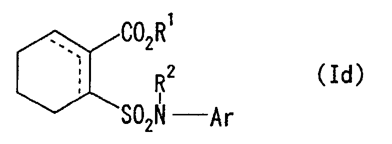 wherein R¹, R² and Ar are as defined in [12], and the group represented by the formula: 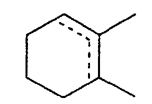 is a group represented by the formula: 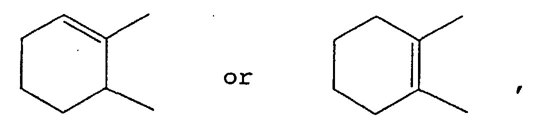 provided that when Ar is a phenyl group, R¹ is a hydrogen atom or an ethyl group, R² is a methyl group, and the group represented by the formula: 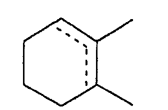 is a group represented by the formula: 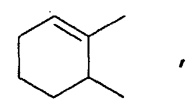
[28] the pharmaceutical agent of [8], wherein the compound represented by the formula (Ie) is a compound represented by the formula: 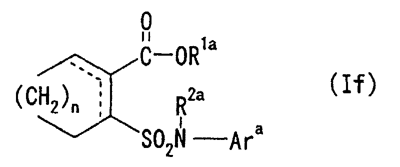 wherein R^{2a} is a hydrogen atom or an aliphatic hydrocarbon group, and R^{1a}, Ar^{a}, n and the group represented by the formula: 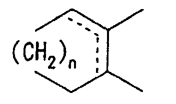 are as defined in [8],
[29] the pharmaceutical agent of [8], wherein the compound represented by the formula (Ie) is a compound represented by the formula: 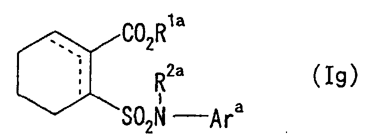 wherein R^{1a}, R^{2a} and Ar^{a} are as defined in [28], and the group represented by the formula: 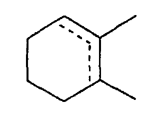 is a group represented by the formula: 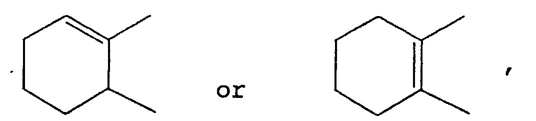
[30] the pharmaceutical agent of [1], wherein the compound is (i) ethyl (6R)-6-[(2-chloro-4-fluoroanilino)sulfonyl]-1-cyclohexene-1-carboxylate, (ii) d-ethyl 6-[N-(2,4-difluorophenyl)sulfamoyl]-1-cyclohexene-1-carboxylate, (iii) ethyl 6-[N-(2-chlorophenyl)sulfamoyl]-1-cyclohexene-1-carboxylate, or (iiii) ethyl 6-[N-(2-chloro-4-methylphenyl)sulfamoyl]-1-cyclohexene-1-carboxylate, or a salt thereof,
[31] the pharmaceutical agent of [1], which is a nitric oxide and/or cytokine production suppressant, and
[32] the pharmaceutical agent of [1], which is a prophylactic or therapeutic agent of a cardiac disease or an autoimmune disease.

As the anti-sepsis drug to be used for the pharmaceutical agent of the present invention, peptidic compounds such as rBPI-21 (bactericidal permeability increasing protein), BI-51017 (antithrombin III), SC-59735 (rTFPI), r-PAF acetylhydrase, LY-203638 (r-activated protein C), anti-TNF-α antibody and the like, and non-peptidic compounds such as JTE-607, E-5531, E-5564, S-5920, FR-167653, ONO-1714, ONO-5046 (sivelestat), GW-273629, RWJ-67657, cycloalkene compound and the like are used. Of these, non-peptidic compounds such as cycloalkene compounds and the like are preferable, and cycloalkene compounds are particularly preferable. As the cycloalkene compounds, the above-mentioned compounds represented by the formula (I) and the formula (II), salts thereof and prodrugs thereof are preferable.

The above-mentioned compounds are explained in detail.

In the specification, R represents an aliphatic hydrocarbon group optionally having substituents, an aromatic hydrocarbon group optionally having substituents, a heterocyclic group optionally having substituents, a group represented by the formula: -OR¹ (wherein R¹ represents a hydrogen atom or an aliphatic hydrocarbon group optionally having substituents) or a group represented by the formula: 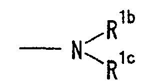 wherein R^{1b} and R^{1c} are the same or different and each is a hydrogen atom or an aliphatic hydrocarbon group optionally having substituents, or R and R⁰ in combination form a bond, with particular preference given to the group represented by the formula: -OR¹ (wherein R¹ is as defined above).

R^{a} represents an aliphatic hydrocarbon group optionally having substituents, an aromatic hydrocarbon group optionally having substituents, a heterocyclic group optionally having substituents, a group represented by the formula: -OR^{1a} (wherein R^{1a} represents a hydrogen atom or an aliphatic hydrocarbon group optionally having substituents) or a group represented by the formula: 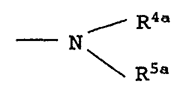 (wherein R^{4a} and R^{5a} are the same or different and each is a hydrogen atom or an aliphatic hydrocarbon group optionally having substituents), or R^{a} and R^{0a} in combination form a bond, with particular preference given to the group represented by the formula: -OR^{1a} (wherein R^{1a} is as defined above).

When R and R⁰ in combination form a bond, the compound represented by the formula (Iaa) can be represented by the formula: 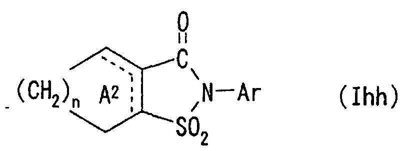 wherein each symbol is as defined above, and specifically can be represented by the formula: 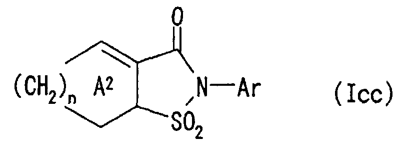 wherein each symbol is as defined above, or 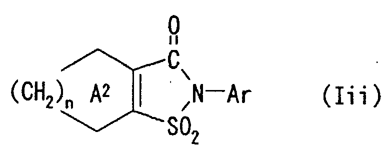 wherein each symbol is as defined above.

When R and R⁰ in combination form a bond, the compound represented by the formula (Ia) can be represented by the formula: 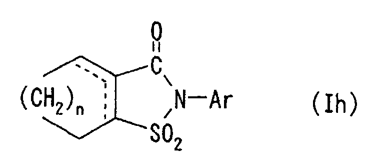 wherein each symbol is as defined above, and specifically can be represented by the formula: 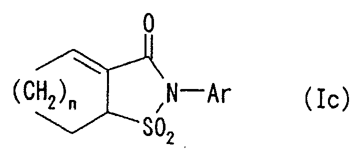 wherein each symbol is as defined above, or the formula: 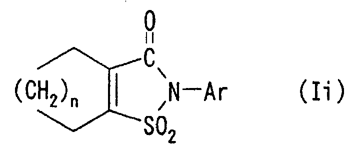 wherein each symbol is as defined above.

When R^{a} and R^{0a} in combination form a bond, the compound represented by the formula (Ie) can be represented by the formula: 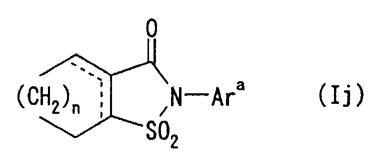 wherein each symbol is as defined above, and specifically can be represented by the formula: 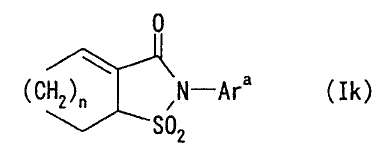 wherein each symbol is as defined above, or the formula: 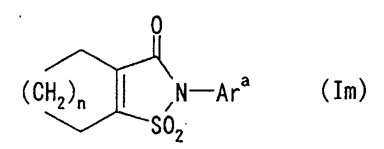 wherein each symbol is as defined above.

When R is a group represented by the formula: -OR¹ (wherein R¹ is as defined above), the compound represented by the formula (Iaa) can be represented by the formula: 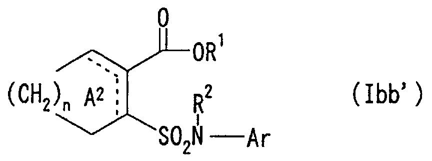 wherein each symbol is as defined above, and specifically can be represented by the formula: 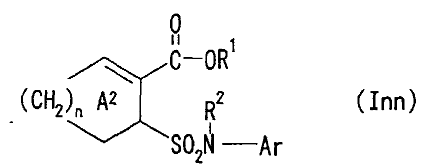 wherein each symbol is as defined above, or the formula: 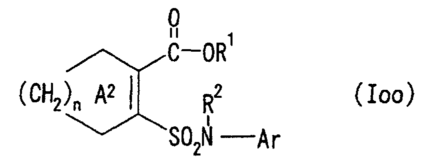 wherein each symbol is as defined above.

When R is a group represented by the formula: -OR¹ (wherein R¹ is as defined above), the compound represented by the formula (Ia) can be represented by the formula: 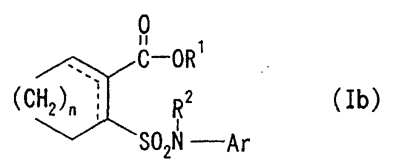 wherein each symbol is as defined above, and specifically can be represented by the formula: 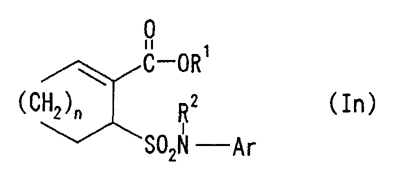 wherein each symbol is as defined above, or the formula: 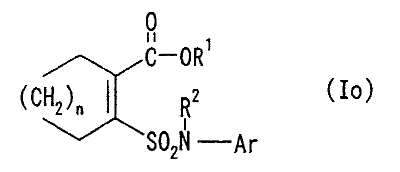 wherein each symbol is as defined above.

When R^{a} is a group represented by the formula: -OR^{1a} (wherein R^{1a} is as defined above), the compound represented by the formula (Ie) can be represented by the formula: 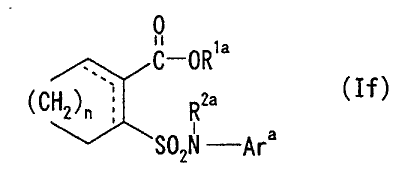 wherein each symbol is as defined above, and specifically can be represented by the formula: 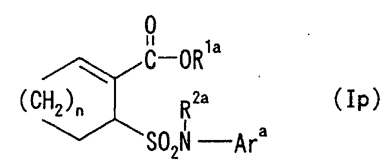 wherein each symbol is as defined above, or the formula: 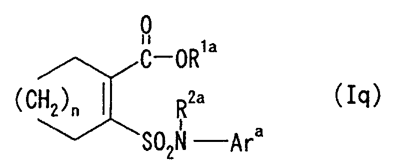 wherein each symbol is as defined above.

As the compound represented by the formula (Iaa), a compound represented by the formula (Icc) or the formula (Inn) is preferable, as the compound represented by the formula (Ia), a compound represented by the formula (Ic) or the formula (In) is preferable, and as the compound represented by the formula (Ie), a compound represented by the formula (Ik) or the formula (Ip) is preferable.

Similarly, the compound represented by the formula (Id) can be represented by the formula: 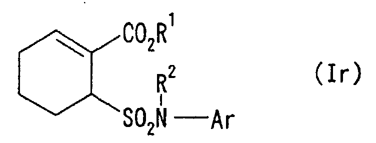 wherein each symbol is as defined above, or the formula: 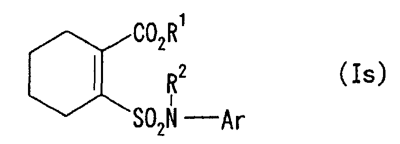 wherein each symbol is as defined above, and the compound represented by the formula (Ig) can be represented by the formula: 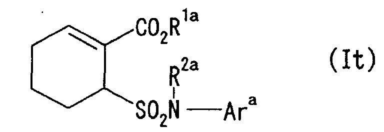 wherein each symbol is as defined above, or the formula: 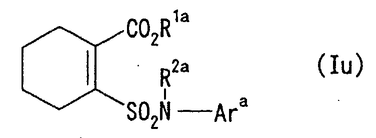 wherein each symbol is as defined above.

As the compound represented by the formula (Id), a compound represented by the formula (Ir) is preferable, and as the compound represented by the formula (Ig), a compound represented by the formula (It) is preferable.

When, in the compounds represented by the formula (Ia), n is 1-4, (i) R¹ is a hydrogen atom or a lower alkyl group optionally having substituents, R⁰ is a lower alkyl group optionally having substituents, and Ar is a phenyl group optionally having substituents, or (ii) R¹ and R⁰ in combination form a bond, and Ar is a phenyl group optionally having substituents, a group represented by the formula: 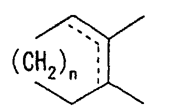 is preferably a group represented by the formula:

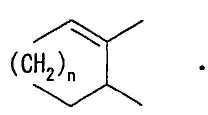

When, in the compound represented by the formula (Ib), n is 1-4, R¹ is a hydrogen atom or a lower alkyl group optionally having substituents, R⁰ is a lower alkyl group optionally having substituents, and Ar is a phenyl group optionally having substituents, a group represented by the formula: 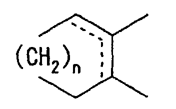 is preferably a group represented by the formula:

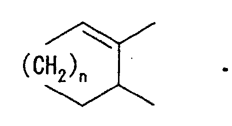

As the "aliphatic hydrocarbon group" of the "aliphatic hydrocarbon group optionally having substituents" represented by R, R¹, R¹¹, R^{1a}, R^{1b}, R^{1c}, R^{4a} and R^{5a}, and the "aliphatic hydrocarbon group" represented by R⁰, R^{0a}, R² and R^{2a}, for example, an alkyl group, a cycloalkyl group, a cycloalkylalkyl group, an alkenyl group, an alkynyl group, etc. are preferable.

As the alkyl group, for example, a linear or branched alkyl group having 1 to 20 carbon atoms (e.g., a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, a dodecyl group, etc.) and the like are preferable, and particularly, for example, a lower alkyl group having 1 to 6 carbon atoms (e.g., a methyl group, an ethyl group, an n-propyl group, an isopropyl group, an n-butyl group, an isobutyl group, a sec-butyl group, a tert-butyl group, etc.) and the like are preferable.

As the cycloalkyl group, for example, a cycloalkyl group having 3 to 10 carbon atoms (e.g., a cyclopropyl group, a cyclobutyl group, a cyclopentyl group, a cyclohexyl group, a cycloheptyl group, a cyclooctyl group, etc.) and the like are preferable, and particularly, for example, a cycloalkyl group having 3 to 6 carbon atoms (e.g., a cyclopropyl group, a cyclobutyl group, a cyclopentyl group, a cyclohexyl group, etc.) and the like are preferable.

As the cycloalkylalkyl group, for example, a cycloalkylalkyl group having 4 to 12 carbon atoms (e.g., a cyclopropylmethyl group, a cyclopentylmethyl group, a cyclohexylmethyl group, a cycloheptylmethyl group, etc.) and the like are preferable, and particularly, for example, a cycloalkylalkyl group having 4 to 8 (particularly 4 to 7) carbon atoms (e.g., a cyclopropylmethyl group, a cyclopentylmethyl group, a cyclohexylmethyl group, etc.) and the like are preferable.

As the alkenyl group, for example, a lower alkenyl group having 3 to 6 carbon atoms (e.g., a propenyl group, a butenyl group, a pentenyl group, etc.) and the like are preferable, and particularly, for example, a lower alkenyl group having 3 or 4 carbon atoms (e.g., a propenyl group, a butenyl group, etc.) and the like are preferable.

As the alkynyl group, for example, a lower alkynyl group having 3 to 6 carbon atoms (e.g., a propynyl group, a butynyl group, a pentynyl group, etc.) and the like are preferable, and particularly, for example, a lower alkynyl group having 3 or 4 carbon atoms (e.g., a propynyl group, a butynyl group, etc.) and the like are preferable.

As the "substituents" of the above-mentioned "aliphatic hydrocarbon group optionally having substituents", for example, a heterocyclic group, an oxo group, a hydroxy group, a C₁₋₆ alkoxy group, a C₃₋₁₀ (particularly C₃₋₆) cycloalkyloxy group, a C₆₋₁₀ aryloxy group, a C₇₋₁₉ (particularly C₇₋₁₂) aralkyloxy group, a heterocyclyloxy group, a C₁₋₆ alkylthio group (sulfur atom may be oxidized), a C₃₋₁₀ (particularly C₃₋₆) cycloalkylthio group (sulfur atom may be oxidized), a C₆₋₁₀ arylthio group (sulfur atom may be oxidized), a C₇₋₁₉ (particularly C₇₋₁₂) aralkylthio group (sulfur atom may be oxidized), a heterocyclylthio group, a heterocyclylsulfinyl group, a heterocyclylsulfonyl group, a nitro group, a halogen atom, a cyano group, a carboxyl group, a C₁₋₁₀ (particularly C₁₋₆) alkoxy-carbonyl group, a C₃₋₆ cycloalkyloxy-carbonyl group, a C₆₋₁₀ aryloxy-carbonyl group, a C₇₋₁₉ (particularly C₇₋₁₂), aralkyloxy-carbonyl group, a heterocyclyloxycarbonyl group, a C₆₋₁₀ aryl-carbonyl group, C₁₋₆ alkanoyl group, C₃₋₅ alkenoyl group, a C₆₋₁₀ aryl-carbonyloxy group, a C₂₋₆ alkanoyloxy group, a C₃₋₅ alkenoyloxy group, a carbamoyl group optionally having substituents, a thiocarbamoyl group optionally having substituents, a carbamoyloxy group optionally having substituents, a C₁₋₆ alkanoylamino group, a C₆₋₁₀ aryl-carbonylamino group, a C₁₋₁₀ (particularly C₁₋₆) alkoxy-carboxamido group, a C₆₋₁₀ aryloxy-carboxamido group, a C₇₋₁₉ (particularly C₇₋₁₂) aralkyloxy-carboxamido group, a C₁₋₁₀ (particularly C₁₋₆) alkoxy-carbonyloxy group, a C₆₋₁₀ aryloxy-carbonyloxy group, a C₇₋₁₉ (particularly C₇₋₁₂) aralkyloxy-carbonyloxy group, a C₃₋₁₀ (particularly C₃₋₆) cycloalkyloxy-carbonyloxy group, a ureido group optionally having substituents, a C₆₋₁₀ aryl group optionally having substituents, etc. are used.

These substituents are substituted at substitutable positions in the above-mentioned "aliphatic hydrocarbon group", wherein the substituents are not limited to a single substituent but may be the same or different plural (preferably 2 to 4) substituents.

As the "C₁₋₆ alkoxy group", for example, a methoxy group, an ethoxy group, an n-propoxy group, an isopropoxy group, an n-butoxy group, a tert-butoxy group, an n-pentyloxy group, an n-hexyloxy group, etc. are used, as the "C₃₋₁₀ cycloalkyloxy group", for example, a cyclopropyloxy group, a cyclohexyloxy group, etc. are used, as the "C₆₋₁₀ aryloxy group", for example, a phenoxy group, a naphthyloxy group, etc. are used, as the "C₇₋₁₉ aralkyloxy group", for example, a benzyloxy group, a 1-phenylethyloxy group, a 2-phenylethyloxy group, a benzhydryloxy group, a 1-naphthylmethyloxy group, etc. are used, as the "C₁₋₆ alkylthio group (sulfur atom may be oxidized)", for example, a methylthio group, an ethylthio group, an n-propylthio group, an n-butylthio group, a methylsulfinyl group, a methylsulfonyl group, etc. are used, as the "C₃₋₁₀ cycloalkylthio group (sulfur atom may be oxidized)", for example, a cyclopropylthio group, a cyclohexylthio group, a cyclopentylsulfinyl group, a cyclohexylsulfonyl group, etc. are used, as the "C₆₋₁₀ arylthio group (sulfur atom may be oxidized)", for example, a phenylthio group, a naphthylthio group, a phenylsulfinyl group, a phenylsulfonyl group, etc. are used, as the "C₇₋₁₉ aralkylthio group (sulfur atom may be oxidized)", for example, a benzylthio group, a phenylethylthio group, a benzhydrylthio group, a benzylsulfinyl group, a benzylsulfonyl group, etc. are used, as the "halogen atom", a fluorine atom, a chlorine atom, a bromine atom and an iodine atom are used, as the "C₁₋₁₀ alkoxy-carbonyl group", for example, a methoxycarbonyl group, an ethoxycarbonyl group, an n-propoxycarbonyl group, an isopropoxycarbonyl group, an n-butoxycarbonyl group, an isobutoxycarbonyl group, a tert-butoxycarbonyl group, etc. are used, as the "C₃₋₆ cycloalkyloxy-carbonyl group", for example, a cyclopropyloxycarbonyl group, a cyclopentyloxycarbonyl group, a cyclohexyloxycarbonyl group, etc. are used, as the "C₆₋₁₀ aryloxy-carbonyl group", for example, a phenoxycarbonyl group, a naphthyloxycarbonyl group, etc. are used, as the "C₇₋₁₉ aralkyloxy-carbonyl group", for example, a benzyloxycarbonyl group, a benzhydryloxycarbonyl group, a 2-phenethyloxycarbonyl group, etc. are used, as the "C₆₋₁₀ aryl-carbonyl group", for example, a benzoyl group, a naphthoyl group, etc. are used, as the "C₁₋₆ alkanoyl group", for example, a formyl group, an acetyl group, a propionyl group, a butyryl group, a valeryl group, a pivaloyl group, etc. are used, as the "C₃₋₅ alkenoyl group", for example, an acryloyl group, a crotonoyl group, etc. are used, as the "C₆₋₁₀ aryl-carbonyloxy group", for example, a benzoyloxy group, a naphthoyloxy group, etc. are used, as the "C₂₋₆ alkanoyloxy group", for example, an acetoxy group, a propionyloxy group, a butyryloxy group, a valeryloxy group, a pivaloyloxy group, etc. are used, and as the "C₃₋₅ alkenoyloxy group", for example, an acryloyloxy group, a crotonoyloxy group, etc. are used.

As the "carbamoyl group optionally having substituents", for example, a carbamoyl group or a cyclicamino (e.g., pyrrolidinyl, piperidinyl, piperazinyl, morpholinyl, etc.) carbonyl group, which may be substituted by 1 or 2 substituents selected from a C₁₋₄ alkyl (e.g., methyl, ethyl, etc.), a phenyl, a C₁₋₇ acyl (e.g., acetyl, propionyl, benzoyl, etc.), a C₁₋₄ alkoxy-phenyl (e.g., methoxyphenyl, etc.), etc., and the like are used, and specifically, for example, a carbamoyl group, an N-methylcarbamoyl group, an N-ethylcarbamoyl group, an N,N-dimethylcarbamoyl group, an N,N-diethylcarbamoyl group, an N-phenylcarbamoyl group, an N-acetylcarbamoyl group, an N-benzoylcarbamoyl group, an N-(p-methoxyphenyl)carbamoyl group, a 1-pyrrolidinylcarbonyl group, a piperidinocarbonyl group, a 1-piperazinylcarbonyl group, a morpholinocarbonyl group, etc. are used. As the "thiocarbamoyl group optionally having substituents", for example, a thiocarbamoyl group which may be substituted by 1 or 2 substituents selected from C₁₋₄ alkyl (e.g., methyl, ethyl, etc.), phenyl, etc. are used, and specifically, for example, a thiocarbamoyl group, an N-methylthiocarbamoyl group, an N-phenylthiocarbamoyl group, etc. are used. As the "carbamoyloxy group optionally having substituents", for example, a carbamoyloxy group which may be substituted by 1 or 2 substituents selected from C₁₋₄ alkyl (e.g., methyl, ethyl, etc.), phenyl, etc. are used, and specifically, for example, a carbamoyloxy group, an N-methylcarbamoyloxy group, an N,N-dimethylcarbamoyloxy group, an N-ethylcarbamoyloxy group, an N-phenylcarbamoyloxy group, etc. are used.

As the "C₁₋₆ alkanoylamino group", for example, an acetamido group, a propionamido group, a butyramido group, a valeramido group, a pivalamido group, etc. are used, as the "C₆₋₁₀ aryl-carbonylamino group", for example, a benzamido group, a naphthamido group, a phthalimido group, etc. are used, as the "C₁₋₁₀ alkoxy-carboxamido group", for example, a methoxycarboxamido (CH₃OCONH-) group, an ethoxycarboxamido group, a tert-butoxycarboxamido group, etc. are used, as the "C₆₋₁₀ aryloxy-carboxamido group", for example, a phenoxycarboxamido (C₆H₅OCONH-) group, etc. are used, as the "C₇₋₁₉ aralkyloxy-carboxamido group", for example, a benzyloxycarboxamido (C₆H₅CH₂OCONH-) group, a benzhydryloxycarboxamido group, etc. are used, as the "C₁₋₁₀ alkoxy-carbonyloxy group", for example, a methoxycarbonyloxy group, an ethoxycarbonyloxy group, an n-propoxycarbonyloxy group, an isopropoxycarbonyloxy group, an n-butoxycarbonyloxy group, a tert-butoxycarbonyloxy group, an n-pentyloxycarbonyloxy group, an n-hexyloxycarbonyloxy group, etc. are used, as the ''C₆₋₁₀ aryloxy-carbonyloxy group", for example, a phenoxycarbonyloxy group, a naphthyloxycarbonyloxy group, etc. are used, as the "C₇₋₁₉ aralkyloxy-carbonyloxy group", for example, a benzyloxycarbonyloxy group, a 1-phenylethyloxycarbonyloxy group, a 2-phenylethyloxycarbonyloxy group, a benzhydryloxycarbonyloxy group, etc. are used, and as the "C₃₋₁₀ cycloalkyloxy-carbonyloxy group", for example, a cyclopropyloxycarbonyloxy group, a cyclohexyloxycarbonyloxy group, etc. are used.

As the "ureido group optionally having substituents", for example, a ureido group optionally substituted by 1 to 3 (preferably 1 or 2) substituents selected from a C₁₋₄ alkyl group (e.g., a methyl group, an ethyl group, etc.), a phenyl group, etc. are used, and, for example, a ureido group, a 1-methylureido group, a 3-methylureido group, a 3,3-dimethylureido group, a 1,3-dimethylureido group, a 3-phenylureido group, etc. are used.

When a heterocyclic group, a heterocyclyloxy group, a heterocyclylthio group, a heterocyclylsulfinyl group, a heterocyclylsulfonyl group or a heterocyclyloxycarbonyl group is used as the "substituents" of the "aliphatic hydrocarbon group optionally having substituents", the heterocyclic group represents a group formed by excluding one hydrogen atom that binds to the heterocycle. It represents, for example, a 5- to 8-membered ring (preferably 5- or 6-membered ring) group containing 1 to a few, preferably 1 to 4 hetero atoms such as a nitrogen atom (optionally oxidized), an oxygen atom, a sulfur atom, etc., or its condensed cyclic group. As these heterocyclic groups, for example, a pyrrolyl group, a pyrazolyl group, an imidazolyl group, a 1,2,3-triazolyl group, a 1,2,4-triazolyl group, a tetrazolyl group, a furyl group, a thienyl group, an oxazolyl group, an isoxazolyl group, a 1,2,3-oxadiazolyl group, a 1,2,4-oxadiazolyl group, a 1,2,5-oxadiazolyl group, a 1,3,4-oxadiazolyl group, a thiazolyl group, an isothiazolyl group, a 1,2,3-thiadiazolyl group, a 1,2,4-thiadiazolyl group, a 1,2,5-thiadiazolyl group, a 1,3,4-thiadiazolyl group, a pyridyl group, a pyridazinyl group, a pyrimidinyl group, a pyrazinyl group, an indolyl group, a pyranyl group, a thiopyranyl group, a dioxinyl group, a dioxolyl group, a quinolyl group, a pyrido[2,3-d]pyrimidyl group, a 1,5-, 1,6-, 1,7-, 1,8-, 2,6- or 2,7-naphthyridyl group, a thieno[2,3-d]pyridyl group, a benzopyranyl group, a tetrahydrofuryl group, a tetrahydropyranyl group, a dioxolanyl group, a dioxanyl group, etc. are used.

These heterocyclic groups may be substituted at substitutable positions by 1 to 3 substituents selected from a C₁₋₄ alkyl (e.g., methyl, ethyl, etc.), a hydroxy, an oxo, a C₁₋₄ alkoxy (e.g., methoxy, ethoxy, etc.), and the like.

As the "C₆₋₁₀ aryl group" of the "C₆₋₁₀ aryl group optionally having substituents", for example, a phenyl group, a naphthyl group, etc. are used. The C₆₋₁₀ aryl group may be substituted at a substitutable position by a substituent selected from those exemplified as the "substituent" (except for a C₆₋₁₀ aryl group optionally having substituents) of the "aliphatic hydrocarbon group optionally having substituents" described above. Such substituent is not limited to a single substituent, but the same or different, more than one (preferably 2 to 4) substituents may be used.

In the "aliphatic hydrocarbon group optionally having substituents", the substituent together with the aliphatic hydrocarbon group may form an optionally substituted fused ring group, and as such fused ring group, an indanyl group, a 1,2,3,4-tetrahydronaphthyl group, etc. are used. This fused ring group may be substituted at a substitutable position by a substituent selected from those exemplified as the "substituent" of the "aliphatic hydrocarbon group optionally having substituents" described above. Such substituent is substituted at a substitutable position of the fused ring group, wherein the substituent is not limited to a single substituent, but the same or different, more than one (preferably 2 to 4) substituents may be used.

As preferable examples of the above-mentioned "aliphatic hydrocarbon group optionally having substituents" for R, R¹, R¹¹, R¹⁸, R^{1b}, R^{1c}, R^{4a} and R^{5a}, for example, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group and the like) optionally having substituents, etc., are used. Of these, a methyl group, an ethyl group, an n-propyl group, an isopropyl group, an n-butyl group, an isobutyl group, etc. are preferable. For example, a methyl group, an ethyl group, an n-propyl group and the like are more preferable, and particularly, an ethyl group, etc. are preferable.

As R² and R^{2a}, for example, a hydrogen atom, a lower alkyl group having 1 to 6 carbon atoms (e.g., a methyl group, an ethyl group, an n-propyl group, an isopropyl group, an n-butyl group, an isobutyl group, a tert-butoxycarbonylmethyl group, a hydroxyethyl group and the like), etc. are preferably used. Of these, a hydrogen atom, a methyl group, etc. are particularly preferably used and more particularly, a hydrogen atom, etc. are preferably used.

As the "aromatic hydrocarbon group" of the "aromatic hydrocarbon group optionally having substituents" represented by R, an aromatic hydrocarbon group having 6 to 14 carbon atoms (e.g., a phenyl group, a naphthyl group, an anthryl group, an indenyl group and the like) and the like are preferable, and particularly for example, an aryl group having 6 to 10 carbon atoms (e.g., phenyl, naphthyl groups etc.) and the like are preferable and, of these, a phenyl group and the like are particularly preferable.

As the "substituent" of the "aromatic hydrocarbon group optionally having substituents" represented by R, for example, a halogen atom (fluorine atom, chlorine atom, bromine atom, iodine atom), a lower (C₁₋₄) alkyl group (e.g., a methyl group, an ethyl group, a propyl group, a butyl group and the like), a lower (C₁₋₄) alkoxy group (e.g., a methoxy group, an ethoxy group, a propoxy group, a butoxy group and the like), a lower (C₁₋₄) alkoxy-carbonyl group (e.g., a methoxycarbonyl group, an ethoxycarbonyl group, a propoxycarbonyl group, a butoxycarbonyl group and the like), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, and the like), a cycloalkyl group having 3 to 6 carbon atoms (e.g., a cyclopropyl group, a cyclopentyl group and the like), an aryl group having 6 to 10 carbon atoms (e.g., a phenyl group, a naphthyl group, an indenyl group and the like), a halogeno-lower (C₁₋₄) alkyl group (e.g., a trifluoromethyl group, a trifluoroethyl group and the like), a halogeno-lower (C₁₋₄) alkoxy group (e.g., a trifluoromethoxy group, a 1,1,2,2-tetrafluoroethoxy group, a 2,2,3,3,3-pentafluoropropoxy group and the like), a lower (C₁₋₄) alkylthio group (e.g., a methylthio group, an ethylthio group, a propylthio group and the like), a lower (C₁₋₄) alkanesulfonyl group (e.g., a methanesulfonyl group, an ethanesulfonyl group, a propanesulfonyl group and the like), a lower (C₁₋₄) alkanoyl group (e.g., a formyl group, an acetyl group, a propionyl group and the like), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group and the like), a carbamoyl group, a lower (C₁₋₄) alkyl-carbamoyl group (e.g., a methylcarbamoyl group, a dimethylcarbamoyl group, a propylcarbamoyl group and the like), a lower (C₁₋₄) alkoxy-carbonyl-lower (C₁₋₄) alkyl-carbamoyl group (e.g., a butoxycarbonylmethylcarbamoyl group, an ethoxycarbonylmethylcarbamoyl group and the like), a 1,3-diacylguanidino-lower (C₁₋₄) alkyl group (e.g., 1,3-diacetylguanidinomethyl, 1,3-bis-(tert-butoxycarbonyl)guanidinomethyl and the like) and the like are used, and a halogen atom (fluorine, chlorine, bromine, iodine atoms), a lower (C₁₋₄) alkyl group (e.g., a methyl group, an ethyl group, a propyl group, a butyl group and the like) and the like are preferably used, and a fluorine atom, a chlorine atom and a methyl group are more preferably used.

These substituents are substituted at substitutable positions of the aromatic hydrocarbon group, and the number of the substituents is preferably 1 to 5, more preferably 1 to 3, most preferably 1 or 2. When two or more of such substituents are present, they may be the same or different.

The "heterocyclic group" of the "heterocyclic group optionally having substituents" represented by R is, for example, a 5 to 8-membered ring (particularly 5 or 6-membered ring) group containing 1 to several, preferably 1 to 4, hetero atoms such as nitrogen atom (optionally oxidized), oxygen atom, sulfur atom and the like, and a fused ring group thereof. As such heterocyclic group, for example, pyrrolyl group, pyrazolyl group, imidazolyl group, 1,2,3-triazolyl group, 1,2,4-triazolyl group, tetrazolyl group, furyl group, thienyl group, oxazolyl group, isoxazolyl group, 1,2,3-oxadiazolyl group, 1,2,4-oxadiazolyl group, 1,2,5-oxadiazolyl group, 1,3,4-oxadiazolyl group, thiazolyl group, isothiazolyl group, 1,2,3-thiadiazolyl group, 1,2,4-thiadiazolyl group, 1,2,5-thiadiazolyl group, 1,3,4-thiadiazolyl group, pyridyl group, pyridazinyl group, pyrimidinyl group, pyrazinyl group, indolyl group, pyranyl group, thiopyranyl group, dioxinyl group, dioxolyl group, quinolyl group, pyrido[2,3-d]pyrimidyl group, 1,5-, 1,6-, 1,7-, 1,8-,2,6- or 2,7-naphthyridinyl group, thieno[2,3-d]pyridyl group, benzopyranyl group, tetrahydrofuryl group, tetrahydropyranyl group, dioxolanyl group, dioxanyl group and the like are used.

These heterocyclic groups are optionally substituted by 1 to 3 substituents selected from C₁₋₄ alkyl (e.g., methyl, ethyl and the like), hydroxy, oxo, C₁₋₄ alkoxy (e.g., methoxy, ethoxy and the like) and the like at substitutable positions.

As the "aromatic hydrocarbon group" of the "aromatic hydrocarbon group optionally having substituents" represented by Ar and Ar^{a}, an aromatic hydrocarbon group having 6 to 14 carbon atoms (e.g., a phenyl group, a naphthyl group, an anthryl group, an indenyl group and the like) and the like are preferable, and particularly for example, an aryl group having 6 to 10 carbon atoms (e.g., phenyl, naphthyl groups etc.) and the like are preferable and, of these, a phenyl group and the like are particularly preferable.

As the "substituent" of the "aromatic hydrocarbon group optionally having substituents" represented by Ar and Ar^{a}, for example, a halogen atom (fluorine, chlorine, bromine, iodine atoms), a lower (C₁₋₄) alkyl group (e.g., a methyl group, an ethyl group, a propyl group, an isopropyl group, a butyl group and the like), a lower (C₁₋₄) alkoxy group (e.g., a methoxy group, an ethoxy group, a propoxy group, a butoxy group and the like), a lower (C₁₋₄) alkoxy-carbonyl group (e.g., a methoxycarbonyl group, an ethoxycarbonyl group, a propoxycarbonyl group, a butoxycarbonyl group and the like), a carboxyl group, a nitro group, a cyano group, a hydroxyl group, an acylamino group (e.g., an alkanoylamino group having 1 to 4 carbon atoms such as an acetylamino group, a propionylamino group, a butyrylamino group and the like, and the like), a cycloalkyl group having 3 to 6 carbon atoms (e.g., a cyclopropyl group, a cyclopentyl group and the like), an aryl group having 6 to 10 carbon atoms (e.g., a phenyl group, a naphthyl group, an indenyl group and the like), a halogeno-lower (C₁₋₄) alkyl group (e.g., a trifluoromethyl group, a trifluoroethyl group and the like), a halogeno-lower (C₁₋₄) alkoxy group (e.g., a trifluoromethoxy group, a 1,1,2,2-tetrafluoroethoxy group, a 2,2,3,3,3-pentafluoropropoxy group and the like), a lower (C₁₋₄) alkylthio group (e.g., a methylthio group, an ethylthio group, a propylthio group and the like), a lower (C₁₋₄) alkanesulfonyl group (e.g., a methanesulfonyl group, an ethanesulfonyl group, a propanesulfonyl group and the like), a lower (C₁₋₄) alkanoyl group (e.g., a formyl group, an acetyl group, a propionyl group and the like), a 5-membered aromatic heterocyclic group (e.g., a 1,2,3-triazolyl group, a 1,2,4-triazolyl group, a tetrazolyl group, a thiazolyl group, an isothiazolyl group, an oxazolyl group, an isoxazolyl group, a thiadiazolyl group, a thienyl group, a furyl group and the like), a carbamoyl group, a lower (C₁₋₄) alkyl-carbamoyl group (e.g., a methylcarbamoyl group, a dimethylcarbamoyl group, a propionylcarbamoyl group and the like), a lower (C₁₋₄) alkoxy-carbonyl-lower (C₁₋₄) alkyl-carbamoyl group (e.g., a butoxycarbonylmethylcarbamoyl group, a tert-butoxycarbonylmethylcarbamoyl group, an ethoxycarbonylmethylcarbamoyl group and the like), a 1,3-diacylguanidino-lower (C₁₋₄) alkyl group (e.g., 1,3-diacetylguanidinomethyl, 1,3-bis-(tert-butoxycarbonyl)guanidinomethyl and the like) and the like are used, and a halogen atom (fluorine, chlorine, bromine, iodine atoms), a lower (C₁₋₄) alkyl group (e.g., a methyl group, an ethyl group, a propyl group, a butyl group and the like) and the like are preferably used, and a fluorine atom, a chlorine atom and a methyl group are more preferably used.

These substituents are substituted at substitutable positions of the aromatic hydrocarbon group, and the number of the substituents is preferably 1 to 5, more preferably 1 to 3, most preferably 1 or 2. When two or more of such substituents are present, they may be the same or different.

Typically, as Ar and Ar^{a}, for example, a phenyl group, a halogenophenyl group, a lower (C₁₋₄) alkylphenyl group, a lower (C₁₋₄) alkoxyphenyl group, a lower (C₁₋₄) alkoxy-carbonylphenyl group, a carboxylphenyl group, a nitrophenyl group, a cyanophenyl group, a halogeno-lower (C₁₋₄) alkylphenyl group, a halogeno-lower (C₁₋₄) alkoxyphenyl group, a lower (C₁₋₄) alkanoyl phenyl group, a 5-membered aromatic heterocycle-substituted phenyl group, a lower (C₁₋₄) alkoxy-carbonyl-lower (C₁₋₄) alkyl-carbamoylphenyl group, 1,3-diacylguanidino-lower (C₁₋₄) alkylphenyl group, a halogen- and lower (C₁₋₄) alkyl-substituted phenyl group, a halogen- and lower (C₁₋₄) alkoxycarbonyl-substituted phenyl group, a halogen- and cyano-substituted phenyl group, a halogen- and 5-membered aromatic heterocycle-substituted phenyl group, a halogen- and lower (C₁₋₄) alkoxy-carbonyl-lower (C₁₋₄) alkyl-carbamoyl-substituted phenyl group and the like are used.

As Ar and Ar^{a}, a phenyl group optionally having substituents are preferable. Of these, a halogenophenyl group, a lower (C₁₋₄) alkylphenyl group, a halogen- and lower (C₁₋₄) alkoxycarbonyl-substituted phenyl group, a halogen- and lower (C₁₋₄) alkyl-substituted phenyl group and the like are preferably used.

As Ar and Ar^{a}, a group represented by the formula: 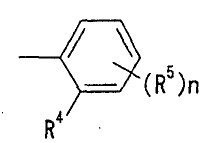 wherein R⁴ and R⁵ are the same or different and each represents a halogen atom or a lower alkyl group, and n is an integer of 0 to 2, is more preferable, in which a group wherein at least one of R⁴ and R⁵ is a halogen atom is still more preferable.

As the halogen atom represented by R⁴ and R⁵, a fluorine atom or a chlorine atom is preferable.

As the halogenophenyl group, for example, a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 2-fluorophenyl group, a 2-chlorophenyl group, a 3-fluorophenyl group, a 3-chlorophenyl group, a 4-fluorophenyl group, a 4-chlorophenyl group, a 4-chloro-2-fluorophenyl group, a 2-chloro-4-fluorophenyl group, a 4-bromo-2-fluorophenyl group, a 2,3,4-trifluorophenyl group, a 2,4,5-trifluorophenyl group, a 2,4,6-trifluorophenyl and the like are used.

As the lower (C₁₋₄) alkylphenyl group, for example, a 2-ethylphenyl group, a 2,6-diisopropylphenyl group and the like are preferably used, and as the lower (C₁₋₄) alkoxyphenyl group, for example, a 4-methoxyphenyl and the like are preferably used.

As the lower (C₁₋₄) alkoxy-carbonylphenyl group, for example, a 2-ethoxycarbonylphenyl group, a 2-methoxycarbonylphenyl group, a 4-methoxycarbonylphenyl group and the like are preferably used, and as the halogeno-lower (C₁₋₄) alkylphenyl group, for example, a 2-trifluoromethylphenyl group and the like are preferably used, and as the halogeno-lower (C₁₋₄) alkoxyphenyl group, for example, a 2-trifluoromethoxyphenyl group, a 4-(2,2,3,3,3-pentafluoropropoxy)phenyl group and the like are preferably used.

As the lower (C₁₋₄) alkanoylphenyl group, for example, a 2-acetylphenyl group and the like are preferably used, and as the 5-membered aromatic heterocycle-substituted phenyl group, for example, a 4-(2H-1,2,3-triazol-2-yl)phenyl group, a 4-(2H-tetrazol-2-yl)phenyl group, a 4-(1H-tetrazol-1-yl)phenyl group, a 4-(1H-1,2,3-triazol-1-yl)phenyl group and the like are preferably used, and as the lower (C₁₋₄) alkoxy-carbonyl-lower (C₁₋₄) alkyl-carbamoylphenyl group, for example, a 4-(N-ethoxycarbonylmethylcarbamoyl)phenyl group and the like are preferably used, and as the 1,3-diacylguanidino-lower (C₁₋₄) alkylphenyl group, for example, a 4-(1,3-bis-tert-butoxycarbonylguanidinomethyl)phenyl group and the like are preferably used.

As the phenyl group substituted by halogen atom and lower (C₁₋₄) alkyl group, for example, a 2-fluoro-4-methylphenyl group, a 2-chloro-4-methylphenyl group, a 4-fluoro-2-methylphenyl group and the like are preferably used, and as the phenyl group substituted by halogen atom and lower (C₁₋₄) alkoxy-carbonyl group, for example, a 2-chloro-4-methoxycarbonylphenyl group and the like are preferably used, and the phenyl group substituted by halogen atom and cyano group, a 2-chloro-4-cyanophenyl group and the like are preferably used, and as the phenyl group substituted by halogen atom and 5-membered aromatic heterocyclic group, for example, a 2-fluoro-4-(1H-1,2,4-triazol-1-yl)phenyl group and the like are preferably used, and as the phenyl group substituted by halogen atom and lower (C₁₋₄) alkoxy-carbonyl-lower (C₁₋₄) alkyl-carbamoyl group, for example, a 2-chloro-4-(N-tert-butoxycarbonylmethylcarbamoyl)phenyl group, a 2-chloro-4-(N-ethoxycarbonylmethylcarbamoyl)phenyl group and the like are preferably used.

More specifically, as Ar and Ar^{a}, a phenyl group, a phenyl group substituted by 1 to 3 (particularly 1 or 2) halogen atoms (e.g., a 2,3-difluorophenyl group, a 2,3-dichlorophenyl group, a 2,4-difluorophenyl group, a 2,4-dichlorophenyl group, a 2,5-difluorophenyl group, a 2,5-dichlorophenyl group, a 2,6-difluorophenyl group, a 2,6-dichlorophenyl group, a 3,4-difluorophenyl group, a 3,4-dichlorophenyl group, a 3,5-difluorophenyl group, a 3,5-dichlorophenyl group, a 4-bromo-2-fluorophenyl group, a 2-fluorophenyl group, a 2-chlorophenyl group, a 3-fluorophenyl group, a 3-chlorophenyl group, a 4-fluorophenyl group, a 4-chlorophenyl group, a 2-fluoro-4-chlorophenyl group, a 2-chloro-4-fluorophenyl group, a 2,3,4-trifluorophenyl group, a 2,4,5-trifluorophenyl group and the like), a phenyl group substituted by halogen atom and lower (C₁₋₄) alkyl group (e.g., a 2-chloro-4-methylphenyl group, a 4-fluoro-2-methylphenyl group and the like), etc. are particularly preferable. Of these, a phenyl group substituted by 1 to 3 (particularly 1 or 2) halogen atoms (e.g., a 2,3-dichlorophenyl group, a 2,4-difluorophenyl group, a 2,4-dichlorophenyl group, a 2,6-dichlorophenyl group, a 2-fluorophenyl group, a 2-chlorophenyl group, a 3-chlorophenyl group, a 2-chloro-4-fluorophenyl group, a 2,4,5-trifluorophenyl group and the like), a phenyl group substituted by halogen atom and lower (C₁₋₄) alkyl group (e.g., a 2-chloro-4-methylphenyl group, a 4-fluoro-2-methylphenyl group and the like), etc. are preferable. Particularly, a 2,4-difluorophenyl group, a 2-chlorophenyl group, a 2-chloro-4-fluorophenyl group, a 2-chloro-4-methylphenyl group and the like are preferable, and a 2,4-difluorophenyl group, a 2-chloro-4-fluorophenyl group and the like are preferable.

In this specification, the ring A¹ represents a cycloalkene optionally substituted by 1 to 4 substituents selected from (i) an aliphatic hydrocarbon group optionally having substituents, (ii) an aromatic hydrocarbon group optionally having substituents, (iii) a group represented by the formula -OR¹¹ (wherein R¹¹ is hydrogen atom or aliphatic hydrocarbon group optionally having substituents) and (iv) a halogen atom, and a cycloalkene optionally substituted by 1 to 4 substituents selected from (i) an aliphatic hydrocarbon group optionally having substituents, (ii) an aromatic hydrocarbon group optionally having substituents and (iv) a halogen atom is preferable.

In this specification, the ring A² represents a cycloalkene substituted by 1 to 4 substituents selected from (i) an aliphatic hydrocarbon group optionally having substituents, (ii) an aromatic hydrocarbon group optionally having substituents, (iii) a group represented by the formula -OR¹¹ (wherein R¹¹ is as defined above) and (iv) a halogen atom, and a cycloalkene substituted by 1 to 4 substituents selected from (i) an aliphatic hydrocarbon group optionally having substituents, (ii) an aromatic hydrocarbon group optionally having substituents and (iv) a halogen atom are preferable.

These substituents (i) - (iv) are substituted on substitutable carbon atoms in the ring A¹ and ring A², and when the ring A¹ or ring A² is substituted by two or more of such substituents, the substituents may be the same or different. A single carbon atom may be substituted by two substituents, and different carbon atoms may be substituted by two or more substituents.

As the "aliphatic hydrocarbon group optionally having substituents" as a substituent on the ring A¹ and ring A², for example, the same those as the "aliphatic hydrocarbon group optionally having substituents" represented by R, R¹, R¹¹, R^{1a}, R^{1b}, R^{1c}, R^{4a} and R^{5a} described above may be used.

As the "aromatic hydrocarbon group optionally having substituents" as a substituent on the ring A¹ and ring A², for example, the same substituents as those of the "aromatic hydrocarbon group optionally having substituents" represented by Ar and Ar^{a} described above may be used.

As the "heterocyclic group optionally having substituents" as a substituent on the ring A¹ and ring A², for example, those similar to the "heterocyclic group" which is a "substituent" on the "aliphatic hydrocarbon group optionally having substituents" represented by R, R¹, R¹¹, R^{1a}, R^{1b}, R^{1c}, R^{4a} and R^{5a} described above may be used.

As the substituents for the ring A¹ and ring A², 1 or 2 C₁₋₆ alkyl groups (e.g., a C₁₋₄ alkyl group such as a methyl group, a tert-butyl group, etc.), a phenyl group, a halogen atom (fluorine, chlorine, bromine, iodine atoms), etc. are preferably used.

The group represented by the formula: 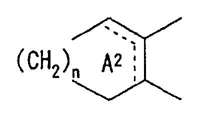 wherein n is as defined above, represents a group represented by the formula: 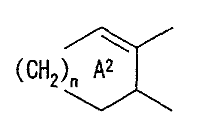 or 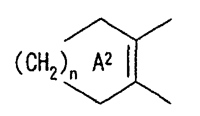 wherein n is as defined above, preferably a group represented by the formula: 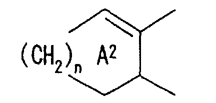 wherein n is as defined above.

The group represented by the formula: 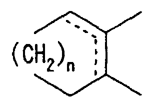 wherein n is as defined above, represents a group represented by the formula: 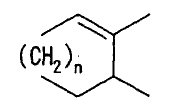 or 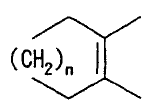 wherein n is as defined above, preferably a group represented by the formula: 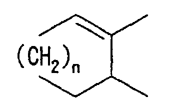 wherein n is as defined above, and
a group represented by the formula: 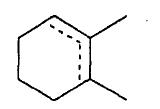 represents a group represented by the formula: 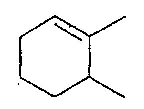 or 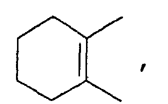 preferably a group represented by the formula:

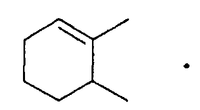

As the integer of 1 to 4 represented by n, 1 to 3 is preferable, and 2 is particularly preferable.

As the compound represented by the formula (Iaa), the compound represented by the formula (Ibb') is preferable, and as the compound represented by the formula (Ia), the compound represented by the formula (Ib) is preferable.

As the compound represented by the formula (Ibb'), the compound represented by the formula (Inn) is preferable, and as the compound represented by the formula (Ib), the compound represented by the formula (In) is preferable.

As the compounds represented by the formulas (Ibb') and (Ib), a compound wherein R¹ is a lower alkyl group optionally having substituents, R² is a hydrogen atom or a lower alkyl group, Ar is a phenyl group optionally having substituents, and n is 1, 2 or 3 is preferable, and a compound wherein R¹ is a lower alkyl group optionally having substituents, R² is a hydrogen atom, Ar is a phenyl group substituted by a halogen atom, and n is 2 is more preferable.

As the compounds represented by the formulas (Icc) and (Ic), a compound wherein Ar is a phenyl group optionally having substituents, and n is 2 is preferable.

Specifically, as the compound represented by the formula (I) or (Ia), a compound obtained in Reference Example B to be mentioned below and the like is used. Among others,
(i) d-ethyl 6-[N-(2,4-difluorophenyl)sulfamoyl]-1-cyclohexene-1-carboxylate,
(ii) ethyl 6-[N-(2-chlorophenyl)sulfamoyl]-1-cyclohexene-1-carboxylate,
(iii) ethyl 6-[N-(2-chloro-4-methylphenyl)sulfamoyl]-1-cyclohexene-1-carboxylate, and
(iiii) ethyl (6R)-6-[(2-chloro-4-fluoroanilino)sulfonyl]-1-cyclohexene-1-carboxylate and salts thereof and the like are preferable.

The compound of the formula (II) is explained in detail in the following.
[1] a compound represented by the formula: 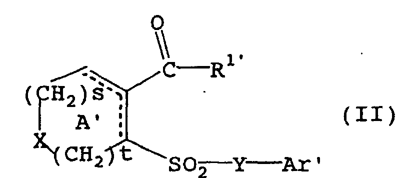 wherein R^{1'} is an aliphatic hydrocarbon group optionally having substituents, an aromatic hydrocarbon group optionally having substituents, a heterocyclic group optionally having substituents, a group represented by the formula: -OR^{1a'} wherein R^{1a'} is a hydrogen atom or an aliphatic hydrocarbon group optionally having substituents, or a group represented by the formula: 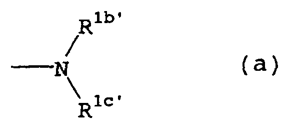 wherein R^{1b'} and R^{1c'} are the same or different and each is a hydrogen atom, or an aliphatic hydrocarbon group optionally having substituents, X is a methylene group, NH, a sulfur atom or an oxygen atom, Y is a methylene group optionally having substituents or NH optionally having substituents, ring A' is a 5 to 8-membered ring optionally further substituted by 1 to 4 substituents selected from (1) an aliphatic hydrocarbon group optionally having substituents, (2)an aromatic hydrocarbon group optionally having substituents, (3) a group represented by the formula: -OR^{2'} wherein R^{2'} is a hydrogen atom or an aliphatic hydrocarbon group optionally having substituents and (4) a halogen atom, Ar' is an aromatic hydrocarbon group optionally having substituents, a group represented by the formula: 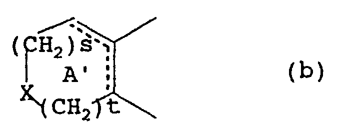 is a group represented by the formula: 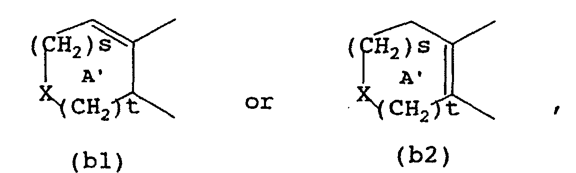 s is an integer of 0 to 2, t is an integer of 1 to 3, and the total of s and t is not more than 4, provided that when X is a methylene group, Y is a methylene group optionally having substituents, or a salt thereof,
[2] the compound of [1], wherein R^{1'} is an aliphatic hydrocarbon group selected from (i) C₁₋₂₀ alkyl group, C₃₋₁₀ cycloalkyl group, C₄₋₁₂ cycloalkylalkyl group, C₃₋₆ alkenyl group and C₃₋₆ alkynyl group
   (These aliphatic hydrocarbon groups may have 1 to 4 substituents selected from a group (hereinafter substituent Group A) consisting of a heterocyclic group, an oxo group, a hydroxyl group, a C₁₋₆ alkoxy group, a C₃₋₁₀ cycloalkyloxy group, a C₆₋₁₀ aryloxy group, a C₇₋₁₉ aralkyloxy group, a heterocyclyloxy group, a C₁₋₆ alkylthio group (said sulfur atom being optionally oxidized), a C₃₋₁₀ cycloalkylthio group (said sulfur atom being optionally oxidized), a C₆₋₁₀ arylthio group (said sulfur atom being optionally oxidized), a C₇₋₁₉ aralkylthio group(said sulfur atom being optionally oxidized), a heterocyclylthio group, a heterocyclylsulfinyl group, a heterocyclylsulfonyl group, a nitro group, a halogen atom, a cyano group, a carboxyl group, a C₁₋₁₀ alkoxy-carbonyl group, a C₃₋₆ cycloalkyloxy-carbonyl group, a C₆₋₁₀ aryloxy-carbonyl group, a C₇₋₁₉ aralkyloxy-carbonyl group, a heterocyclyloxycarbonyl group, a C₆₋₁₀ aryl-carbonyl group, a c₁₋₆ alkanoyl group, a C₃₋₅ alkenoyl group, a C₆₋₁₀ aryl-carbonyloxy group, a C₂₋₆ alkanoyloxy group, a C₃₋₅ alkenoyloxy group, a carbamoyl group (optionally substituted by 1 or 2 substituents selected from C₁₋₄ alkyl, phenyl, C₁₋₇ acyl and C₁₋₄ alkoxy-phenyl), a thiocarbamoyl group (optionally substituted by 1 or 2 substituents selected from C₁₋₄ alkyl and phenyl), a carbamoyloxy group (optionally substituted by 1 or 2 substituents selected from C₁₋₄ alkyl and phenyl), a C₁₋₆ alkanoylamino group, a C₆₋₁₀ aryl-carbonylamino group, a C₁₋₁₀ alkoxy-carboxamido group, a C₆₋₁₀ aryloxy-carboxamido group, a C₇₋₁₉ aralkyloxy-carboxamido group, a C₁₋₁₀ alkoxy-carbonyloxy group, a C₆₋₁₀ aryloxy-carbonyloxy group, a C₇₋₁₉ aralkyloxy-carbonyloxy group, a C₃₋₁₀ cycloalkyloxy-carbonyloxy group and a ureido group (optionally substituted by 1 to 3 substituents selected from C₁₋₄ alkyl group and phenyl group) and a group (hereinafter substituent Group B) consisting of C₆₋₁₀ aryl groups optionally having 1 to 4 substituents selected from the substituent Group A.
   The aforementioned heterocycle is a 5 to 8-membered heterocyclic group containing, besides carbon atom, 1 to 4 hetero atoms selected from nitrogen atom (optionally oxidized), oxygen atom and sulfur atom, or a fused ring group thereof, and may have 1 to 3 substituents selected from C₁₋₄ alkyl, hydroxy, oxo and C₁₋₄ alkoxy.
   The above-mentioned substituents may form, together with the aliphatic hydrocarbon group, a fused ring group optionally having 1 to 4 substituents selected from the substituent Group B.),
   (ii) C₆₋₁₄ aryl group
   (This C₆₋₁₄ aryl group may have 1 to 5 substituents selected from a group (hereinafter substituent Group C) consisting of a halogen atom, a C₁₋₄ alkyl group, a C₁₋₄ alkoxy group, a C₁₋₄ alkoxy-carbonyl group, a carboxyl group, a nitro group, a cyano group, a hydroxyl group, a C₁₋₄ alkanoylamino group, a C₃₋₆ cycloalkyl group, a C₆₋₁₀ aryl group, a halogeno C₁₋₄ alkyl group, a halogeno C₁₋₄ alkoxy group, a C₁₋₄ alkylthio group, a C₁₋₄ alkylsulfonyl group, a C₁₋₄ alkanoyl group, a 5-membered aromatic heterocyclic group, a carbamoyl group, a C₁₋₄ alkyl-carbamoyl group, a C₁₋₄ alkoxy-carbonyl-C₁₋₄ alkyl-carbamoyl group and a 1,3-diacylguanidino-C₁₋₄ alkyl group.)
   (iii) a 5 to 8-membered heterocyclic group containing, besides carbon atom, 1 to 4 hetero atoms selected from nitrogen atom (optionally oxidized), oxygen atom and sulfur atom, or a fused ring group thereof,
   (This heterocyclic group may have 1 to 3 substituents selected from C₁₋₄ alkyl, hydroxy, oxo and C₁₋₄ alkoxy.)
   (iv) a group represented by the formula: -OR^{1a'} wherein R^{1a'} is a hydrogen atom or an aliphatic hydrocarbon group, which is selected from C₁₋₂₀ alkyl group, C₃₋₁₀ cycloalkyl group, C₄₋₁₂ cycloalkylalkyl group, C₃₋₆ alkenyl group and C₃₋₆ alkynyl group, and optionally has substituents selected from substituent Group B.)
   or
   (v) a group represented by the formula: 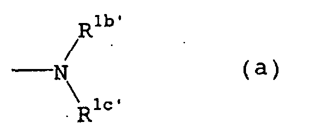 wherein R^{1b'} and R^{1c'} are the same or different and each is a hydrogen atom, or an aliphatic hydrocarbon group, which is selected from C₁₋₂₀ alkyl group, C₃₋₁₀ cycloalkyl group, C₄₋₁₂ cycloalkylalkyl group, C₃₋₆ alkenyl group and C₃₋₆ alkynyl group, and optionally has substituents selected from substituent Group B,
   X is a methylene group, NH, a sulfur atom or an oxygen atom,
   Y is (i) a methylene group optionally having substituents selected from C₁₋₆ alkyl group, hydroxy-substituted-C₁₋₆ alkyl group and C₁₋₄ alkoxy-carbonyl-C₁₋₄ alkyl group or (ii) NH optionally having substituents selected from C₁₋₆ alkyl group, hydroxy-substituted-C₁₋₆ alkyl group and C₁₋₄ alkoxy-carbonyl-C₁₋₄ alkyl group,
   ring A' is a 5 to 8-membered ring optionally further substituted by 1 to 4 substituents selected from (1) an aliphatic hydrocarbon group, which is selected from C₁₋₂₀ alkyl group, C₃₋₁₀ cycloalkyl group, C₄₋₁₂ cycloalkylalkyl group, C₃₋₆ alkenyl group and C₃₋₆ alkynyl group, and optionally has substituents selected from substituent Group B, (2) a C₆₋₁₄ aryl group optionally having substituents selected from substituent Group C, (3) a group represented by the formula: -OR^{2'} wherein R^{2'} is a hydrogen atom, or an aliphatic hydrocarbon group, which is selected from C₁₋₂₀ alkyl group, C₃₋₁₀ cycloalkyl group, C₄₋₁₂ cycloalkylalkyl group, C₃₋₆ alkenyl group and C₃₋₆ alkynyl group, and optionally has substituents selected from substituent Group B ) and (4) a halogen atom,
   Ar' is a C₆₋₁₄ aryl group optionally having substituents selected from substituent Group C,
   a group represented by the formula: 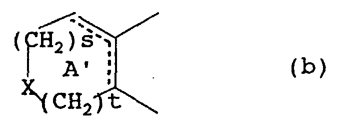 is a group represented by the formula: 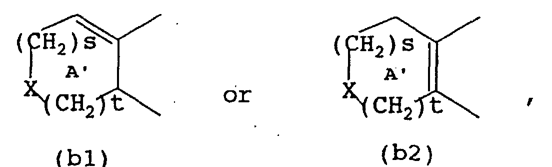 s is an integer of 0 to 2, t is an integer of 1 to 3, and the total of s and t is not more than 4,
[3] the compound of [1] wherein ring A' is a 5 to 8-membered ring optionally substituted by lower alkyl, phenyl or halogen, R^{1'} is the formula: -OR^{10'}, R^{1a'} is a lower alkyl group optionally having substituents, and Ar' is a phenyl group optionally having substituents,
[4] the compound of [3] wherein R^{1a'} is an ethyl group,
[5] the compound of [3] wherein Ar' is a halogenophenyl group, a lower alkylphenyl group, or a phenyl group substituted by halogen and lower alkyl,
[6] the compound of [3] wherein Ar' is a group represented by the formula: 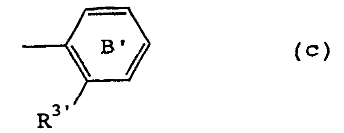 wherein R^{3'} is a halogen atom or a lower alkyl group and ring B' is optionally further substituted by halogen atom,
[7] the compound of [1] wherein the group represented by the formula: 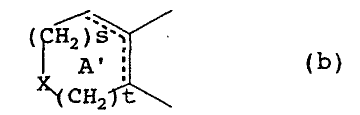 is a group represented by the formula: 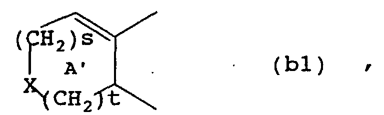
[8] the compound of [6] wherein Ar' is a group represented by the formula: 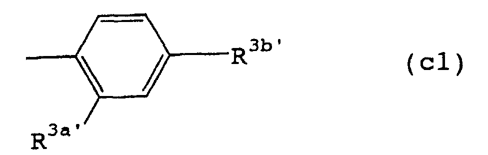 wherein R^{3a'} and R^{3b'} are the same or different and each is a halogen atom,
[9] the compound of [1] wherein R^{1'} is a group represented by the formula: -OR^{1a'} (R^{1a'} is a hydrogen atom, or an aliphatic hydrocarbon group optionally having substituents), s is 1, and t is 1,
[10] the compound of [1] wherein R^{1'} is a group represented by the formula: -OR^{1a'} (R^{1a'} is a C₁₋₆ alkyl group), the group represented by the formula: 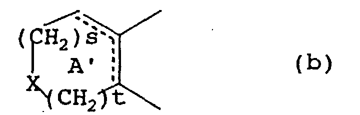 is a group represented by the formula: 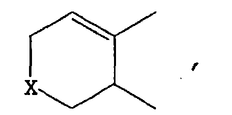 X is methylene or an oxygen atom, Y is methylene or NH, Ar' is a phenyl group optionally having 1 or 2 substituents selected from the group consisting of halogen atom and C₁₋₆ alkoxy,
[11] the compound of [1] wherein R^{1'} is a group represented by the formula: -OR^{1a'} (R^{1a'} is a C₁₋₆ alkyl group), the group represented by the formula: 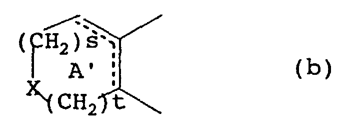 is a group represented by the formula: 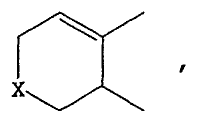 X is methylene and Y is methylene, or X is an oxygen atom and
   Y is NH, and Ar' is a phenyl group optionally having 2 halogen atoms (e.g., 2-chloro-4-fluorophenyl group and the like), or
[12] ethyl 6-[(2-chloro-4-fluorobenzyl)sulfonyl]-1-cyclohexene-1-carboxylate, ethyl (+)-6-[(2-chloro-4-fluorobenzyl)sulfonyl]-1-cyclohexene-1-carboxylate or ethyl 3-[(2-chloro-4-fluorophenyl)sulfamoyl]-3,6-dihydro-2H-pyran-4-carboxylate.

In the present specification, R^{1'} is an aliphatic hydrocarbon group optionally having substituents, an aromatic hydrocarbon group optionally having substituents, a heterocyclic group optionally having substituents, a group represented by the formula: -OR^{1a'} or a group represented by the formula (a), with particular preference given to a group represented by the formula: -OR^{1a'}.

As the "aliphatic hydrocarbon group optionally having substituents" "aromatic hydrocarbon group optionally having substituents", "heterocyclic group optionally having substituents" and "group represented by the formula: -OR^{1a'}" represented by R^{1'}, those similar to these substituents for R can be used.

As the "aliphatic hydrocarbon group optionally having substituents" represented by R^{1a'}, for example, those similar to the aforementioned "aliphatic hydrocarbon group optionally having substituents" represented by R can be used. As R^{1a'}, for example, C₁-C₆ lower alkyl group optionally having substituents (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As the "aliphatic hydrocarbon group optionally having substituents" represented by R^{1b'} and R^{1c'}, for example, those similar to the aforementioned "aliphatic hydrocarbon group optionally having substituents" represented by R can be used. As R^{1b'} and R^{1c'}, for example, a C₁-C₆ lower alkyl group optionally having substituents (e.g., methyl group, ethyl group, n-propyl group, isopropyl group, n-butyl group, isobutyl group, tert-butoxycarbonylmethyl group, hydroxyethyl group and the like) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As R^{1'}, for example, a C₁-C₆ lower alkyl group optionally having substituents (e.g., methyl group, ethyl group, n-propyl group, isopropyl group, n-butyl group, isobutyl group, tert-butoxycarbonylmethyl group, hydroxyethyl group etc.) and the like are preferably used. Of these, for example, methyl group, ethyl group, n-propyl group, isopropyl group, n-butyl group, isobutyl group and the like are preferably used. Particularly, for example, methyl group, ethyl group, n-propyl group and the like are preferable, and ethyl group and the like are specifically preferable.

As the "substituent" of the "methylene group optionally having substituents" represented by Y, for example, C₁₋₆ alkyl group (e.g., methyl group, ethyl group, n-propyl group, isopropyl group, n-butyl group, isobutyl group and the like), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted methylene is particularly preferable.

As the "substituent" of the "NH optionally having substituents" represented by Y, C₁₋₆ alkyl group (e.g., methyl group, ethyl group, n-propyl group, isopropyl group, n-butyl group, isobutyl group etc.), hydroxy-substituted-C₁₋₆ alkyl group (e.g., hydroxymethyl group, hydroxyethyl group etc.), C₁₋₄ alkoxy-carbonyl-C₁₋₄ alkyl group (e.g., methoxycarbonylmethyl group, ethoxycarbonylmethyl group, tert-butoxycarbonylmethyl group, methoxycarbonylethyl group, ethoxycarbonylethyl group, tert-butoxycarbonylethyl group etc.) and the like can be mentioned. Of these, methyl group is preferable. Unsubstituted NH is particularly preferable.

In the "aromatic hydrocarbon group optionally having substituents" represented by Ar', those similar to the "aromatic hydrocarbon group optionally having substituents" for Ar can be used.

Particularly, as Ar', those similar to Ar are preferable. Among others, a group represented by the formula (c) is preferable, and a group represented by the formula (c1) is more preferable.

As the halogen atom represented by R^{3'} in the formula (c), halogen atom which is a substituent of ring B' in the formula (c) and the halogen atom represented by R^{3a'} and R^{3b'} in the formula (c1), fluorine atom and chlorine atom are preferable. As the lower alkyl group represented by R^{3'} in the formula (c), for example, C₁₋₄ alkyl group such as methyl, ethyl, propyl and the like can be mentioned. Of the groups represented by the formula (c), a 2,4-difluorophenyl group, a 2-chloro-4-fluorophenyl group, a 2-methyl-4-chlorophenyl group and the like are preferable. Of the groups represented by the formula (c1), a 2,4-difluorophenyl group, a 2-chloro-4-fluorophenyl group and the like are preferable.

X represents a methylene group, NH, a sulfur atom or an oxygen atom, wherein NH, a sulfur atom and an oxygen atom are preferable.

Ring A' is a 5 to 8-membered ring substituted by a group represented by the formula: -CO-R^{1'} wherein R^{1'} is as defined above and a group represented by the formula: -SO₂-Y-Ar' wherein Y and Ar' are as defined above, and optionally further substituted by 1 to 4 substituents selected from the group consisting of (i) an aliphatic hydrocarbon group optionally having substituents, (ii) an aromatic hydrocarbon group optionally having substituents, (iii) a group represented by the formula: -OR^{2'} wherein R^{2'} is as defined above and (iv) a halogen atom, with preference given to a 5 to 8-membered ring optionally substituted by 1 to 4 substituents selected from (i) an aliphatic hydrocarbon group optionally having substituents, (ii) an aromatic hydrocarbon group optionally having substituents and (iv) a halogen atom.

These substituents are substitutable at substitutable positions on the ring A'. When X constituting the ring is NH or a methylene group, they can substitute the NH and methylene group. When ring A' is substituted by plural substituents, the kinds of such substituents may be the same or different. In addition, two substituents may substitute on the same carbon atom.

As the "aliphatic hydrocarbon group optionally having substituents" and "aromatic hydrocarbon group optionally having substituents", which are substituents of ring A', for example, those similar to the aforementioned groups for R can be mentioned.

As the "aliphatic hydrocarbon group optionally having substituents" for R^{2'}, for example, those similar to the aforementioned groups for R can be mentioned.

As the substituent for ring A', 1 or 2 C₁₋₆ alkyl groups (e.g., C₁₋₄ alkyl group such as methyl group, tert-butyl group etc.), phenyl groups, halogen atoms (e.g., fluorine, chlorine, bromine, iodine etc.) and the like are preferably used.

The "s" is an integer of 0 to 2, "t" is an integer of 1 to 3, and the total of "s" and "t" is not more than 4, with preference given to "s" being 1 and "t" being 1.

As the compound represented by the formula (II), for example, the following compounds and the like are preferable.
(1) Compound (II) wherein R^{1'} is a group represented by the formula: -OR^{1a'} (R^{1a'} is C₁₋₆ alkyl group),
   the group represented by the formula: 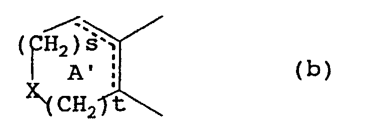 is a group represented by the formula: 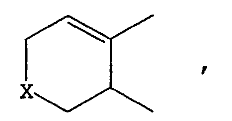 X is methylene or an oxygen atom,
   Y is methylene or NH, and
   Ar' is a phenyl group optionally having 1 or 2 substituents selected from the group consisting of a halogen atom and a C₁₋₆ alkoxy.
(2) Compound (II) wherein R^{1'} is a group represented by the formula: -OR^{1a'} (R^{1a'} is C₁₋₆ alkyl group),
   the group represented by the formula: 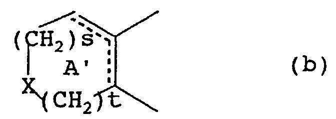 is a group represented by the formula: 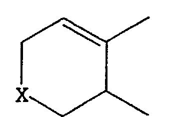 X and Y are each methylene, or X is an oxygen atom and Y is NH, and
   Ar' is a phenyl group optionally having two halogen atoms (e.g., 2-chloro-4-fluorophenyl group and the like).
(3) Ethyl 6-(benzylsulfonyl)-1-cyclohexene-1-carboxylate (compound 1),
   ethyl 6-[(4-methoxybenzyl)sulfonyl]-1-cyclohexene-1-carboxylate (compound 2),
   ethyl 6-[(2,4-difluorobenzyl)sulfonyl]-1-cyclohexene-1-carboxylate (compound 3),
   ethyl 6-[(2-chloro-4-fluorobenzyl)sulfonyl]-1-cyclohexene-1-carboxylate (compound 4),
   ethyl (-)-6-[(2-chloro-4-fluorobenzyl)sulfonyl]-1-cyclohexene-1-carboxylate (compound 5),
   ethyl (+)-6-[(2-chloro-4-fluorobenzyl)sulfonyl]-1-cyclohexene-1-carboxylate (compound 6),
   ethyl 3-[(2,4-difluorophenyl)sulfamoyl]-3,6-dihydro-2H-pyran-4-carboxylate (compound 7), and
   ethyl 3-[(2-chloro-4-fluorophenyl)sulfamoyl]-3,6-dihydro-2H-pyran-4-carboxylate (compound 8).
(4) Ethyl 6-[(2-chloro-4-fluorobenzyl)sulfonyl]-1-cyclohexene-1-carboxylate (compound 4),
   ethyl (+)-6-[(2-chloro-4-fluorobenzyl)sulfonyl]-1-cyclohexene-1-carboxylate (compound 6), and ethyl 3-[(2-chloro-4-fluorophenyl)sulfamoyl]-3,6-dihydro-2H-pyran-4-carboxylate (compound 8).

When the compounds represented by the formulas (I), (Iaa), (Ibb), (Icc), (Ia), (Ib), (Ic), (Id), (Ie), (If), (Ig) and (II) have stereoisomers, each stereoisomer and a mixture of these stereoisomers are encompassed in the present invention.

Furthermore, when the compound represented by the formula (Iaa) is a compound represented by the formula (Icc) or (Inn), the compound represented by the formula (Ia) is a compound represented by the formula (Ic) or (In), the compound represented by the formula (Ie) is a compound represented by the formula (Ik) or (Ip), the compound represented by the formula (Id) is a compound represented by the formula (Ir), the compound represented by the formula (Ig) is a compound represented by the formula (It), and the formula (b) of the compound represented by the formula (II) is the formula (b1) and s and t are 1, each has an optical isomer based on the asymmetric carbon in cycloalkene or cyclohexene ring. Such optical isomer and a mixture of such optical isomers are both encompassed in the present invention.

The compounds (I), (Iaa), (Ia), (Ib), (Ic), (Id), (Ie), (If), (Ig), (Ibb), (Icc) and (II) (hereinafter to be simply referred to as a Compound A), which are used for the pharmaceutical composition of the present invention, may be converted into a salt with an inorganic base, organic base, inorganic acid, organic acid, basic or acidic amino acid, and the like. The salt with an inorganic base may, for example, be used an alkaline metal salt such as sodium and potassium salts, etc.; an alkaline earth metal salt such as calcium and magnesium salts, etc.; aluminum salt; ammonium salt; and the like. The salt with an organic base may, for example, be used a salt with trimethylamine, triethylamine, pyridine, picoline, ethanolamine, diethanolamine, triethanolamine, dicyclohexylamine, N,N'-dibenzylethylenediamine, etc. The salt with an inorganic acid may, for example, be used a salt with hydrochloric acid, hydrobromic acid, nitric acid, sulfuric acid, phosphoric acid, etc. The salt with an organic acid may, for example, be used a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, and the like. The salt with a basic amino acid may, for example, be used a salt with arginine, lysine, ornithine, etc. The salt with acidic amino acid may, for example, be used a salt with aspartic acid, glutamic acid, and the like.

A prodrug of Compound A or a salt thereof is a compound which is converted into Compound A as a result of a reaction with an enzyme, gastric acid etc. under physiological conditions in vivo. Thus, the compound is converted into Compound A by enzymatical oxidation, reduction, hydrolysis etc., by hydrolysis due to gastric acid etc. A prodrug of Compound A may be a compound obtained by subjecting an amino group of Compound A to an acylation, alkylation or phosphorylation (e.g., a compound obtained by subjecting an amino group of Compound A to an eicosanoylation, alanylation, pentylaminocarbonylation, 2-hydroxypropionylation, 2-acetoxypropionylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group in Compound A to an acylation, alkylation, phosphorylation and boration (e.g., a compound obtained by subjecting a hydroxy group of Compound A to an acetylation, palmitoylation, propanoylation, pivaloylation, succinylation, fumarylation, alanylation, dimethylaminomethylcarbonylation, etc.); a compound obtained by subjecting a carboxyl group of Compound A to an esterification or amidation (e.g., a compound obtained by subjecting a carboxyl group of Compound A to an ethyl-esterification, phenyl-esterification, carboxymethyl-esterification, dimethylaminomethyl-esterification, pivaloyloxymethyl-esterification, ethoxycarbonyloxyethyl-esterification, phthalidyl-esterification, (5-methyl-2-oxo-1,3-dioxolen-4-yl)methyl-esterification, cyclohexyloxycarbonylethyl-esterification and methylamidation, etc.) and the like. Any of these compounds can be produced from Compound A by a method known *per se.*

A prodrug of Compound A may also be one which is converted into Compound A under a physiological condition, such as those described in "IYAKUHIN no KAIHATSU (Development of Pharmaceuticals)", Vol. 7, Design of Molecules, p. 163-198, Published by HIROKAWA SHOTEN (1990).

The compound (I), a salt thereof and a prodrug thereof can be produced according to a method known *per se,* for example, a production method described in WO99/46242 or a method analogous thereto. The compound (II), a salt thereof and a prodrug thereof can be produced according to a method known *per se,* for example, a production method described in WO01/10826 or a method analogous thereto.

Of the compounds A, a crystal of d-ethyl 6-[N-(2-chloro-4-fluorophenyl)sulfamoyl]-1-cyclohexene-1-carboxylate (ethyl (6R)-6-[(2-chloro-4-fluoroanilino)sulfonyl]-1-cyclohexene-1-carboxylate)(hereinafter sometimes abbreviated as "crystal of compound B") can be produced by crystallization of d-ethyl 6-[N-(2-chloro-4-fluorophenyl)sulfamoyl]-1-cyclohexene-1-carboxylate (ethyl (6R)-6-[(2-chloro-4-fluoroanilino)sulfonyl]-1-cyclohexene-1-carboxylate) according to a method known *per se.*

As a method for such crystallization, for example, crystallization from solution, crystallization from vapor and crystallization from a molten form can be mentioned.

As the method for the "crystallization from solution", for example, concentration methods, slow cooling methods, reaction methods (diffusion method, electrolysis method), hydrothermal growth methods, fusing agent methods and the like can be mentioned. As the solvent to be used, for example, aromatic hydrocarbons (e.g., benzene, toluene, xylene etc.), halogenated hydrocarbons (e.g., dichloromethane, chloroform etc.), saturated hydrocarbons (e.g., hexane, heptane, cyclohexane etc.), ethers (e.g., diethyl ether, diisopropyl ether, tetrahydrofuran, dioxane etc.), nitriles (e.g., acetonitrile etc.), ketones (e.g., acetone etc.), sulfoxides (e.g., dimethyl sulfoxide etc.), acid amides (e.g., N,N-dimethylformamide etc.), esters (e.g., ethyl acetate etc.), alcohols (e.g., methanol, ethanol, isopropyl alcohol etc.), water and the like can be mentioned. These solvents are used alone or upon mixing two or more kinds at a suitable ratio (e.g., 1:1 to 1:100). Preferably, ethanol, methanol, isopropyl alcohol, hexane, heptane, diisopropyl ether, ethyl acetate, water, and the like are used, where these solvents are used alone or upon mixing two or more kinds at a suitable ratio (e.g., 1:1 to 1:100).

As the method for the "crystallization from vapor", for example, gasification methods (sealed tube method, gas stream method), gas phase reaction methods, chemical transportation methods and the like can be mentioned.

As the method for the "crystallization from a molten form", for example, normal freezing methods (pulling-up method, temperature gradient method, Bridgman method), zone melting methods (zone leveling method, float zone method), special growth methods (VLS method, liquid phase epitaxis method) and the like can be mentioned.

As the method for analysis of the obtained crystals, crystal analysis methods by X-ray diffraction are generally used. Furthermore, as the method for determining the crystal orientation, mechanical methods, optical methods and the like can be mentioned.

The d-ethyl 6-[N-(2-chloro-4-fluorophenyl)sulfamoyl]-1-cyclohexene-1-carboxylate (ethyl (6R)-6-[(2-chloro-4-fluoroanilino)sulfonyl]-1-cyclohexene-1-carboxylate) and a salt thereof are known substances and can be produced by, for example, the method described in WO99/46242 or a method analogous thereto. By applying this to the above-mentioned crystallization methods, crystal of compound B can be obtained.

The crystals of compound B have a melting point of, for example, about 60°C or higher, preferably about 65°C - about 75°C, more preferably about 67°C - about 70°C, and show a diffraction pattern having characteristic peaks at lattice spacing (d value) of about 10.3, about 9.28, about 6.72, about 5.89, about 5.16, about 4.54, about 4.38, about 3.59, about 3.52, about 3.45 and about 3.39 angstroms in powder X-ray diffraction.

The crystals of compound B have high purity (purity 99% or above) and high quality, low hygroscopicity and are free of denaturation even after long term preservation under general conditions, and extremely superior in stability.

A compound represented by the formula 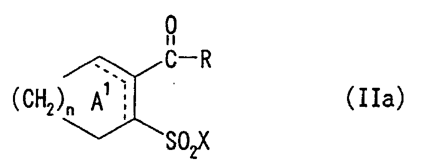 wherein X is a halogen atom, and other symbols are as defined above, or a salt thereof, which is a synthetic intermediate of compound (I) or a salt thereof, can be produced by reacting a compound represented by the formula 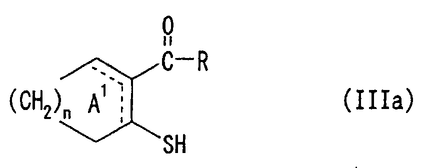 wherein each symbol is as defined above, or a salt thereof with a halogenating agent.

As the halogenating agent, halogen atoms (e.g., chlorine atom, bromine atom, iodine atom etc.), hydrohalogenic acid (e.g., hydrogen chloride, hydrogen bromide etc.), organic acid chlorides (e.g., oxalyl chloride etc.), N-haloamides (e.g., N-chlorosuccinamide, N-bromosuccinamide etc.), halogenomeldrum's acid (e.g., dibromomeldrum's acid etc.), thionyl chloride and the like can be mentioned.

The amount of the halogenating agent to be used is generally 1.0-15 equivalents relative to compound (IIIa) or a salt thereof.

As the reaction solvent, for example, sulfoxides (e.g., dimethyl sulfoxide etc.), ethers (e.g., diethyl ether, dioxane etc.), nitriles (e.g., acetonitrile etc.), aromatic hydrocarbons (e.g., benzene, toluene, xylene etc.), halogenated hydrocarbons (e.g., dichloromethane, chloroform, 1,2-dichloroethane etc.), esters (e.g., ethyl acetate etc.), amides (e.g., dimethylformamide, acetamide, dimethylacetamide, 1,3-dimethyl-2-imidazoline, 1-methyl-2-pyrrolidone etc.), organic acids (e.g., acetic acid etc.), water and the like are used. These solvents can be used alone or in a mixture.

The reaction temperature is generally -50°C to 100°C, preferably -30°C to 40°C.

The reaction time is generally 0.1-6 hrs, preferably 0.3-3 hrs.

When the optically active compound or a salt thereof contains an enantiomer, general separation means may be applied such as diastereomeric salt methods wherein a salt with an optically active acid (e.g., camphor sulfonic acid etc.) or optically active base (e.g., 1-methylbenzylamine etc.) is formed, inclusion compound methods using an optically active host molecule (e.g., 1,6-bis(2-chlorophenyl)-1,6-diphenylhexa-2,4-diyn-1,6-diol), various chromatographies (e.g., liquid chromatography using an optically active column etc.), fractional recrystallization and the like, whereby an . optically pure compound can be obtained.

The Compound A, a salt thereof and a prodrug thereof may be a hydrate or non-hydrate.

The Compound A, a salt thereof and a prodrug thereof may be labeled with an isotope (e.g., ³H, ¹⁴C, ³⁵S, ¹²⁵I etc.) and the like.

Compound A is highly safe for humans and can be used for mammals (e.g., rat, mouse, guinea pig, monkey, cattle, dog, pig, human and the like) as a pharmaceutical agent (e.g., agent for prophylaxis or therapy of various diseases), veterinary drugs and the like.

Since Compound A has low toxicity, a nitric oxide (NO) production-inhibitory effect and an inhibitory effect on the production of inflammatory cytokines such as TNF-_{α}, IL-1, IL-6, etc., Compound A is useful as a therapeutic and/or prophylactic agent in a mammal (e.g., cat, cattle, dog, horse, goat, monkey, human and the like) against diseases such as cardiac disease, autoimmune disease, inflammatory disease, central nervous system disease, infectious disease, sepsis, septic shock, immune dysfunction and the like, including, for example, septicemia, endotoxin shock, exotoxin shock, systemic inflammatory response syndrome (SIRS), compensatory anti-inflammatory response syndrome (CARS), burn, trauma, post-operative complications, cardiac deficiency, shock, hypotension, rheumatoid arthritis, osteoarthritis, gastritis, ulcerative colitis, peptic ulcer, stress-induced gastric ulcer, Crohn's disease, autoimmune disease, post-transplant tissue failure and rejection, postischemic re-perfusion failure, acute coronary microvascular embolism, shock-induced vascular embolism (disseminated intravascular coagulation (DIC) and the like), ischemic cerebral disorder, arterial sclerosis, pernicious anemia, Fanconi's anemia, drepanocythemia, pancreatitis, nephrose syndrome, nephritis, renal failure, insulin-dependent diabetes, insulin-independent diabetes, hepatic porphyria, alcoholism, Parkinson's disease, chronic leukemia, acute leukemia, tumor, myeloma, alleviation of side effects caused by anticancer agents, infantile and adult respiratory distress syndrome, pulmonary emphysema, dementia, Alzheimer's disease, multiple sclerosis, vitamin E deficiency, aging, sunburn, muscular dystrophy, myocarditis, cardiomyopathy, myocardial infarction, myocardial post infarction syndrome, osteoporosis, pneumonia, hepatitis, psoriasis, pain, cataract, influenza infection, malaria, human immunodeficiency virus (HIV) infection, radiation hazard, burn, in vitro fertilization efficiency, hypercalcemia, tonic spondylitis, osteopenia, bone Paget's disease, osteomalacia, fracture, acute bacterial meningitis, *Helicobacter pylori* infection, invasive staphylococcal infection, tuberculosis, systemic mycosis, herpes simplex virus infection, varicella-zoster virus infection, human papilloma virus infection, acute viral encephalitis, encephalitis, meningitis, immune dysfunction due to infections, asthma, atopic dermatitis, allergic rhinitis, reflux esophargitis, fever, hyper cholesteremia, hyperglycemia, hyperlipidemia, diabetic complication, diabetic renal disease, diabetic neuropathy, diabetic retinopathy, gout, gastric atony, hemorrhoid, systemic lupus erythematosus, spinal damage, insomnia, schizophrenia, epilepsy, cirrhosis, hepatic failure, instable angina, valvular disease, dialysis-induced thrombocytopenia, acute ischemic cerebral apoplexy, acute cerebral thrombosis, cancer metastasis, urinary bladder cancer, mammary cancer, uterine cervical cancer, colon cancer, gastric cancer, ovarian cancer, prostatic cancer, parvicellular pulmonary cancer, non-parvicellular pulmonary cancer, malignant melanoma, Hodgkin's disease, non-Hodgkin lymphoma, side effects caused by administration of immunosuppressants and the like. Particularly, it is useful as a prophylactic or therapeutic agent against sepsis, septic shock and the like.

Accordingly, the combination drug of the present invention, which contains an anti-sepsis drug such as Compound A or a salt thereof or a prodrug thereof and the like, is useful as a therapeutic and/or a prophylactic agent in a mammal (e.g., cat, cattle, dog, horse, goat, monkey, human and the like) against diseases such as cardiac disease, autoimmune disease, inflammatory disease, central nervous system disease, infectious disease, sepsis, septic shock, immune dysfunction and the like, including, for example, septicemia, endotoxin shock, exotoxin shock, systemic inflammatory response syndrome (SIRS), compensatory anti-inflammatory response syndrome (CARS), burn, trauma, post-operative complications, cardiac deficiency, shock, hypotension, rheumatoid arthritis, osteoarthritis, gastritis, ulcerative colitis, peptic ulcer, stress-induced gastric ulcer, Crohn's disease, autoimmune disease, post-transplant tissue failure and rejection, postischemic re-perfusion failure, acute coronary microvascular embolism, shock-induced vascular embolism (disseminated intravascular coagulation (DIC) and the like), ischemic cerebral disorder, arterial sclerosis, pernicious anemia, Fanconi's anemia, drepanocythemia, pancreatitis, nephrose syndrome, nephritis, renal failure, insulin-dependent diabetes, insulin-independent diabetes, hepatic porphyria, alcoholism, Parkinson's disease, chronic leukemia, acute leukemia, tumor, myeloma, infantile and adult respiratory distress syndrome, pulmonary emphysema, dementia, Alzheimer's disease, multiple sclerosis, vitamin E deficiency, aging, sunburn, muscular dystrophy, myocarditis, cardiomyopathy, myocardial infarction, myocardial post infarction syndrome, osteoporosis, pneumonia, hepatitis, psoriasis, pain, cataract, influenza infection, malaria, human immunodeficiency virus (HIV) infection, radiation hazard, burn, in vitro fertilization efficiency, hypercalcemia, tonic spondylitis, osteopenia, bone Paget's disease, osteomalacia, fracture, acute bacterial meningitis, *Helicobacter pylori* infection, invasive staphylococcal infection, tuberculosis, systemic mycosis, herpes simplex virus infection, varicella-zoster virus infection, human papilloma virus infection, acute viral encephalitis, encephalitis, meningitis, immune dysfunction due to infections, asthma, atopic dermatitis, allergic rhinitis, reflux esophargitis, fever, hyper cholesteremia, hyperglycemia, hyperlipidemia, diabetic complication, diabetic renal disease, diabetic neuropathy, diabetic retinopathy, gout, gastric atony, hemorrhoid, systemic lupus erythematosus, spinal damage, insomnia, schizophrenia, epilepsy, cirrhosis, hepatic failure, instable angina, valvular disease, dialysis-induced thrombocytopenia, acute ischemic cerebral apoplexy, acute cerebral thrombosis, cancer metastasis, urinary bladder cancer, mammary cancer, uterine cervical cancer, colon cancer, gastric cancer, ovarian cancer, prostatic cancer, parvicellular pulmonary cancer, non-parvicellular pulmonary cancer, malignant melanoma, Hodgkin's disease, non-Hodgkin lymphoma, side effects due to the administration of anticancer agents and immunosuppressants. Particularly, it is useful as a prophylactic or therapeutic agent against sepsis, septic shock and the like.

The drugs that can be used concurrently with Compound A (hereinafter sometimes to be briefly referred to as a drug in combination) are, for example, antibacterial agent, antifungal agent, non-steroidal antiinflammatory drug, steroid, anticoagulant, antithrombotic drug, thrombolytic drug, immunomodulator, antiprotozoal, antibiotic, antiviral agent, antitussive and expectorant drug, sedative, anesthetic, antiulcer drug, antiarrhythmic, hypotensive diuretic, tranquilizer, antipsychotic, antitumor drug, hypolipidemic drug, muscle relaxant, anticonvulsant, antidepressant, antiallergic drug, cardiac, antiarrhythmic, vasodilator, vasoconstrictor, hypotensive diuretic, antidiabetic drug, antinarcotic, vitamin, vitamin derivative, therapeutic agent for arthritis, antirheumatic, antiasthmatic, therapeutic agent for pollakisuria/anischuria, therapeutic agent for atopic dermatitis, therapeutic agent for allergic rhinitis, hypertensor, protease drug, protease inhibitor, anti-AIDS drug, anti-sepsis drug, anti-septic shock drug, endotoxin-antagonist or -antibody, signal transduction inhibitor, inhibitor of inflammatory mediator activity, antibody to inhibit inflammatory mediator activity, inhibitor of inflammatory mediator production, inhibitor of anti-inflammatory mediator activity, antibody to inhibit anti-inflammatory mediator activity, inhibitor of anti-inflammatory mediator, α1-adrenergic stimulating agent, and the like. Of these, antibacterial agent, antifungal agent, non-steroidal antiinflammatory drug, steroid, anticoagulant and the like are preferable. Specific examples thereof include the following.
(1) antibacterial agent
   (A) sulfa drug
      sulfamethizole, sulfisoxazole, sulfamonomethoxine, sulfamethizole, salazosulfapyridine, silver sulfadiazine and the like.
   (B) quinoline antibacterial agent
      nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   (C) antiphthisic
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   (D) antiacidfast bacterium drug
      diaphenylsulfone, rifampicin and the like.
   (E) antiviral drug
      idoxuridine, acyclovir, vidarabine, ganciclovir and the like.
   (F) anti-HIV agent
      zidovudine, didanosine, zalcitabine, indinavir sulfate ethanolate, ritonavir and the like.
   (G) antispirochetele
   (H) antibiotic
      tetracycline hydrochloride, ampicillin, piperacillin, gentamicin, dibekacin, kanendomycin, lividomycin, tobramycin, amikacin, fradiomycin, sisomycin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)] and the like.
(2) antifungal agent
   (A) polyethylene antibiotic (e.g., amphotericin B, nystatin, trichomycin).
   (B) griseofulvin, pyrrolnitrin and the like.
   (C) cytosine metabolism antagonist (e.g., flucytosine)
   (D) imidazole derivative (e.g., econazole, clotrimazole, miconazole nitrate, bifonazole, croconazole)
   (E) triazole derivative (e.g. fluconazole, itraconazole, azole compound [2-[(lR,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone]
   (F) thiocarbamic acid derivative (e.g. trinaphthol)
   (G) echinocandin derivative (e.g., caspofungin, micafungin, anidulafungin) and the like.
(3) non-steroidal antiinflammatory drug
   acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, epirizole, tiaramide hydrochloride, zaltoprofen, gabexate mesilate, camostat mesilate, urinastatin, colchicine, probenecid, sulfinpyrazone, benzbromarone, allopurinol, gold sodium thiomalate, sodium hyaluronate, sodium salicylate, morphine hydrochloride, salicylic acid, atropine, scopolamine, morphine, pethidine, levorphanol, ketoprofen, naproxen, oxymorphone or a salt thereof, and the like.
(4) steroid
   dexamethasone, hexestrol, methimazole, betamethasone, triamcinolone, triamcinolone acetonide, fluocinonide, fluocinolone acetonide, prednisolone, methylprednisolone, cortisone acetate, hydrocortisone, fluorometholone, beclometasone propionate, estriol and the like.
(5) anticoagulant
   heparin sodium, sodium citrate, activated protein C, tissue factor pathway inhibitor, antithrombin III, dalteparin sodium, warfarin potassium, argatroban, gabexate, sodium citrate and the like.
(6) antithrombotic drug
   ozagrel sodium, ethyl icosapentate, beraprost sodium, alprostadil, ticlopidine hydrochloride, pentoxifylline, dipyridamole and the like.
(7) thrombolytic drug
   tisokinase, urokinase, streptokinase and the like.
(8) immunomodulator
   cyclosporin, tacrolimus, gusperimus, azathioprine, antilymphocyte serum, dried sulfonated immunoglobulin, erythropoietin, colony-stimulating factor, interleukin, interferon and the like.
(9) antiprotozoal
   metronidazole, tinidazole, diethylcarbamazine citrate, quinine hydrochloride, quinine sulfate and the like.
(10) antitussive and expectorant drug
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, chloperastine, protokylol, isoproterenol, salbutamol, terbutaline, oximetebanol, morphine hydrochloride, dextromethorphan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(11) sedative
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(12) anesthetic
   (12-1) local anesthetic
   cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine) and the like.
   (12-2) general anesthetic
   (A) inhalation anesthetic (e.g., ether, halothane, nitrous oxide, isoflurane, enflurane),
   (B) intravenous anesthetic (e.g., ketamine hydrochloride, droperidol, thiopental sodium, thiamylal sodium, pentobarbital) and the like.
(13) antiulcer drug
   metoclopromide, histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.
(14) antiarrhythmic
   (A) Na channel blocker (e.g., quinidine, procainamide, disopyramide, ajmaline, lidocaine, mexiletine, phenitoin),
   (B) β-blocker (e.g., propranolol, alprenolol, bufetolol, oxprenolol, atenolol, acebutolol, metoprolol, bisoprolol, pindolol, carteolol, arotinolol),
   (C) K channel blocker (e.g., amiodarone),
   (D) Ca channel blocker (e.g., verapamil, diltiazem) and the like.
(15) hypotensive diuretic
   hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophyllin and the like.
(16) tranquilizer
   diazepam, lorazepam, oxazepam, chlordiazepoxide, medazepam, oxazolam, cloxazolam, clotiazepam, bromazepam, etizolam, fludiazepam, hydroxyzine and the like.
(17) antipsychotic
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(18) antitumor drug
   6-O-(N-chloroacetylcarbamoyl) fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulphan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestol, chlormadinone acetate, leuprorelin acetate, buserelin acetate and the like.
(19) hypolipidemic drug
   clofibrate, ethyl 2-chloro-3-[4-(2-methyl-2-phenylpropoxy)-phenyl]propionate [Chemical and Pharmaceutical Bulletin (Chem. Pharm. Bull.), 38, 2792-2796 (1990)], pravastatin, simvastatin, probucol, bezafibrate, clinofibrate, nicomol, cholestyramine, dextran sulfate sodium and the like.
(20) muscle relaxant
   pridinol, tubocurarine, pancuronium, tolperisone hydrochloride, chlorphenesin carbamate, baclofen, chlormezanone, mephenesin, chlorzoxazone, eperisone, tizanidine and the like.
(21) anticonvulsant
   phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(22) antidepressant
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(23) antiallergic drug
   diphenhydramine, chlorpheniramine, tripelennamine, metodilamine, clemizole, diphenylpyraline, methoxyphenamine, sodium cromoglycate, tranilast, repirinast, amlexanox, ibudilast, ketotifen, terfenadine, mequitazine, azelastine, epinastine, ozagrel hydrochloride, pranlkast hydrate, seratrodast and the like.
(24) cardiac
   trans-pi-oxocamphor, terephyllol, aminophyllin, etilefrine, dopamine, dobutamine, denopamine, aminophyllin, bencirin, amrinone, pimobendan, ubidecarenone, digitoxin, digoxin, methyldigoxin, lanatoside C, G-strophanthin and the like.
(25) vasodilator
   oxyfedrine, diltiazem, tolazoline, hexobendine, bamethan, clonidine, methyldopa, guanabenz and the like.
(26) vasoconstrictor
   dopamine, dobutamine, denopamine and the like.
(27) hypotensive diuretic
   hexamethonium bromide, pentolinium, mecamylamine, ecarazine, clonidine, diltiazem, nifedipine and the like.
(28) antidiabetic drug
   tolbutamide, chlorpropamide, acetohexamide, glibenclamide, tolazamide, acarbose, epalrestat, troglitazone, glucagon, glymidine, glipzide, phenformin, buformin, metformin and the like.
(29) antinarcotic
   levallorphan, nalorphine, naloxone or a salt thereof and the like.
(30) vitamin
   (A) vitamin A: vitamin A₁, vitamin A₂ and retinol palmitate
   (B) vitamin D: vitamin D₁, D₂, D₃, D₄ and D₅
   (C) vitamin E:α-tocopherol, β-tocopherol, γ-tocopherol, 6-tocopherol, dl-α-tocopherol nicotinate
   (D) vitamin K: vitamin K₁, K₂, K₃ and K₄
   (E) folic acid (vitamin M) and the like
   (F) vitamin B: vitamin B₁, vitamin B₂, vitamin B₃, vitamin B₅, vitamin B₆ and vitamin B₁₂
   (G) biotin (vitamin H) and the like.
(31) vitamin derivative
   various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like.
(32) antiasthmatic
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, iprotropium bromide, oxitropium bromide, flutropium bromide, theophyline, aminophyllin, sodium cromoglycate, tranilast, repirinast, anrexanone, ibudilast, ketotifen, terfenadine, mequitazine, azelastine, epinastine, ozagrel hydrochloride, pranlkast hydrate, seratrodast, dexamethasone, prednisolone, hydrocortisone, beclometasone dipropionate and the like.
(33) therapeutic agent for pollakisuria/anischuria flavoxate hydrochloride and the like.
(34) therapeutic agent for atopic dermatitis sodium cromoglycate and the like.
(35) therapeutic agent for allergic rhinitis sodium cromoglycate, chlorpheniramine maleate, alimemazine tartrate, clemastine fumarate, homochlorcyclizine hydrochloride, terfenadine, mequitazine and the like.
(36) hypertensor
   dopamine, dobutamine, denopamine, digitoxin, digoxin, methyldigoxin, lanatoside C, G-strophanthin and the like.
(37) Others
   hydroxycam, diaserine, megestrol acetate, nicerogolin, prostaglandins and the like.

A combination drug of the present invention provides the following effects.
(1) The dose of the above-mentioned cyclohexene compound, a salt thereof, a prodrug thereof and a drug in combination can be lower than in the case of a sole administration of the composition of the present invention.
(2) A synergistic therapeutic effect can be achieved against the above-mentioned sepsis, septic shock, inflammatory diseases, infectious diseases and the like.
(3) A broad range of therapeutic effects can be achieved against various diseases developed in association with viral infection and the like.

With regard to the use of a combination drug of the present invention, the Compound A and a drug in combination are free of any limitation on the timing of the administration. The Compound A or a pharmaceutical composition thereof and the drug in combination or a pharmaceutical composition thereof may be simultaneously administered to the administration object, or may be administered with time difference. The dose of the drug in combination follows a clinical dose and can be appropriately determined depending on the administration object, administration route, disease, combination and the like.

The mode of administration of the combination drug of the present invention is not particularly limited, as long as the Compound A and the drug in combination are combined for administration. As the mode of such administration, for example, (1) administration of a single preparation obtained by simultaneous addition of the Compound A or the a pharmaceutical composition thereof and the combination drug, (2) simultaneous administration of two kinds of preparations obtained by separate preparation of the Compound A or a pharmaceutical composition thereof and the drug in combination or a pharmaceutical composition thereof, by a single administration route, (3) time staggered administration of two kinds of preparations obtained by separate preparation of the Compound A or a pharmaceutical composition thereof and the drug in combination or a pharmaceutical composition thereof, by the same administration route, (4) simultaneous administration of two kinds of preparations obtained by separate preparation of the Compound A or a pharmaceutical composition thereof and the drug in combination or a pharmaceutical composition thereof, by different administration routes, (5) time staggered administration of two kinds of preparations obtained by separate preparation of the Compound A or a pharmaceutical composition thereof and the drug in combination or a pharmaceutical composition thereof, by different administration routes, such as administration in the order of the Compound A or the pharmaceutical composition thereof and then the drug in combination, or the pharmaceutical composition thereof in a reversed order, and the like are exemplified.

The combination drug of the present invention has low toxicity. For example, it can be administered safely by admixing the combination drug Compound A and/or the above-mentioned drug in combination with a pharmacologically acceptable carrier according to a method known per se to give a pharmaceutical composition, such as tablets (inclusive of sugar-coated tablets and film-coated tablets), powders, granules, capsules, (inclusive of soft capsules), liquids, injections, suppositories, sustained release agents and the like, for oral or parenteral (e.g., topical, rectal or intravenous administration) administration. An injection can be administered intravenously, intramuscularly, subcutaneously, into the organs or directly into the lesion.

As the pharmacologically acceptable carrier usable for the production of the combination drug in the present invention, there are mentioned various conventional organic or inorganic carriers as a material for the preparation. Examples thereof include excipients, lubricants, binders and disintegrators for solid preparations, and solvents, solubilizers, suspending agents, isotonic agents, buffers, soothing agents, and the like for liquid preparations. Where necessary, conventional additives such as antiseptics, antioxidants, coloring agents, sweeteners, absorbents, moistening agents and the like can be used appropriately in suitable amounts.

As the excipient, there are mentioned, for example, lactose, sucrose, D-mannitol, starch, corn starch, crystalline cellulose, light anhydrous silicic acid and the like.

As the lubricant, there are mentioned, for example, magnesium stearate, calcium stearate, talc, colloidal silica and the like.

As the binder, there are mentioned, for example, crystalline cellulose, sucrose, D-mannitol, dextrin, hydroxypropylcellulose, hydroxypropylmethylcellulose, polyvinylpyrrolidone, starch, sucrose, gelatin, methylcellulose, carboxymethylcellulose sodium and the like.

As the disintegrator, there are mentioned, for example, starch, carboxymethylcellulose, carboxymethylcellulose calcium, sodium carboxymethyl starch, L-hydroxypropylcellulose and the like.

As the solvent, there are mentioned, for example, injectable water, alcohol, propylene glycol, Macrogol, sesame oil, corn oil, olive oil and the like.

As the solubilizer, there are mentioned, for example, polyethylene glycol, propylene glycol, D-mannitol, benzyl benzoate, ethanol, tris-aminomethane, cholesterol, triethanolamine, sodium carbonate, sodium citrate and the like.

As the suspending agent, there are mentioned, for example, surfactants such as stearyl triethanolamine, sodium lauryl sulfate, lauryl aminopropionate, lecithin, benzalkonium chloride, benzethonium chloride, glyceryl monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like, and the like.

As the isotonic agent, there are mentioned, for example, glucose, D-sorbitol, sodium chloride, glycerine, D-mannitol and the like.

As the buffer, there are mentioned, for example, buffers such as phosphate, acetate, carbonate, citrate etc., and the like.

As the soothing agent, there are mentioned, for example, benzyl alcohol and the like.

As the antiseptic, there are mentioned, for example, p-oxybenzoates, chlorobutanol, benzyl alcohol, phenethyl alcohol, dehydroacetic acid, sorbic acid and the like.

As the antioxidant, there are mentioned, for example, sulfite, ascorbic acid, α-tocopherol and the like.

The combination ratio of Compound A and a drug in combination in the combination drug of the present invention can be appropriately determined depending on the administration object, administration route, disease and the like.

The content of the Compound A in the combination drug of the present invention varies depending on the form of the preparation. It is generally about 0.01-100 wt%, preferably about 0.1 - 50 wt%, more preferably about 0.5 - 20 wt%, based on the preparation in total.

The content of the drug in combination in the combination drug of the present invention varies depending on the form of the preparation. It is generally about 0.01 - 100 wt%, preferably about 0.1 - 50 wt%, more preferably about 0.5 - 20 wt%, based on the preparation in total.

The content of the additive, such as a carrier, in the combination drug of the present invention varies depending on the form of the preparation. It is generally about 1-99.99 wt%, preferably about 10-90 wt%, based on the preparation in total.

When the Compound A and the drug in combination are prepared into separate pharmaceutical preparations, the contents as mentioned above can be employed.

The pharmaceutical composition can be produced by a method known per se, which is generally employed for the preparation of a pharmaceutical composition.

For example, a Compound A and a drug in combination can be prepared into an aqueous injection together with a dispersant (e.g., Tween 80 (manufactured by ATLASPOWDER USA), HCO 60 (manufactured by NIKKO CHEMICALS), polyethylene glycol, carboxymethylcellulose, sodium arginate, hydroxypropylmethylcellulose, dextrin and the like), a stabilizing agent (e.g., ascorbic acid, sodium pyrosulfite and the like), a surfactant (e.g., polysorbate 80, Macrogol and the like), a solubilizer (e.g., glycerine, ethanol and the like), a buffering agent (e.g., phosphoric acid, alkali metal salt thereof, citric acid, alkali metal salt thereof and the like), an isotonic agent (e.g., sodium chloride, potassium chloride, mannitol, sorbitol, glucose and the like), a pH adjusting agent (e.g., hydrochloric acid, sodium hydroxide and the like), a preservative (e.g., ethyl p-oxybenzoate, benzoic acid, methyl paraben, propyl paraben, benzyl alcohol and the like), a solubilizer (e.g., conc. glycerine, meglumine and the like), a solubilizer (e.g., propylene glycol, sucrose and the like), a soothing agent (e.g., glucose, benzyl alcohol and the like) and the like, or into an oil-based injection by dissolving, suspending or emulsifying using a vegetable oil such as olive oil, sesame oil, cottonseed oil, corn oil and the like and a solubilizer such as propylene glycol and the like.

An oral formulation can be produced by a method known per se by, for example, compressing a Compound A or a combination drug together with an excipient (e.g., lactose, sucrose, starch and the like), a disintegrant (e.g., starch, calcium carbonate and the like), a binder (e.g., starch, gum arabic, carboxymethyl cellulose, polyvinyl pyrrolidone, hydroxypropyl cellulose and the like), a lubricant (e.g., talc, magnesium stearate, polyethylene glycol 6000 and the like), and the like, followed by, where necessary, a coating process known per se for the purpose of masking a taste, forming an enteric coat, or achieving a sustained release. For such coating may be used, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (manufactured by ROHM, Germany, a copolymer of methacrylic acid and acrylic acid), a dye (e.g., colcothar, titanium dioxide and the like) and the like. The preparation for oral administration may be either a rapid release preparation or a sustained release preparation.

For example, when a suppository is produced, a Compound A or a drug in combination may be formulated also as an oil or aqueous, solid or semi-solid or liquid suppository by a method known per se. An oil base may be, for example, a high fatty acid glyceride (e.g., cocoa butter, WITEPSOL (manufactured by DYNAMIT NOBEL, Germany) and the like), a middle fatty acid (e.g., MYGLYOL (manufactured by DYNAMIT NOBEL, Germany) and the like), a vegetable oil (e.g., sesame oil, soybean oil, cottonseed oil and the like) and the like. An aqueous base may be, for example, polyethylene glycol or propylene glycol, and an aqueous gel base may be, for example, a natural gum, a cellulose derivative, a vinyl polymer, an acrylic polymer and the like.

Examples of the above-mentioned sustained release preparation include sustained release microcapsules and the like.

A sustained release microcapsule can be prepared by a method known per se. For example, a sustained release preparation shown in the following [2] is preferably formed and administered.

A Compound A can be preferably formed into a preparation for oral administration such as a solid preparation (e.g., powder, granule, tablet, capsule) and the like, or a preparation for rectal administration such as a suppository and the like, particularly the preparation for oral administration.

The drug in combination can take the above-mentioned dosage form depending on the kind of the drug.

In the following, [1] an injection of the Compound A or the drug in combination and preparation thereof, [2] a sustained release preparation or a rapid release preparation of the Compound A or the drug in combination and preparation thereof, and [3] a sublingual tablet, buccal or oral cavity rapid disintegrator of the Compound A or the drug in combination and preparation thereof are concretely explained.

### [1] Injection and preparation thereof

An injection containing the Compound A or the drug in combination dissolved in water is preferable. The injection may contain benzoic acid salt and/or salicylic acid salt.

The injection is obtained by dissolving both the Compound A and the drug in combination and, where desired, benzoate and/or salicylate in water. Where necessary, additives, which are described below, can be also added. They may be dissolved in any order, which is performed suitably in the same manner as in conventional production methods for injections.

The salt of the above-mentioned benzoic acid and salicylic acid includes, for example, alkali metal salts such as sodium, potassium and the like, alkaline earth metal salts such as calcium, magnesium and the like, ammonium salt, meglumine salt, and organic acid salt such as trometamol and the like, and the like.

The concentration of the Compound A or the drug in combination in the injection is about 0.5 - 50 w/v%, preferably about 3 ∼ 20 w/v%. The concentration of the benzoic acid salt and/or salicylic acid salt is preferably 0.5 - 50 w/v%, more preferably 3 - 20 w/v%, of the injection.

The injection may contain additives generally used for injections, such as a stabilizing agent (ascorbic acid, sodium pyrosulfite and the like), a surfactant (polysorbate 80, Macrogol and the like), a solubilizing agent (glycerine, ethanol and the like), a buffer (phosphoric acid, alkali metal salt thereof, citric acid, alkali metal salt thereof and the like), an isotonic agent (sodium chloride, potassium chloride and the like), a dispersing agent (hydroxypropylmethylcellulose, dextrin), a pH adjusting agent (hydrochloric acid, sodium hydroxide and the like), a preservative (ethyl p-oxybenzoate, benzoic acid and the like), a solubilizer (e.g., conc. glycerine, meglumine and the like), a solubilizing agent (propylene glycol, sucrose and the like), a soothing agent (e.g., glucose, benzyl alcohol and the like) and the like as appropriate. Generally, these additives are added in a proportion generally employed for injections.

The injection is preferably adjusted to pH 2 - 12, preferably 2.5 - 8.0, by the use of a pH adjusting agent.

The injectable aqueous solution is preferably heated and, in the same manner as with conventional injections, subjected to, for example, sterilization by filtration, high pressure sterilization by heating and the like to provide an injection.

The injectable aqueous solution is preferably subjected to high pressure sterilization by heating at, for example, 100°C - 121°C for 5 min - 30 min.

It may be prepared into an antibacterial solution, so that it can be used as a preparation for plural subdivided administrations.

### [2] Sustained release preparation or rapid release preparation and preparation thereof

A sustained release preparation wherein a core containing the Compound A or the drug in combination is covered on demand with a film forming agent, such as a water-insoluble material, a swellable polymer and the like, is preferable. For example, a sustained release preparation for oral administration once a day is preferable.

The water-insoluble materials to be used for the film forming agent are, for example, cellulose ethers such as ethylcellulose, butylcellulose and the like; cellulose esters such as cellulose acetate, cellulose propionate and the like; polyvinyl esters such as poly(vinyl acetate), poly(vinyl butyrate) and the like; acrylic polymers such as acrylic acid/methacrylic acid copolymer, methyl methacrylate copolymer, ethoxyethyl methacrylate/cinnamoethyl methacrylatelaminoalkyl methacrylate copolymer, polyacrylic acid, polymethacrylic acid, methacrylic acid alkylamide copolymer, poly(methyl methacrylate), polymethacrylate, polymethacrylic amide, aminoalkyl methacrylate copolymer, poly(methacrylic anhydride) and glycidyl methacrylate copolymer, particularly acrylic-polymer such as Eudragits (Rohm Pharma) (e.g., Eudragit RS-100, RL-100, RS-30D, RL-30D, RL-PO, RS-PO (ethyl acrylate·methyl methacrylate·trimethyl chloride methacrylate·ammonium ethyl copolymer), Eudragit NE-30D (methyl methacrylate·ethyl acrylate copolymer) and the like), and the like; hydrogenated oils such as hydrogenated castor oil (e.g., Lovely Wax (Freund Inc.) and the like) and the like; waxes such as carnauba wax, fatty acid glycerine ester, paraffin and the like; polyglycerine fatty acid ester and the like.

As the swellable polymer, polymers, which have an acid dissociable group and show pH-dependent swelling, are preferable, and polymers, which have an acid dissociable group and show less swelling in an acidic range, such as in the stomach, but show more swelling in a neutral range, such as in the small intestine and large intestine, are preferable.

Examples of the polymer, which has an acidic dissociable group, and shows pH-dependent swelling, include crosslinking type polyacrylic acid polymers such as Carbomer 934P, 940, 941, 974P, 980, 1342 and the like, polycarbophil, calcium polycarbophil (all mentioned above are manufactured by BF Goodrich), HI-BIS-WAKO 103, 104, 105, 304 (all manufactured by Waco Pure Chemicals Industries, Ltd.) and the like.

The film forming agent to be used for the sustained release preparation may further contain a hydrophilic material.

Examples of the hydrophilic material include polysaccharides optionally having a sulfuric acid group such as pullulan, dextrin, alkali metal salt of alginic acid and the like; polysaccharides having a hydroxyalkyl group or a carboxyalkyl group such as hydroxypropylcellulose, hydroxypropylmethylcellulose, carboxymethylcellulose sodium and the like; methylcellulose; polyvinylpyrrolidone; polyvinyl alcohol; polyethylene glycol and the like.

The content of the water-insoluble material of the film forming agent for a sustained release preparation is about 30 - about 90% (w/w), preferably about 35 - about 80% (w/w), more preferably about 40 - 75% (w/w), and the content of the swellable polymer is about 3 - about 30% (w/w), preferably about 3 - about 15% (w/w). The film forming agent may further contain a hydrophilic material, in which case the content of the hydrophilic material for film forming agent is not more than about 50% (w/w), preferably about 5 - about 40% (w/w), more preferably about 5 - about 35% (w/w). As used herein, the above-mentioned % (w/w) is a weight percentage relative to the film forming agent composition wherein the solvent (e.g., water, lower alcohol such as methanol, ethanol etc., and the like) has been removed from the film forming liquid agent.

A sustained release preparation is produced by preparing a core containing a drug as exemplarily mentioned below, and then coating the resulting core with a film forming liquid agent prepared by heating or dissolving or dispersing in a solvent a water-insoluble material, a swellable polymer and the like.

### I. Preparation of core containing a drug

The form of the core containing a drug (hereinafter sometimes simply referred to as a core) to be coated with a film forming agent is not particularly limited, but it is preferably formed into particles such as granules, subtilized granules and the like.

When the core is made of granules or subtilized granules, the average particle size thereof is preferably about 150 - 2,000 µm, more preferably about 500 - about 1,400 µm.

The core can be prepared by a typical production method. For example, a drug is mixed with suitable excipients, binders, disintegrators, lubricants, stabilizers and the like, and the mixture is subjected to wet extrusion granulation, fluidized bed granulation and the like.

The drug content of the core is about 0.5 - about 95% (w/w), preferably about 5.0 - about 80% (w/w), more preferably about 30 - about 70% (w/w).

Examples of the excipient to be contained in the core include saccharides such as sucrose, lactose, mannitol, glucose and the like, starch, crystalline cellulose, calcium phosphate, corn starch and the like. Of these, crystalline cellulose and corn starch are preferable.

Examples of the binder include polyvinyl alcohol, hydroxypropylcellulose, polyethylene glycol, polyvinylpyrrolidone, Pluronic F68, gum arabic, gelatin, starch and the like. Examples of the disintegrator include carboxymethylcellulose calcium (ECG505), croscarmellose sodium (Ac-Di-Sol), crosslinked polyvinylpyrrolidone (Crospovidone), low substituted hydroxypropylcellulose (L-HPC) and the like. Of these, hydroxypropylcellulose, polyvinylpyrrolidone and low substituted hydroxypropylcellulose are preferable. Examples of the lubricant and coagulation preventive include talc, magnesium stearate and inorganic salts thereof, and examples of the lubricant include polyethylene glycol and the like. Examples of the stabilizer include acids such as tartaric acid, citric acid, succinic acid, fumaric acid, maleic acid and the like.

The core can be also prepared by, besides the above-mentioned production methods, for example, rolling granulation wherein a mixture of a drug, an excipient, a lubricant and the like or a drug is added by small portions while spraying a binder dissolved in a suitable solvent such as water, lower alcohol (e.g., methanol, ethanol and the like) and the like on an inert carrier particles to be the center of the core, a pan coating method, a fluidized bed coating method or a melt granulating method. Examples of the inert carrier particle include those prepared from sucrose, lactose, starch, crystalline cellulose and waxes, which preferably has an average particle size of about 100 µm - about 1,500 µm.

To separate the drug contained in the core from the film forming agent, the surface of the core may be coated with a protective agent. Examples of the protective agent include the aforementioned hydrophilic material, water-insoluble material and the like. As the protective agent, preferably polyethylene glycol, polysaccharides having a hydroxyalkyl group or a carboxyalkyl group, more preferably hydroxypropylmethylcellulose and hydroxypropylcellulose are used. The protective agent may contain, as a stabilizer, an acid such as tartaric acid, citric acid, succinic acid, fumaric acid, maleic acid and the like, a lubricant such as talc and the like. When the protective agent is used, the coating amount of the film forming agent is about 1 - about 15% (w/w), preferably about 1 - about 10% (w/w), more preferably about 2 - about 8% (w/w), relative to the core.

The protective agent can be coated by a typical coating method. Specifically, the protective agent is, for example, spray-coated to the core by a fluidized bed coating method, a pan coating method, and the like.

### II. Coating of core with a film forming agent

The core obtained in the aforementioned I is coated with a film forming liquid agent prepared by dissolving by heating or dissolving or dispersing in a solvent the aforementioned water-insoluble material, the pH-dependent swellable polymer, and the hydrophilic material to provide a sustained release preparation.

For coating a film forming liquid agent to a core, for example, a spray coating method and the like can be employed.

The composition ratio of the water-insoluble material, swellable polymer or hydrophilic material in the film forming liquid agent is determined such that the content of each component of the coating film will fall within the range of the aforementioned content.

The coating amount of the film forming agent is about 1 - about 90% (w/w), preferably about 5 - about 50% (w/w), more preferably about 5 - 35% (w/w), relative to the core (exclusive of the coating amount of protective agent).

As the solvent for the film forming liquid agent, water and organic solvents can be used alone or in a mixture of the both. The mixing ratio (water/organic solvent: weight ratio) of water and the organic solvent in the mixture can vary within the range of 1 - 100%, which is preferably 1 - about 30%. The organic solvent is not subject to any particular limitation as long as it dissolves the water-insoluble material. For example, lower alcohols such as methyl alcohol, ethyl alcohol, isopropyl alcohol, n-butyl alcohol and the like, lower alkanone such as acetone and the like, acetonitrile, chloroform, methylene chloride and the like are used. Of these, lower alcohol is preferable, and ethyl alcohol and isopropyl alcohol are particularly preferable. Water and a mixture of water with an organic solvent are preferably used as a solvent of the film forming agent. Where necessary, the film forming liquid agent may contain an acid such as tartaric acid, citric acid, succinic acid, fumaric acid, maleic acid and the like for the stabilization of the film forming liquid agent.

When spray coating is employed, the method follows a conventional coating method, which is specifically a spray coating of a film forming liquid agent to the core by, for example, a fluidized bed coating method, a pan coating method and the like. Where necessary, talc, titanium oxide, magnesium stearate, calcium stearate, light anhydrous silicic acid and the like may be added as a lubricant, and glycerine fatty acid ester, hydrogenated castor oil, triethyl citrate, cetyl alcohol, stearyl alcohol and the like may be added as a plasticizer.

After coating with a film forming agent, an antistatic agent such as talc and the like may be added as necessary.

A rapid release preparation may be a liquid (solution, suspension, emulsion and the like) or a solid (particles, pill, tablet and the like). While an oral administration agent, and a parenteral administration agent such as injection and the like are used, preferred is an agent for the oral administration.

A rapid release preparation may generally contain, in addition to the drug, which is an active ingredient, carriers, additives and excipients (hereinafter sometimes simply referred to as an excipient), which are conventionally used in the field of preparation. The excipient for a preparation is not subject to any particular limitation as long as it is conventionally employed as an excipient for a preparation. For example, the excipient for the oral solid preparation includes lactose, starch, corn starch, crystalline cellulose (manufactured by Asahi Kasei Corporation, Avicel PH101 and the like), powder sugar, granulated sugar, mannitol, light anhydrous silicic acid, magnesium carbonate, calcium carbonate, L-cysteine and the like, preferably corn starch and mannitol and the like. These excipients may be used alone or in combination. The content of the excipient is, for example, about 4.5 - about 99.4 w/w%, preferably about 20 - about 98.5 w/w%, more preferably about 30 - about 97 w/w%, relative to the total amount of the rapid release preparation.

The drug content of the rapid release preparation is appropriately determined from the range of about 0.5 - about 95%, preferably about 1 - about 60%, relative to the total amount of the rapid release preparation.

When the rapid release preparation is an oral solid preparation, it generally contains a disintegrator in addition to the above-mentioned components. Examples of the disintegrator include calcium carboxymethylcellulose (ECG-505 manufactured by GOTOKU CHEMICAL COMPANY LTD.), croscarmellose sodium (e.g., Actisol manufactured by Asahi Kasei Corporation), Crospovidone (e.g., KollidonCL manufactured by BASF), low substituted hydroxypropylcellulose (Shin-Etsu Chemical Co., Ltd.), carboxymethyl starch (Matsutani Chemical Industry Co., Ltd., sodium carboxymethyl starch (Exprotab manufactured by Kimura Sangyo), partially α starch (PCS manufactured by Asahi Kasei Corporation) and the like. For example, one capable of disintegrating granules by water absorption, swelling, forming a channel between the active ingredient constituting the core and an excipient upon contact with water and the like can be used. These disintegrators can be used alone or in combination. The composition amount of the disintegrator is appropriately determined depending on the kind of the drug to be used and composition amount thereof, design of the release preparation and the like. It is, for example, about 0.05 - about 30 w/w%, preferably about 0.5 - about 15 w/w%, relative to the total amount of the rapid release preparation.

When the rapid release preparation is an oral solid preparation, the oral solid preparation may further contain, in addition to the above-mentioned composition, routine additives used for solid preparation on demand. The additive can be used as, for example, a binder (e.g., sucrose, gelatin, gum arabic powder, methylcellulose, hydroxypropylcellulose, hydroxypropylmethylcellulose, carboxymethylcellulose, polyvinylpyrrolidone, Pullulan, dextrin and the like), a lubricant (e.g., polyethylene glycol, magnesium stearate, talc, light anhydrous silicic acid (e.g., Aerosil (Nippon Aerosil)), a surfactant (e.g., anionic surfactant such as sodium alkylsulfate and the like, non-ionic surfactant such as polyoxyethylene fatty acid ester and polyoxyethylenesorbitan fatty acid ester, polyoxyethylene castor oil derivative and the like, and the like), a coloring agent (e.g., synthetic color, caramel, iron oxide red, titanium oxide, riboflavins), where necessary, a corrigent (e.g., a sweetener, flavor and the like), an absorbent, an antiseptic, a moistening agent, an antistatic agent and the like. As the stabilizer, an organic acid such as tartaric acid, citric acid, succinic acid, fumaric acid and the like may be added.

The above-mentioned binder preferably is used hydroxypropylcellulose, polyethylene glycol, polyvinylpyrrolidone and the like.

The rapid release preparation can be prepared based on the conventional preparation method, by mixing each of the aforementioned components, and where necessary, further kneading and forming. The above-mentioned mixing can be performed by a conventional method, such as mixing, kneading and the like. Specifically, for example, when a rapid release preparation is formed into particles, a vertical granulator, a universal kneader (manufactured by Hata Tekkosho), a fluidized bed granulator FD-5S (manufactured by Powrex Corporation) and the like are used for mixing, which is followed by granulating by wet extrusion granulation, fluidized bed granulation and the like, to give the preparation, as in the preparation of the core of the aforementioned sustained release preparation.

The rapid release preparation and the sustained release preparation thus obtained may be used as they are. Alternatively, after suitable separate preparation together with an excipient for a preparation and the like according to a conventional method, they may be administered simultaneously or at optional administration intervals. Alternatively, they may be prepared into a single preparation for oral administration (e.g., granule, subtilized granule, tablet, capsule and the like) as they are or together with excipient for preparation and the like as appropriate. The both preparations are converted to granules or subtilized granules and filled in a single capsule and the like to give a preparation for oral administration.

### [3] A sublingual tablet, buccal or oral cavity rapid disintegrator and preparation thereof

The sublingual tablet, buccal preparation and oral cavity rapid disintegrator may be a solid preparation such as tablet and the like or an oral cavity mucous membrane adhesion tablet (film).

As the sublingual tablet, buccal or oral cavity rapid disintegrator, a preparation containing the Compound A or a drug in combination and an excipient is preferable. It may contain auxiliaries such as a lubricant, an isotonic agent, a hydrophilic carrier, a water dispersible polymer, a stabilizer and the like. For easy absorption and enhanced bioavailability, β-cyclodextrin or β-cyclodextrin derivative (e.g., hydroxypropyl-β-cyclodextrin and the like) and the like may be contained.

The above-mentioned excipient include lactose, sucrose, D-mannitol, starch, crystalline cellulose, light anhydrous silicic acid and the like. The lubricant include magnesium stearate, calcium stearate, talc, colloidal silica and the like, magnesium stearate and colloidal silica are particularly preferable. The isotonic agent include sodium chloride, glucose, fructose, mannitol, sorbitol, lactose, saccharose, glycerine, urea and the like, mannitol is particularly preferable. The hydrophilic carrier include swellable hydrophilic carriers such as crystalline cellulose, ethylcellulose, crosslinked polyvinylpyrrolidone, light anhydrous silicic acid, silicic acid, dicalcium phosphate, calcium carbonate and the like, crystalline cellulose (e.g., microcrystalline cellulose and the like) is particularly preferable. Examples of the water dispersible polymer include gum (e.g., gum tragacanth, acacia gum, guar gum), alginate (e.g., sodium alginate), cellulose derivative (e.g., methylcellulose, carboxymethylcellulose, hydroxymethylcellulose, hydroxypropylcellulose, hydroxypropylmethylcellulose), gelatin, water-soluble starch, polyacrylic acid (e.g., carbomer), polymethacrylic acid, polyvinyl alcohol, polyethylene glycol, polyvinylpyrrolidone, polycarbofil, ascorbic acid, palmitate and the like, with preference given to hydroxypropylmethylcellulose, polyacrylic acid, alginate, gelatin, carboxymethylcellulose, polyvinylpyrrolidone, polyethylene glycol and the like. Particularly, hydroxypropylmethylcellulose is preferable. Examples of the stabilizer include cysteine, thiosorbitol, tartaric acid, citric acid, sodium carbonate, ascorbic acid, glycine, sodium sulfite and the like, particularly, citric acid and ascorbic acid are preferable.

The sublingual tablet, buccal and oral cavity rapid disintegrator can be produced by mixing the Compound A or a drug in combination and an excipient by a method known per se. Where desired, the above-mentioned auxiliaries such as a lubricant, an isotonic agent, a hydrophilic carrier, a water dispersible polymer, a stabilizer, a coloring agent, a sweetener, an antiseptic and the like may be contained. After mixing the above-mentioned components simultaneously or with time staggering, the mixture is compression formed under pressure to give sublingual tablet, buccal tablet or oral cavity rapid disintegrator. To achieve a suitable hardness, a solvent such as water, alcohol and the like may be used to moisten or wet as necessary before and after the compression forming. After the forming, the tablets are dried.

When a mucous membrane adhesion tablet (film) is produced, the Compound A or a drug in combination and the above-mentioned water dispersible polymer (preferably, hydroxypropylcellulose, hydroxypropylmethylcellulose), an excipient and the like are dissolved in a solvent such as water and the like, and the obtained solution is cast to give a film. In addition, an additive such as a plasticizer, a stabilizer, an antioxidant, a preservative, a coloring agent, a buffer, a sweetener and the like may be added. To impart suitable elasticity to the film, glycols such as polyethylene glycol, propylene glycol and the like may be added, and, bioadhesive polymer (e.g., polycarbofil, carbopol) may be added to increase adhesion of the film to the oral cavity mucous membrane lining. The casting includes pouring the solution on a non-adhesive surface, spreading the solution in a uniform thickness (preferably about 10 - 1000 µ) with a coating tool such as doctor blade and the like and drying the solution to give a film. The film thus formed may be dried at room temperature or under heating and cut into a desired surface area.

Examples of preferable oral cavity rapid disintegrator is a solid rapid diffusing administration agent having a net structure of the Compound A or a drug in combination and water-soluble or water diffusable carrier which are inert to the drug in combination. The net structure can be obtained by sublimation of a solvent from the solid composition consisting of a solution obtained by dissolving the Compound A or a drug in combination in a suitable solvent.

The composition of oral cavity rapid disintegrator preferably contains, in addition to the Compound A or a drug in combination, a matrix-forming agent and a secondary component.

The matrix-forming agent include animal proteins or vegetable proteins such as gelatins, dextrins, soybeans, wheat, psyllium seed protein and the like; rubber substances such as gum arabic, guar gum, agar, xanthan and the like; polysaccharides; alginic acids; carboxymethylcelluloses; carragheenans; dextrans; pectins; synthetic polymers such as polyvinylpyrrolidone and the like; a material derived from a gelatin-gum arabic complex and the like. In addition, saccharides such as mannitol, dextrose, lactose, galactose, trehalose and the like; cyclic saccharides such as cyclodextrin and the like; inorganic salts such as sodium phosphate, sodium chloride, aluminum silicate and the like; amino acid having 2 to 12 carbon atoms such as glycine, L-alanine, L-aspartic acid, L-glutamine acid, L-hydroxyproline, L-isoleucine, L-leucine, L-phenylalanine and the like are exemplified.

It is possible to introduce one or more matrices forming agents into a solution or suspension before preparation into a solid. Such matrix-forming agent may exist with a surfactant or without a surfactant. The matrix-forming agent can form a matrix, and also can help maintain the diffusion of the Compound A or a drug in combination in the solution or suspension.

The composition may contain a secondary component such as a preservative, an antioxidant, a surfactant, a thickener, a coloring agent, a pH adjusting agent, a flavor, a sweetener, a taste masking reagent and the like. Suitable coloring agents include red, black and yellow ferric oxides and FD&C dyes of Elis and Eberald, such as FD&C blue No. 2, FD&C red No. 40 and the like. A suitable flavor contains mint, raspberry, licorice, orange, lemon, grapefruit, caramel, vanilla, cherry, grape flavor and a combination of these. Suitable pH adjusting agent includes citric acid, tartaric acid, phosphoric acid, hydrochloric acid and maleic acid. Suitable sweetener includes aspartame, acesulfame K, thaumatin and the like. Suitable taste masking agent includes sodium bicarbonate, ion exchange resin, cyclodextrin inclusion compound, adsorbent substance and microcapsuled apomorphine.

The preferable preparation is a preparation (the above-mentioned sublingual tablet, buccal and the like) capable of dissolving 90% or more of the Compound A or a drug in combination (in water) within about 1 min - about 60 min, preferably about 1 min - about 15 min, more preferably about 2 min - about 5 min, and an oral cavity rapid disintegrator that disintegrates within 1 - 60 sec, preferably 1-30 sec, more preferably 1-10 sec, after being placed in an oral cavity, which contains the Compound A or a drug in combination generally in a proportion of about 0.1 - about 50 wt%, preferably about 0.1 - about 30 wt%.

The content of the above-mentioned excipient in the whole preparation is about 10 - about 99 wt%, preferably about 30 - about 90 wt%. The content of the β-cyclodextrin or β-cyclodextrin derivative relative to the whole preparation is 0 - about 30 wt%. The content of the lubricant relative to the whole preparation is about 0.01 - about 10 wt%, preferably about 1 - about 5 wt%. The content of the isotonic agent relative to the whole preparation is about 0.1 - about 90 wt%, preferably about 10 - about 70 wt%. The content of the hydrophilic carrier relative to the whole preparation is about 0.1 - about 50 wt%, preferably about 10 - about 30 wt%. The content of the water dispersible polymer relative to the whole preparation is about 0.1 - about 30 wt%, preferably about 10 - about 25 wt%. The content of the stabilizer relative to the whole preparation is about 0.1 - about 10 wt%, preferably about 1 - about 5 wt%. The above-mentioned preparation may contain additives such as a coloring agent, a sweetener, an antiseptic and the like as necessary.

While the dose of the combination drug varies depending on the kind of the compound, the patient's age, body weight and condition, the dosage form, the mode and the period of the treatment, the amount of the combination drug may be, for example, generally about 0.01 to about 1000 mg/kg, preferably about 0.01 to about 100 mg/kg, more preferably about 0.1 to about 100 mg/kg, most preferably about 0.1 to about 50 mg/kg, and particularly about 1.5 to about 30 mg/kg, as the amount of the Compound A and the drug in combination, per day for a patient with sepsis (adult weighing about 60 kg), said daily dose being given intravenously all at once or in several portions during a day. It is a matter of course that a lower daily dose may be sufficient or an excessive dose may be required since the dose may vary depending on various factors as discussed above.

The drug in combination may be contained in any amount as long as a side effect does not pose a problem. While the daily dose of the drug in combination may vary depending on the disease state, the age, sex, body weight and difference in sensitivity of the administration object, timing and interval of administration, characteristics, dispensing and kind of the pharmaceutical preparation, the kind of active ingredient and the like and is not particularly limited, the amount of the drug is generally about 0.001 - 2000 mg, preferably about 0.01 - 500 mg, more preferably about 0.1 - 100 mg, per 1 kg body weight of mammal by oral administration, which is generally administered all at once or in 2 to 4 portions during a day.

When the pharmaceutical composition of the present invention is administered, a Compound A and a drug in combination may be administered at the same time, or a drug in combination may be administered first, and then the Compound A may be administered. Alternatively, the Compound A may be administered first, and then the drug in combination may be administered. For time stagger administration, the time difference varies depending on the active ingredient to be administered, dosage form and administration route. For example, when a drug in combination is to be administered first, the Compound A is administered within 1 min - 3 days, preferably 10 min - 1 day, more preferably 15 min - 1 hour, after the administration of the drug in combination. When the Compound A is to be administered first, the drug in combination is administered within 1 min - 1 day, preferably 10 min - 6 hours, more preferably 15 min - 1 hour, after the administration of the drug in combination.

### Examples

The present invention is further described in the following by referring to Reference Examples, Examples and Experimental Examples, which are not intended to restrict the invention.

The ¹H-NMR spectrum was determined by a VARIAN GEMINI 200 (200 MHz) spectrometer using tetramethyl silane as an internal standard and represented as the entire δ values in ppm. The number in a bracket when a solvent mixture was employed is the volume ratio of each solvent, wherein % means % by weight unless otherwise specified. The ratio of the solvents in silica gel chromatography shows the volume ratio of the solvents to be admixed.

A high polarity diastereomer means a diastereomer having a smaller Rf value, and a low polarity diastereomer means a diastereomer having a larger Rf value, when determined by normal phase thin layer chromatography under the same conditions (e.g., using ethyl acetate/hexane etc. as a solvent).

The melting point was measured using melting point measuring apparatus (manufactured by Yanako). The data of the powder X-ray diffraction was measured using RINT2500 (Rigaku Industrial Corporation) using Cu-K_{α1} ray as a ray source.

Each symbol in examples mean the following:
s: singlet d: doublet: t: triplet q: quartet
dd: double doublet tt: triple triplet m: multiplet
br: broad J: coupling constant

### Examples

The following Reference Examples A can be produced according to Reference Examples of WO99/46242, Reference Example B can be produced according to Examples of WO99/46242, Reference Examples C can be produced according to Reference Examples of WO01/10826, and Reference Examples D can be produced according to Examples of WO01/10826.

### [Reference Examples A]

Reference Example A1 ethyl 2-sulfo-1-cyclohexene-1-carboxylate
Reference Example A2 ethyl 2-chlorosulfonyl-1-cyclohexene-1-carboxylate
Reference Example A3 ethyl 2-chlorosulfonyl-1-cyclopentene-1-carboxylate
Reference Example A4 ethyl 2-chlorosulfonyl-1-cycloheptene-1-carboxylate
Reference Example A5 sodium 6-[N-(4-chloro-2-fluorophenyl)-sulfamoyl]-1-cyclohexene-1-carboxylate
Reference Example A6 1-(3-fluoro-4-nitrophenyl)-1H-1,2,4-- triazole
Reference Example A7 l-(4-amino-3-fluorophenyl)-1H-1,2,4-triazole
Reference Example A8 methyl 4-(benzyloxycarbonylamino)-3-chlorobenzoate
Reference Example A9 4-(benzyloxycarbonylamino)-3-chlorobenzoic acid
Reference Example A10 tert-butyl N-(4-benzyloxycarbonylamino-3-chlorobenzoyl)glycinate
Reference Example A11 tert-butyl N-(4-amino-3-chlorobenzoyl)-glycinate
Reference Example A12 6-[N-(2,4-difluorophenyl)sulfamoyl]-1-cyclohexene-1-carboxylic acid
Reference Example A13 ethyl 2-mercapto-5-phenyl-1-cyclohexene-1-carboxylate
Reference Example A14 ethyl 2-chlorosulfonyl-5-phenyl-1-cyclohexene-1-carboxylate
Reference Example A15 ethyl 5-tert-butyl-2-mercapto-1-cyclohexene-1-carboxylate
Reference Example A16 ethyl 5-tert-butyl-2-chlorosulfonyl-1-cyclohexene-1-carboxylate
Reference Example A17 ethyl 5,5-dimethyl-2-mercapto-1-cyclohexene-1-carboxylate
Reference Example A18 ethyl 2-chlorosulfonyl-5,5-dimethyl-1-cyclohexene-1-carboxylate

### [Reference Examples B]

Reference Example B1 ethyl 6-[N-(4-chloro-2-fluorophenyl)-sulfamoyl]-1-cyclohexene-1-carboxylate (compound 1)
Reference Example B2 ethyl 6-[N-(4-chloro-2-fluorophenyl)-N-methylsulfamoyl]-1-cyclohexene-1-carboxylate (compound 2)
Reference Example B3 ethyl 6-[N-(2,4-difluorophenyl)sulfamoyl]-1-cyclohexene-1-carboxylate (compound 3)
Reference Example B4 ethyl 6-[N-(2,6-diisopropylphenyl)-sulfamoyl]-1-cyclohexene-1-carboxylate (compound 4)
Reference Example B5 ethyl 6-[N-(4-nitrophenyl)sulfamoyl]-1-cyclohexene-1-carboxylate (compound 5)
Reference Example B6 ethyl 6-(N-phenylsulfamoyl)-1-cyclohexene-1-carboxylate (compound 6)
ethyl 2-(N-phenylsulfamoyl)-1-cyclohexene-1-carboxylate (compound 7)
Reference Example B7 ethyl 2-[N-(4-chloro-2-fluorophenyl)-sulfamoyl]-1-cyclohexene-1-carboxylate (compound 9)
Reference Example B8 2-(4-methoxyphenyl)-4,5,6,7-tetrahydro-1,2-benzisothiazol-3(2H)-one 1,1-dioxide (compound 67)
ethyl 2-[N-(4-methoxyphenyl)sulfamoyl]-1-cyclohexene-1-carboxylate (compound 8)
Reference Example B9 ethyl 6-[N-(2-fluorophenyl)sulfamoyl]-1-cyclohexene-1-carboxylate (compound 10)
Reference Example B10 ethyl 6-[N-(3-fluorophenyl)sulfamoyl]-1-cyclohexene-1-carboxylate (compound 11)
Reference Example B11 2-(4-fluorophenyl)-4,5,6,7-tetrahydro-1,2-benzisothiazol-3(2H)-one 1,1-dioxide (compound 68)
ethyl 6-[N-(4-fluorophenyl)sulfamoyl]-1-cyclohexene-1-carboxylate (compound 12)
ethyl 2-[N-( 4-fluorophenyl)sulfamoyl]-1-cyclohexene-1-carboxylate (compound 18)
Reference Example B12 ethyl 6-[N-(2,6-difluorophenyl)-sulfamoyl]-1-cyclohexene-1-carboxylate (compound 13)
Reference Example B13 ethyl 6-[N-(2,3-difluorophenyl)-sulfamoyl]-1-cyclohexene-1-carboxylate (compound 14)
Reference Example B14 ethyl 6-[N-(2,5-difluorophenyl)-sulfamoyl]-1-cyclohexene-1-carboxylate (compound 15)
Reference Example B15 ethyl 6-[N-(3,4-difluorophenyl)-sulfamoyl]-1-cyclohexene-1-carboxylate (compound 16)
Reference Example B16 ethyl 6-[N-(3,5-difluorophenyl)-sulfamoyl]-1-cyclohexene-1-carboxylate (compound 17)
Reference Example B17 1-ethyl 6-[N-(2,4-difluorophenyl)-sulfamoyl]-1-cyclohexene-1-carboxylate (compound 19)
d-ethyl 6-[N-(2,4-difluorophenyl) sulfamoyl]-1-cyclohexene-1-carboxylate (compound 20)
Reference Example B18 ethyl 6-[N-(2-ethoxycarbonylphenyl)-sulfamoyl]-1-cyclohexene-1-carboxylate (compound 21)
Reference Example B19 methyl 6-[N-(2,4-difluorophenyl)-sulfamoyl]-1-cyclohexene-1-carboxylate (compound 22)
Reference Example B20 propyl 6-[N-(2,4-difluorophenyl)-sulfamoyl]-1-cyclohexene-1-carboxylate (compound 23)
Reference Example B21 methyl 6-[N-(4-chloro-2-fluorophenyl)-sulfamoyl]-1-cyclohexene-1-carboxylate (compound 24)
Reference Example B22 isopropyl 6-[N-(2,4-difluorophenyl)-sulfamoyl]-1-cyclohexene-1-carboxylate (compound 25)
Reference Example B23 ethyl 6-[N-(2-methoxycarbonylphenyl)-sulfamoyl]-1-cyclohexene-1-carboxylate (compound 26)
Reference Example B24 ethyl 6-[N-(2-fluoro-4-methylphenyl)-sulfamoyl]-1-cyclohexene-1-carboxylate (compound 27)
Reference Example B25 ethyl 6-[N-(2-chlorophenyl)sulfamoyl]-1-cyclohexene-1-carboxylate (compound 28)
Reference Example B26 ethyl 6-[N-(2-chloro-4-fluorophenyl)-sulfamoyl]-1-cyclohexene-1-carboxylate (compound 29)
Reference Example B27 ethyl 6-[N-(4-chlorophenyl)sulfamoyl]-1-cyclohexene-1-carboxylate (compound 30)
Reference Example B28 ethyl 6-[N-(2,3,4-trifluorophenyl)-sulfamoyl]-1-cyclohexene-1-carboxylate (compound 31)
Reference Example B29 isobutyl 6-[N-(2,4-difluorophenyl)-sulfamoyl]-1-cyclohexene-1-carboxylate (compound 32)
Reference Example B30 butyl 6-[N-(2,4-difluorophenyl)-sulfamoyl]-1-cyclohexene-1-carboxylate (compound 33)
Reference Example B31 ethyl 6-[N-(4-bromo-2-fluorophenyl)-sulfamoyl]-1-cyclohexene-1-carboxylate (compound 34)
Reference Example B32 ethyl 6-[N-(2,4-dichlorophenyl)-sulfamoyl]-1-cyclohexene-1-carboxylate (compound 35)
Reference Example B33 ethyl 6-[N-(2-acetoxyphenyl)sulfamoyl]-1-cyclohexene-1-carboxylate (compound 36)
Reference Example B34 ethyl 6-[N-(3-chlorophenyl)sulfamoyl]-1-cyclohexene-1-carboxylate (compound 37)
Reference Example B35 ethyl 6-[N-(2,3-dichlorophenyl)-sulfamoyl]-1-cyclohexene-1-carboxylate (compound 38)
Reference Example B36 ethyl 6-[N-(2-ethylphenyl)sulfamoyl]-1-cyclohexene-1-carboxylate (compound 39)
Reference Example B37 ethyl 6-[N-[4-(2H-1,2,3-triazol-2-yl)phenyl]sulfamoyl]-1-cyclohexene-1-carboxylate (compound 40)
Reference Example B38 ethyl 6-[N-(2,5-dichlorophenyl)-sulfamoyl]-1-cyclohexene-1-carboxylate (compound 41)
Reference Example B39 ethyl 6-[N-(2-trifluoromethoxyphenyl)-sulfamoyl]-1-cyclohexene-1-carboxylate (compound 42)
Reference Example B40 ethyl 6-[N-(2,4,5-trifluorophenyl)-sulfamoyl]-1-cyclohexene-1-carboxylate (compound 43)
Reference Example B41 ethyl 6-[N-[4-(2H-tetrazol-2-yl)phenyl]sulfamoyl]-1-cyclohexene-1-carboxylate (compound 44)
Reference Example B42 ethyl 6-[N-(2-chloro-4-methylphenyl)-sulfamoyl]-1-cyclohexene-1-carboxylate (compound 45)
Reference Example B43 ethyl 6-[N-(4-fluoro-2-methylphenyl)-sulfamoyl]-1-cyclohexene-1-carboxylate (compound 46)
Reference Example B44 ethyl 6-[N-(2,6-dichlorophenyl)-sulfamoyl]-1-cyclohexene-1-carboxylate (compound 47)
Reference Example B45 ethyl 6-[N-[4-(lH-tetrazol-1-yl)phenyl]sulfamoyl]-1-cyclohexene-1-carboxylate (compound 48)
Reference Example B46 ethyl 6-[N-(4-(lH-1,2,3-triazol-1-yl)phenyl]sulfamoyl]-1-cyclohexene-1-carboxylate (compound 49)
Reference Example B47 ethyl 6-[N-(2-trifluoromethylphenyl)-sulfamoyl]-1-cyclohexene-1-carboxylate (compound 50)
Reference Example B48 ethyl 6-[N-(4-methoxycarbonylphenyl)-sulfamoyl]-1-cyclohexene-1-carboxylate (compound 51)
Reference Example B49 benzyl 6-[N-(2,4-difluorophenyl)-sulfamoyl]-1-cyclohexene-1-carboxylate(compound 52)
Reference Example B50 ethyl 6-[N-[4-[2,3-bis(tert-butoxycarbonyl) guanidinomethyl] phenyl] sulfamoyl]-1-cyclohexene-1-carboxylate (compound 53)
Reference Example B51 ethyl 6-[N-(2-chloro-4-methoxycarbonylphenyl)sulfamoyl]-1-cyclohexene-1-carboxylate (compound 54)
Reference Example B52 ethyl 6-[N-(2-chloro-4-cyanophenyl)-sulfamoyl]-1-cyclohexene-1-carboxylate (compound 55)
Reference Example B53 2-hydroxyethyl 6-[N-(2,4-difluorophenyl)sulfamoyl]-1-cyclohexene-1-carboxylate (compound 56)
Reference Example B54 ethyl 6-[N-[2-fluoro-4-(lH-1,2,4-triazol-1-yl)phenyl] sulfamoyl]-1-cyclohexene-1-carboxylate (compound 57)
Reference Example B55 ethyl 2-[N-(2,4-difluorophenyl)-sulfamoyl]-1-cyclopentene-1-carboxylate (compound 66)
ethyl 5-[N-(2,4-difluorophenyl)sulfamoyl]-1-cyclopentene-1-carboxylate (compound 58)
Reference Example B56 tert-butyl [6-[N-(2,4-difluorophenyl)-sulfamoyl]-1-cyclohexen-1-yl]carbonyloxyacetate (compound 59)
Reference Example B57 [6-[N-(2,4-difluorophenyl)sulfamoyl]-1-cyclohexen-1-yl]carbonyloxyacetic acid (compound 60)
Reference Example B58 ethyl 7-[N-(2,4-difluorophenyl)-sulfamoyl]-1-cycloheptene-1-carboxylate (compound 61)
Reference Example B59 ethyl 6-[N-[2-chloro-4-(N-tert-butoxycarbonylmethylcarbamoyl) phenyl] sulfamoyl]-1-cyclohexene-1-carboxylate (compound 62)
Reference Example B60 ethyl 6-[N-[2-chloro-4-(N-ethoxycarbonylmethylcarbamoyl)phenyl]sulfamoyl]-1-cyclohexene-1-carboxylate (compound 63)
Reference Example B61 ethyl 5-[N-(2-chloro-4-fluorophenyl)-sulfamoyl]-1-cyclopentene-1-carboxylate (compound 64)
Reference Example B62 2-[4-(2,2,3,3,3-pentafluoropropoxy)-phenyl]-4,5,6,7-tetrahydro-1,2-benzisothiazol-3(2H)-one 1,1-dioxide (compound 69)
Reference Example B63 ethyl 7-[N-(2-chloro-4-fluorophenyl)-sulfamoyl]-1-cycloheptene-1-carboxylate (compound 65)
Reference Example B64 2-(2,4-difluorophenyl)-5,6,7,7a-tetrahydro-1,2-benzisothiazol-3(2H)-one 1,1-dioxide (compound 70)
Reference Example B65 ethyl 6-[N-(2-chloro-4-fluorophenyl)-sulfamoyl]-1-cyclohexene-1-carboxylate (compound 29)
Reference Example B66 ethyl (6S)-6-[(2-chloro-4-fluoroanilino)sulfonyl]-1-cyclohexene-1-carboxylate (1-ethyl
6-[N-(2-chloro-4-fluorophenyl)sulfamoyl]-1-cyclohexene-1-carboxylate) (compound 71)
ethyl (6R)-6-[(2-chloro-4-fluoroanilino)sulfonyl]-1-cyclohexene-1-carboxylate (d-ethyl 6-[N-(2-chloro-4-fluorophenyl)sulfamoyl]-1-cyclohexene-1-carboxylate) (compound 72)
Reference Example B67 ethyl 6-[N-(2-bromo-4-fluorophenyl)-sulfamoyl]-1-cyclohexene-1-carboxylate (compound 73)
Reference Example B68 ethyl 6-[N-(4-bromo-2-chlorophenyl)-sulfamoyl]-1-cyclohexene-1-carboxylate (compound 74)
Reference Example B69 high polarity diastereomer (compound 75) and low polarity diastereomer (compound 76) of ethyl 6-[N-(2,4-difluorophenyl)sulfamoyl]-3-phenyl-1-cyclohexene-1 carboxylate
Reference Example B70 high polarity diastereomer (compound 77) and low polarity diastereomer (compound 78) of ethyl 6-[N-(2-chloro-4-fluorophenyl)sulfamoyl]-3-phenyl-1-cyclohexene-1-carboxylate
Reference Example B71 high polarity diastereomer (compound 79) and low polarity diastereomer (compound 80) of ethyl 6-[N-(2,4-difluorophenyl)sulfamoyl]-3-tert-butyl-1-cyclohexene-1-carboxylate
Reference Example B72 high polarity diastereomer (compound 81) and low polarity diastereomer (compound 82) of ethyl 6-[N-(2-chloro-4-fluorophenyl)sulfamoyl]-3-tert-butyl-1-cyclohexene-1-carboxylate
Reference Example B73 ethyl 6-[N-(2,4-difluorophenyl)-sulfamoyl]-3,3-dimethyl-1-cyclohexene-1-carboxylate (compound 83)
Reference Example B74 ethyl 6-[N-(2-chloro-4-fluorophenyl)-sulfamoyl]-3,3-dimethyl-1-cyclohexene-1-carboxylate (compound 84)
Reference Example B75 ethyl 3-bromo-6-[N-(2,4-difluorophenyl)-sulfamoyl]-1-cyclohexene-1-carboxylate (compound 85)

Furthermore, specific examples are shown in Tables 1 - 5.

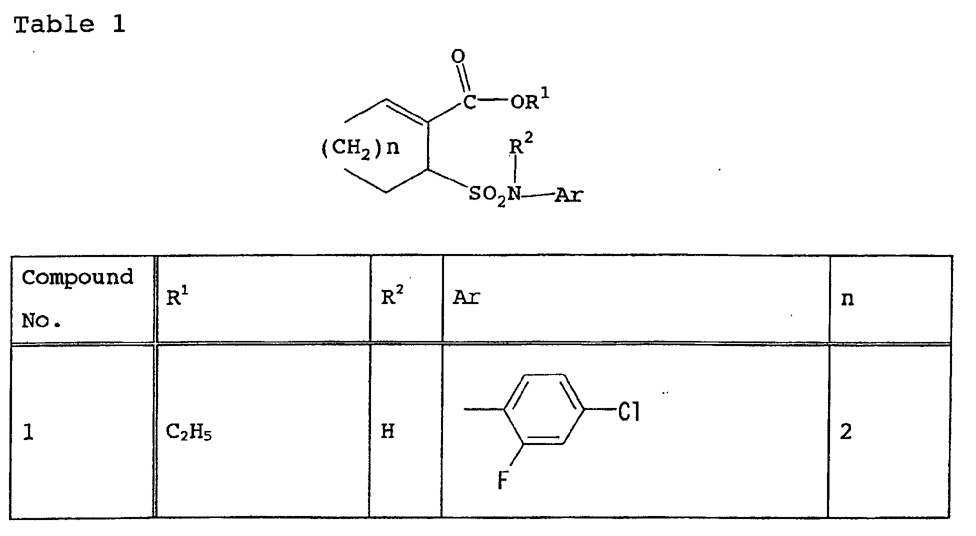

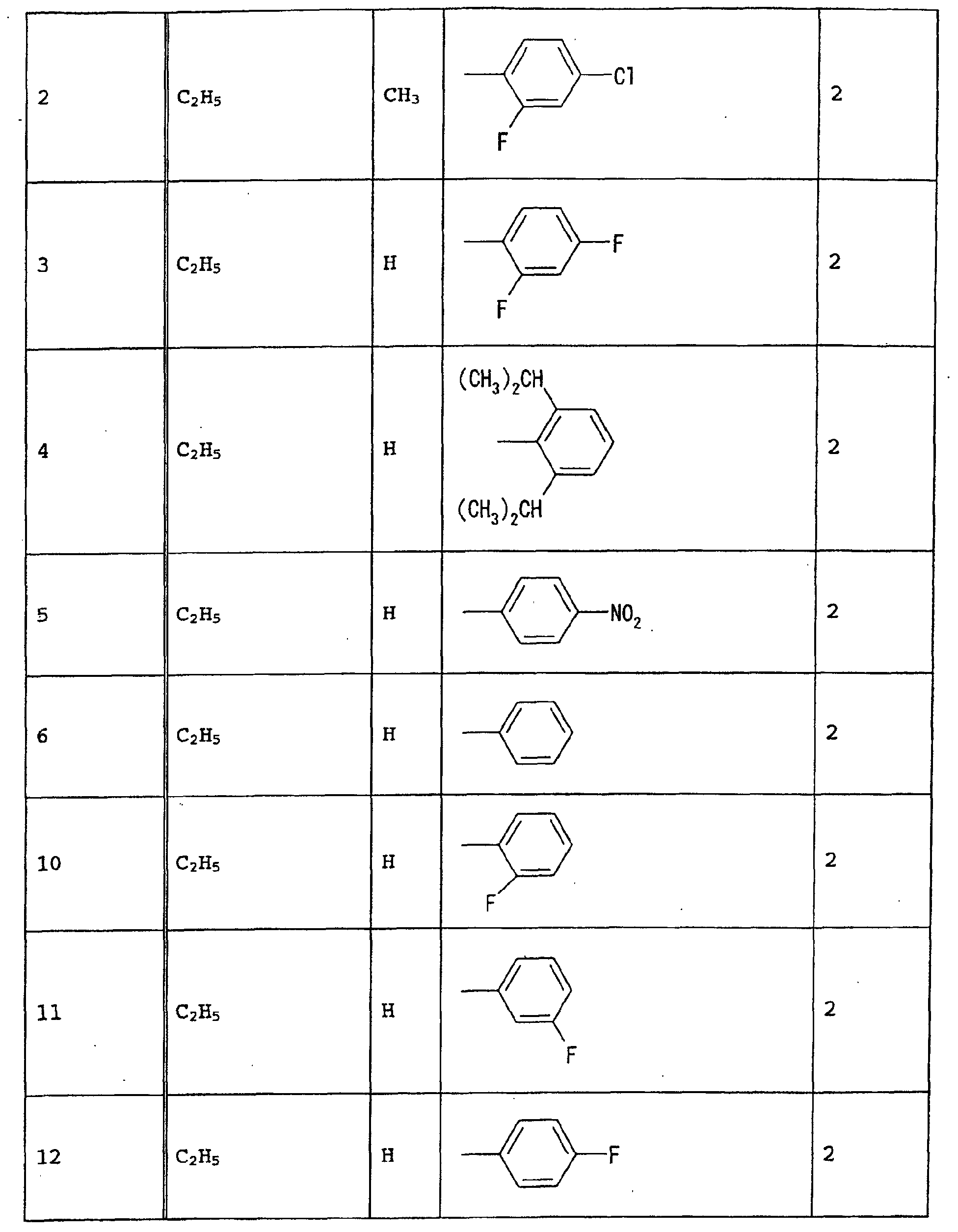

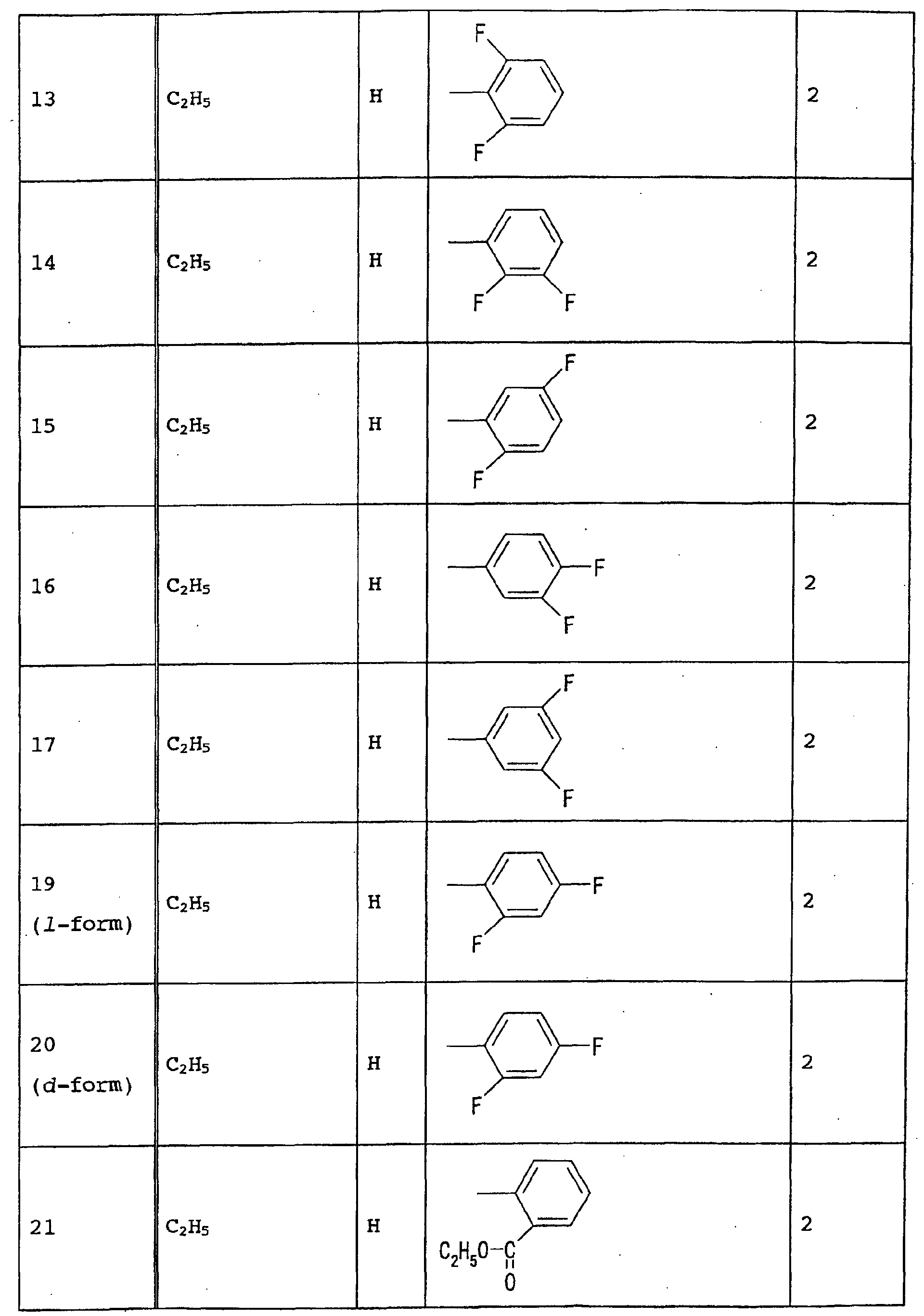

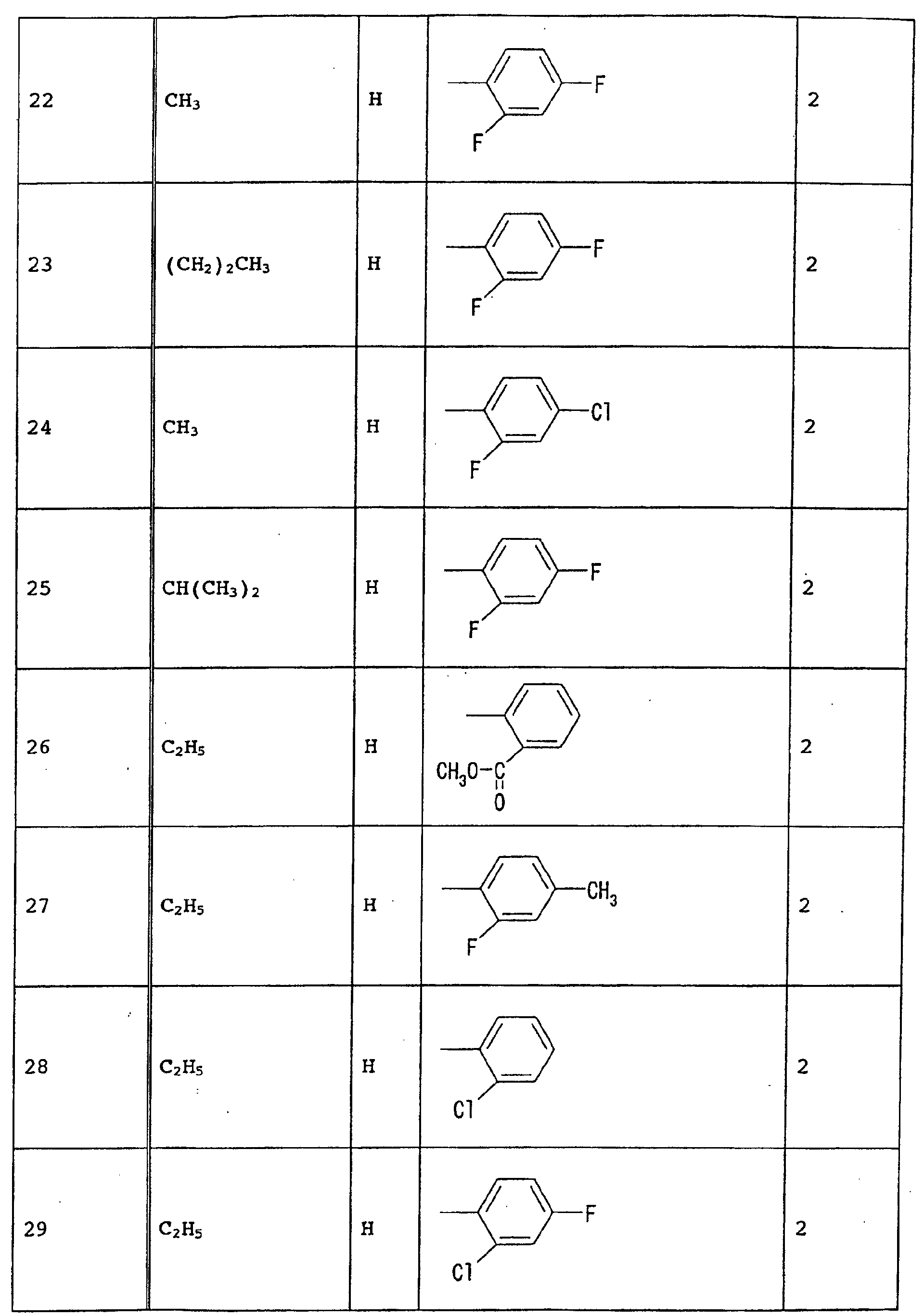

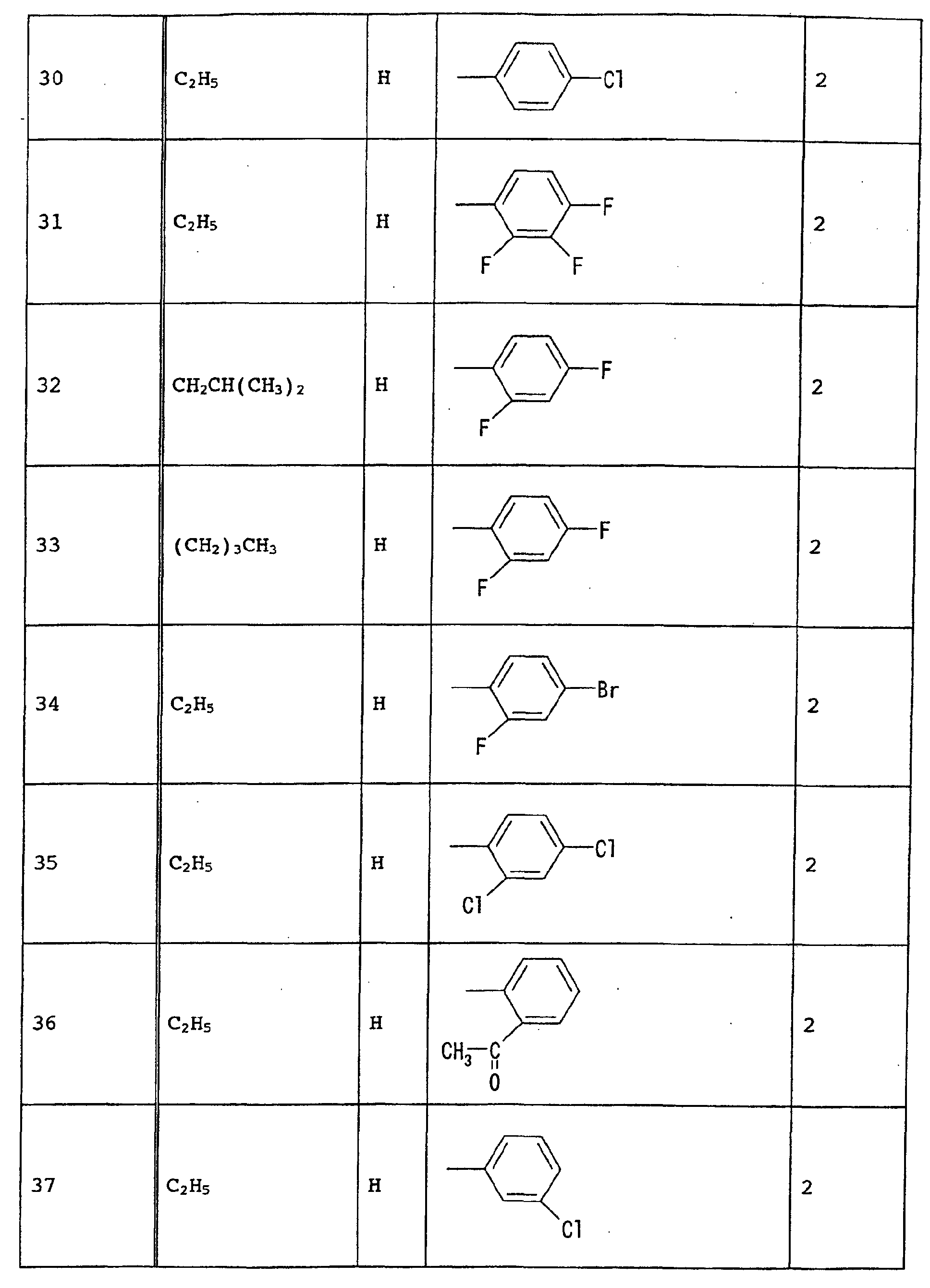

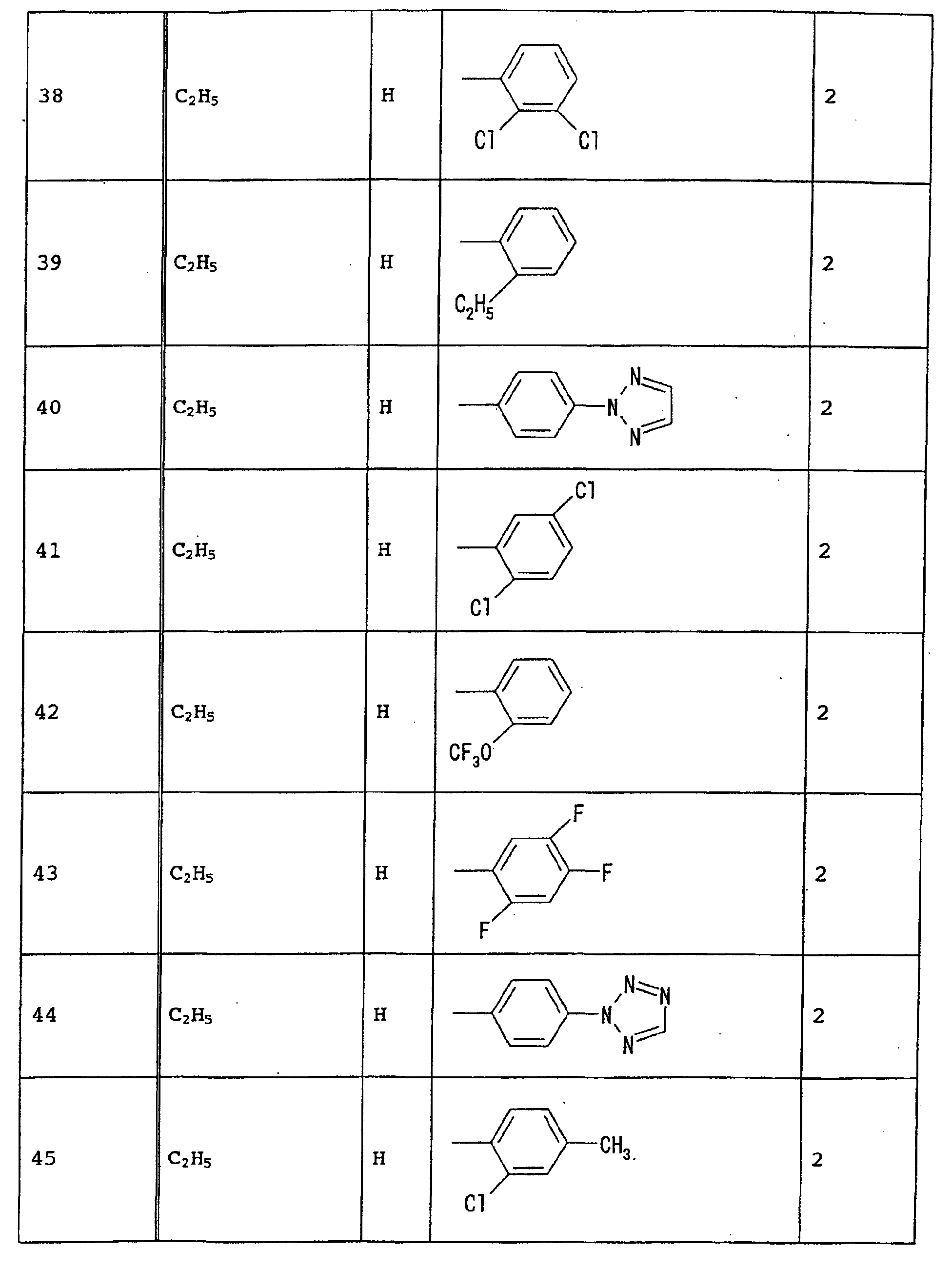

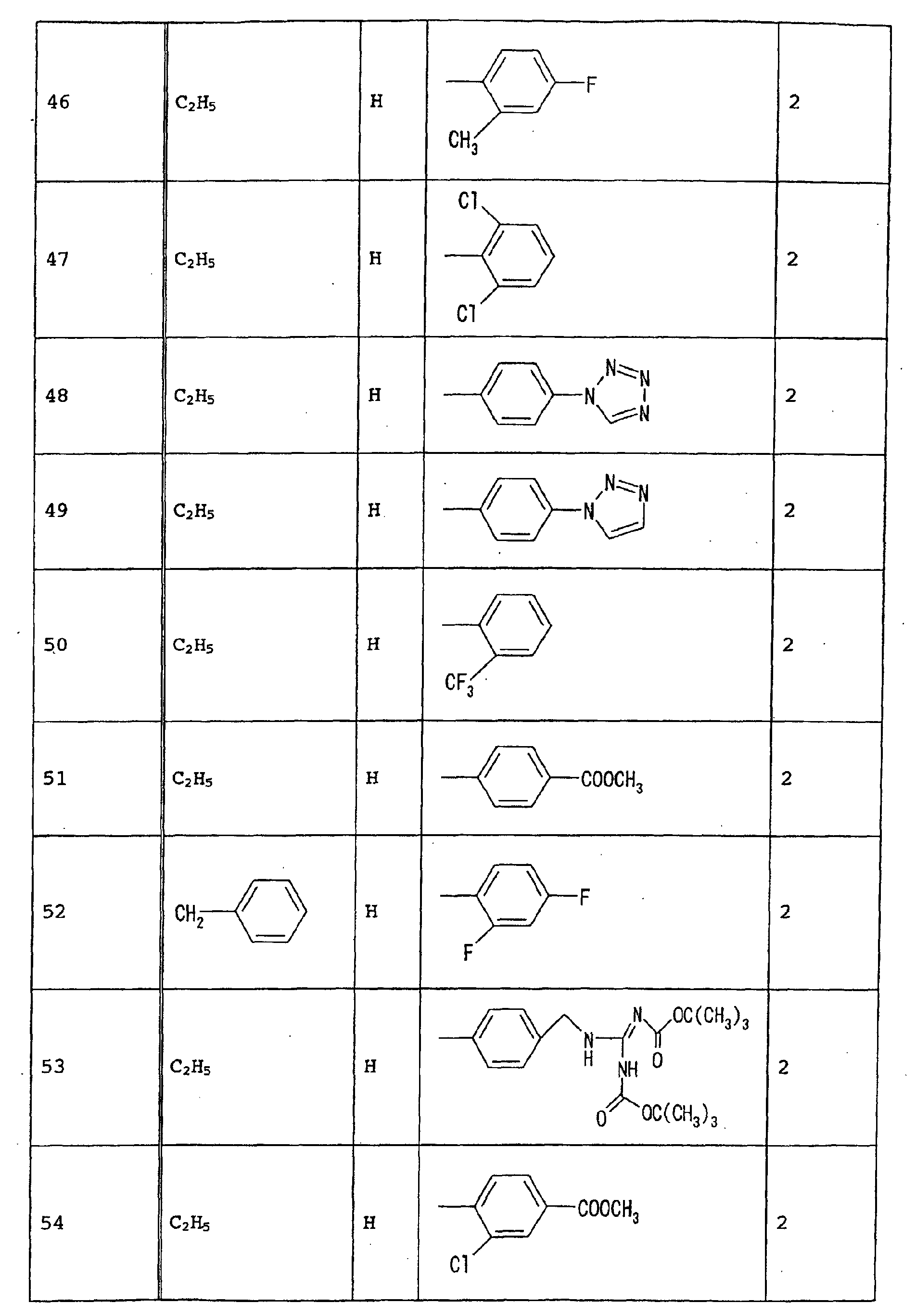

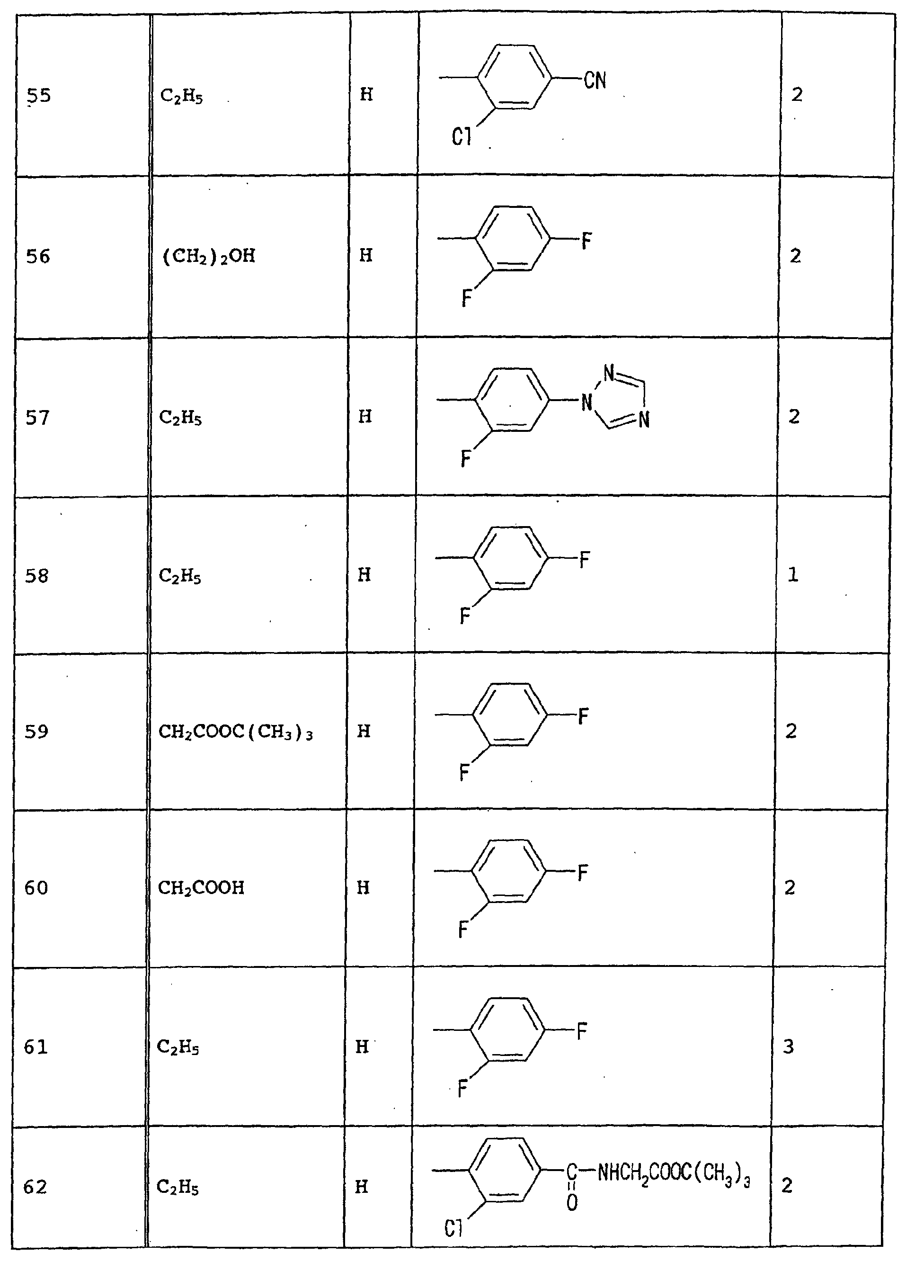

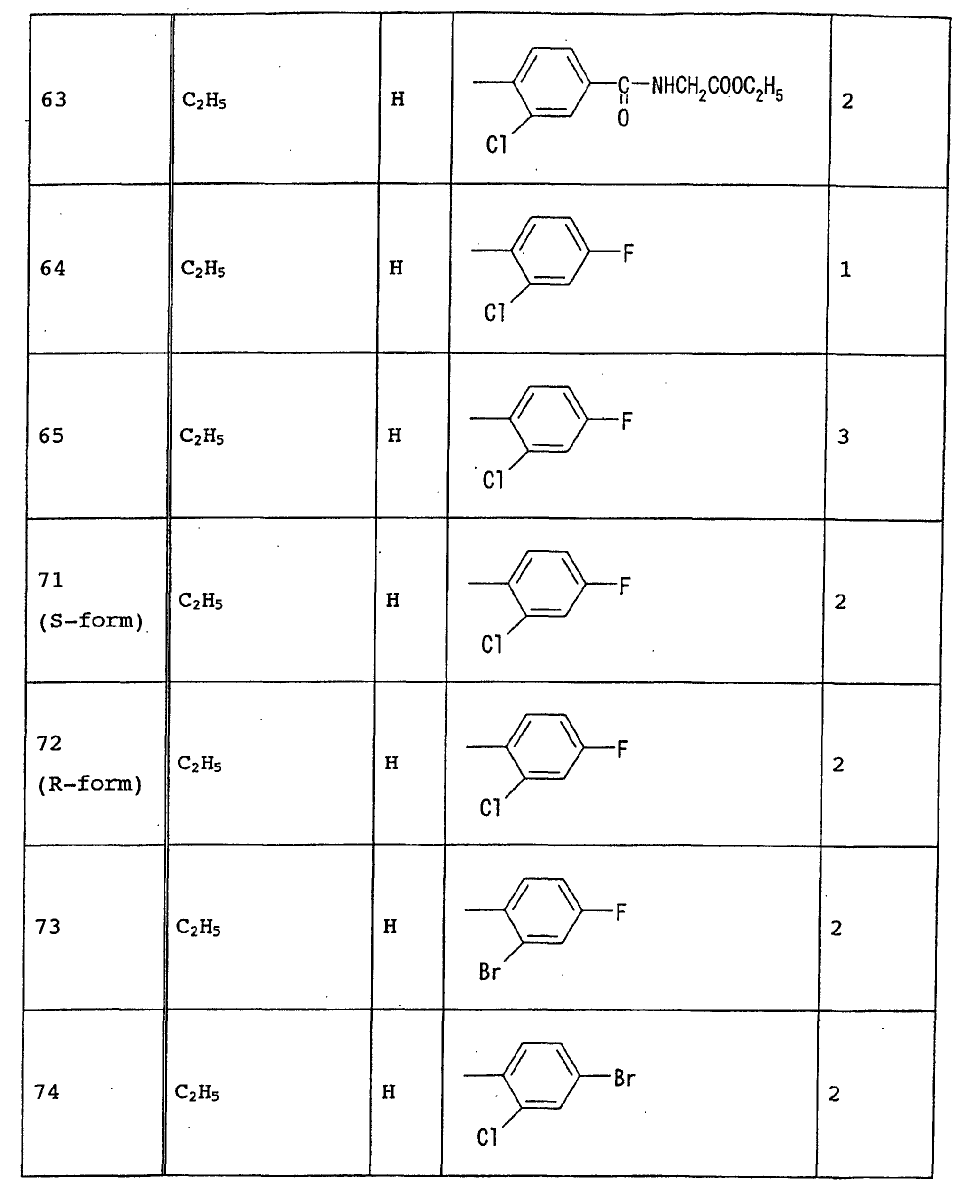

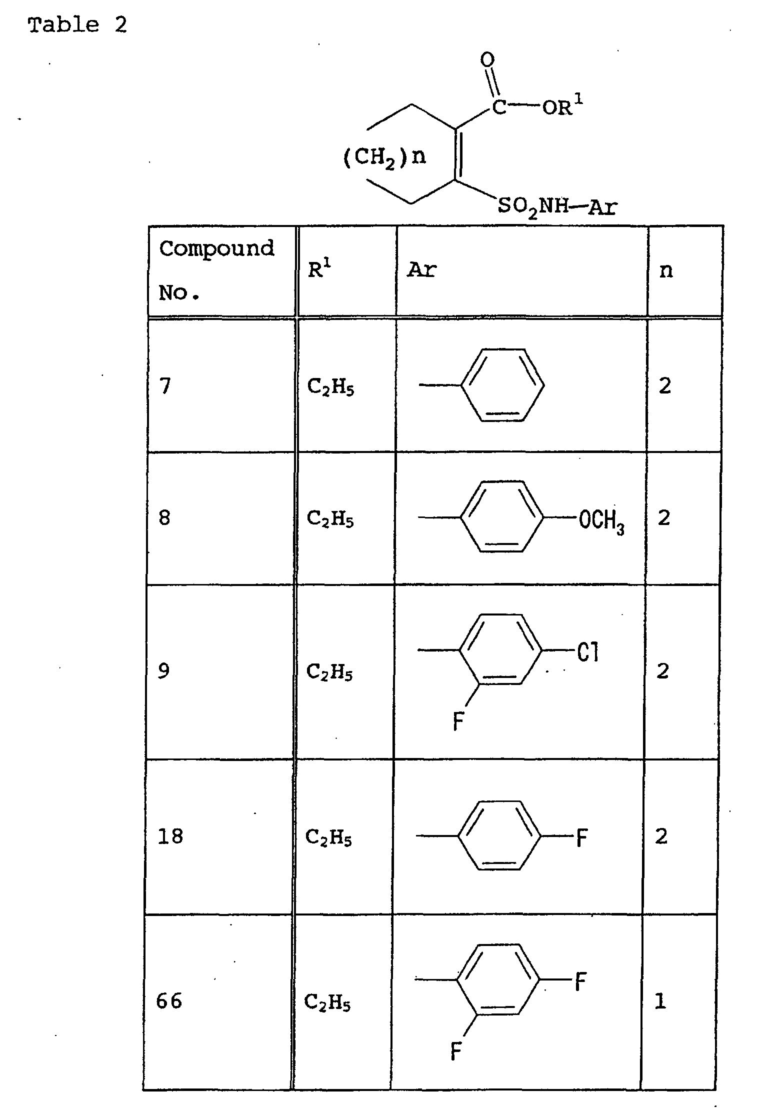

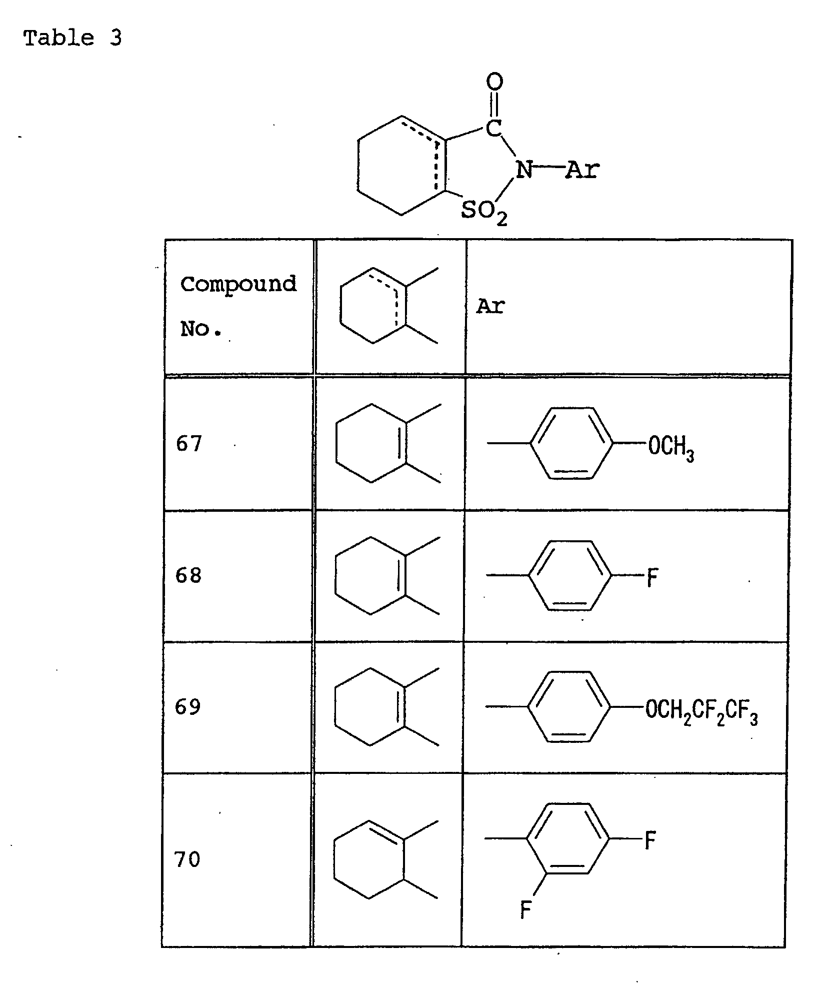

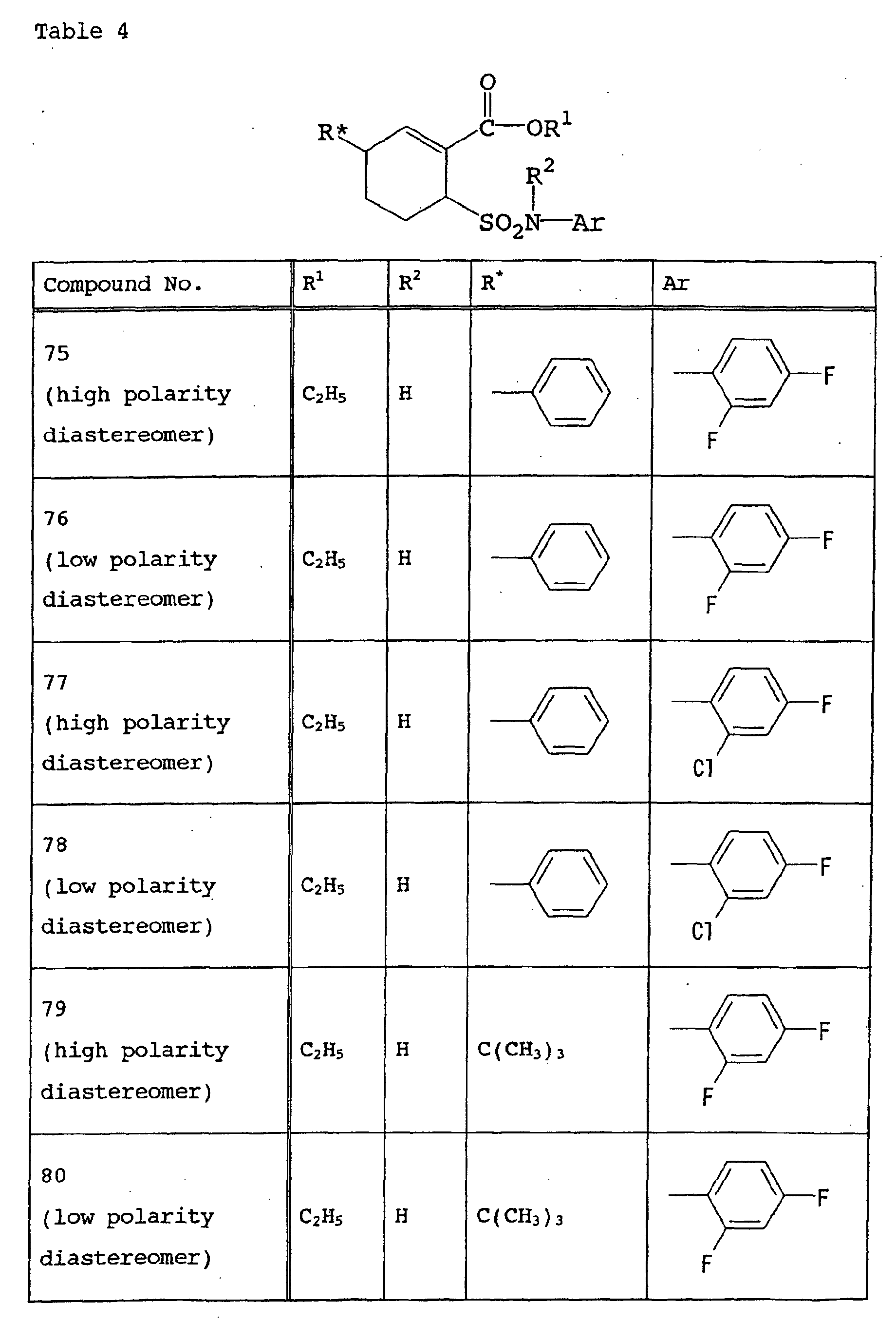

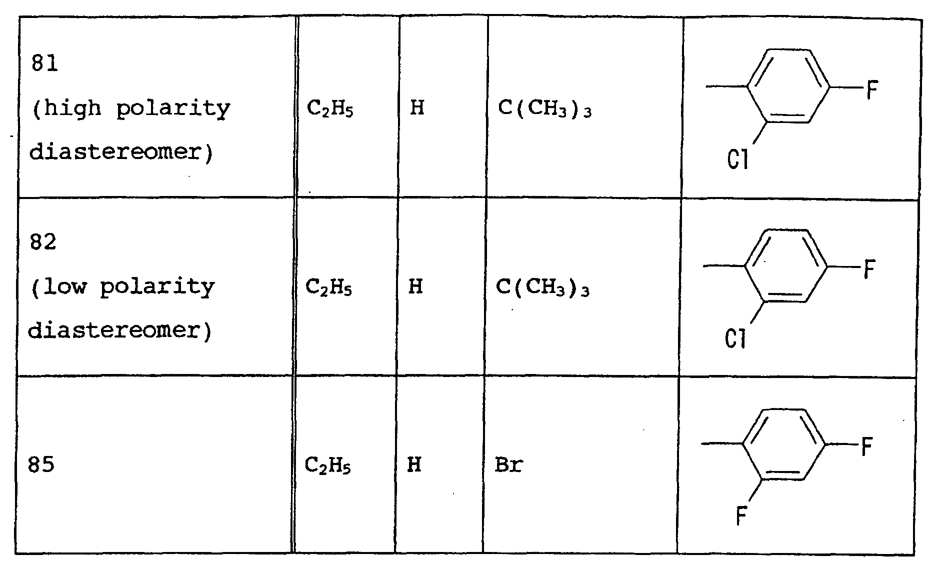

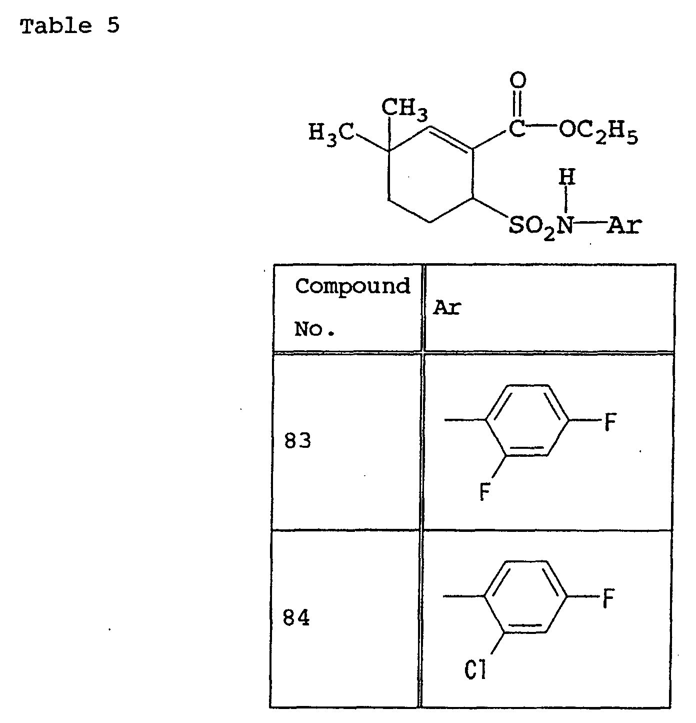

### Reference Example C1

To a solution of ethyl 6-[N-(2,4-difluorophenyl)sulfamoyl]-1-cyclohexene-l-carboxylate (1 g, synthesized by the method described in WO99/46242) and phenylmethanethiol (719 mg) in N/N-dimethylformamide (20 ml) was dropwise added 1,8-diazabicyclo[5,4,0]-7-undecene (441 mg) under ice-cooling, and the mixture was stirred at room temperature for 18 hrs. After diluting the reaction mixture with ethyl acetate (100 ml), the mixture was washed with water (70 ml x 2) and saturated brine (70 ml), and dried over anhydrous sodium sulfate. The solvent was evaporated and the obtained residue was subjected to flash silica gel column chromatography (eluent: toluene) for purification to give ethyl 6-(benzylsulfanyl)-1-cyclohexene-1-carboxylate (673 mg) as a colorless oil.
¹H-NMR(CDCl₃) δ: 1.27 (3H, t, J = 7.0Hz), 1.55-2.36 (6H, m), 3.76 (1H, m), 3.86 (2H, s), 4.19 (2H, q, J = 7.0Hz), 6.95 (1H, t, J = 4.0Hz), 7.22-7.39 (5H, m).

### Reference Example C2

In the same manner as in Reference Example C1, ethyl 6-[N-(2,4-difluorophenyl)sulfamoyl]-1-cyclohexene-1-carboxylate (1 g) was reacted with (4-methoxyphenyl)methanethiol (893 mg) to give ethyl 6-[(4-methoxybenzyl)sulfanyl]-1-cyclohexene-1-carboxylate (848 mg) as a colorless oil.
¹H-NMR(CDCl₃) δ: 1.28 (3H, t, J = 7.0Hz), 1.57-2.32 (6H, m), 3.74 (1H, m), 3.80 (3H, s), 3.82 (2H, s), 4.20 (2H, q, J = 7.0Hz), 6.84 (2H, d, J = 8.4Hz), 6.94 (1H, t, J = 4.0Hz), 7.29 (2H, d, J = 8.4Hz).

### Reference Example C3

In the same manner as in Reference Example C1, ethyl 6-[N-(2,4-difluorophenyl)sulfamoyl]-1-cyclohexene-1-carboxylate (455 mg) was reacted with (2,4-difluorophenyl)methanethiol (421 mg) to give ethyl 6-[(2,4-difluorobenzyl)sulfanyl]-1-cyclohexene-1-carboxylate (185 mg) as a colorless oil.
¹H-NMR (CDCl₃) δ; 1.26 (3H, t, J = 7.0Hz), 1.60-2.40 (6H, m), 3.78 (1H, m), 3.84 (2H, s), 4.18 (2H, q, J = 7.0Hz), 6.76-6.88 (2H, m), 6.97 (1H, t, J = 4.4Hz), 7.34-7.46 (1H, m).

### Reference Example C4

In the same manner as in Reference Example C1, ethyl 6-[N-(2,4-difluorophenyl)sulfamoyl]-1-cyclohexene-1-carboxylate (835 mg) was reacted with (2-chloro-4-fluorophenyl)methanethiol (853 mg) to give ethyl 6-[(2-chloro-4-fluorobenzyl)sulfanyl]-1-cyclohexene-1-carboxylate (625 mg) as a colorless oil.
¹H-NMR (CDCl₃) δ: 1.26 (3H, t, J = 7.0Hz), 1.56-2.36 (6H, m), 3.82 (1H, m), 3.94 (2H, s), 4.19 (2H, q, J = 7.0Hz), 6.96 (1H, td, J = 8.6Hz, 2.6Hz), 6.98 (1H, m), 7.12 (1H, dd, J= 8.6Hz, 2.6Hz), 7.46 (1H, dd, J= 8.6Hz, 6.0Hz).

### Reference Example C5

In the same manner as shown in Tetrahedron, vol. 19, p. 1625 (1963), 3-pyranone (20.0 g) was reacted to give ethyl 5-hydroxy-3,6-dihydro-2H-pyran-4-carboxylate (7.52 g) as a colorless oil.
¹H-NMR (CDCl₃) δ: 1.32 (3H, t, J = 7.2 Hz), 2.31-2.38 (2H, m) , 3.79 (2H, t, J = 5.6 Hz), 4.14 (2H, t, J = 1.8 Hz), 4.24 (2H, q, J = 7.2 Hz), 11.85 (1H, s).
SIMS: 172(M⁺).

### Reference Example C6

In the same manner as shown in Tetrahedron, vol. 30, p. 3753 (1974), ethyl 5-hydroxy-3,6-dihydro-2H-pyran-4-carboxylate (12.9 g) was reacted to give ethyl 5-sulfanyl-3,6-dihydro-2H-pyran-4-carboxylate (12.0 g) as a pale-blue oil.
¹H-NMR (CDCl₃) δ: 1.32 (3H, t, J = 7.2 Hz), 2.42-2.50 (2H, m), 3.70 (1H, s), 3.84 (2H, t, J = 5.6 Hz), 4.22 (2H, t, J = 2.2 Hz), 4.25 (2H, q, J = 7.2 Hz).
elemental analysis: for C₈H₁₂O₃S
Calculated (%): C,51.04; H,6.43; S, 17.03.
Found (%): C,50.99; H,6.54; S, 16.91.

### Reference Example C7

Sodium peroxoborate 4 hydrate (24.5 g) was added to acetic acid (130 ml), and the mixture was heated to 50-55°C. Thereto was dropwise added a solution of ethyl 5-sulfanyl-3,6-dihydro-2H-pyran-4-carboxylate (10.0 g) in acetic acid (30 ml) over 2 hr. The mixture was stirred at 50-55°C for 3 hr, and the reaction mixture was concentrated under reduced pressure. Acetonitrile (230 ml) was added to the residue, and the mixture was stirred at room temperature for 2 days. The resulting insoluble material was filtered off. The insoluble material was washed with acetonitrile (70 ml). The filtrate and washing were combined and concentrated under reduced pressure. The residue was dissolved in acetonitrile (160 ml), and the mixture was stirred at room temperature for 6 hr. The resulting insoluble material was filtered off, and the filtrate was concentrated under reduced pressure. Diisopropyl ether (100 ml) was added to the residue, and the precipitated insoluble material was filtered off to give 4-(ethoxycarbonyl)-5,6-dihydro-2H-pyran-3-sulfonic acid as a pale-yellow oil (27.6 g) containing an inorganic product. ¹H-NMR(DMSO-d₆) δ: 1.19 (3H, t, J = 7.2 Hz), 2.17-2.21 (2H, m), 3.65 (2H, t, J = 5.5 Hz), 4.04 (2H, q, J = 7.2 Hz), 4.16 (2H, t, J = 2.4 Hz).

### Reference Example C8

4-(Ethoxycarbonyl) -5,6-dihydro-2H-pyran-3-sulfonic acid (27.5 g) was dissolved in thionyl chloride (82.6 ml), and the mixture was stirred at room temperature→85°C for 3 hr. The reaction mixture was evaporated under reduced pressure to dryness, and the residue was dissolved in ethyl acetate (100 ml). The obtained solution was partitioned by adding diluted brine (120 ml), and the ethyl acetate layer was washed twice with saturated brine (50 ml) and dried over anhydrous sodium sulfate. The solvent was evaporated and the obtained residue was subjected to silica gel column chromatography (eluent: ethyl acetate/hexane = 1/7→1/5) for purification. The objective product was concentrated under reduced pressure, and the resulting frozen crystals were washed with hexane to give ethyl 5-(chlorosulfonyl)-3,6-dihydro-2H-pyran-4-carboxylate (7.81 g) as pale-yellow crystals.
¹H-NMR (CDCl₃) δ: 1.37 (3H, t, J = 7.2 Hz), 2.62-2.70 (2H, m), 3.87 (2H, t, J = 5.5 Hz), 4.34 (2H, q, J = 7.2 Hz), 4.53 (2H, t, J = 2.6 Hz).
Elemental analysis: for C₈H₁₁ClO₅S
Calculated (%): C,37.73; H,4.35.
Found (%): C,37.64; H,4.27.

### Reference Example D1

To a solution of ethyl 6-(benzylsulfanyl)-1-cyclohexene-1-carboxylate (100 mg) obtained in Reference Example C1 in methylene chloride (3 ml) was added m-chlorobenzoic acid (196 mg) under ice-cooling, and the mixture was stirred at room temperature for 1 hr. A saturated aqueous sodium hydrogencarbonate solution (20 ml) was added to the reaction solution and extracted with ethyl acetate (20 ml x 2). The ethyl acetate layer was washed with a saturated aqueous sodium hydrogencarbonate solution (20 ml), water (20 ml) and saturated brine (20 ml), and dried over anhydrous sodium sulfate. The solvent was evaporated, and the obtained residue was subjected to flash silica gel chromatography (eluent: hexane→ethyl acetate/hexane = 1/30) for purification. Crystallization from hexane gave ethyl 6-(benzylsulfonyl)-1-cyclohexene-1-carboxylate (compound 1', 106 mg) as white crystals.
¹H-NMR (CDCl₃) δ: 1.34 (3H, t, J = 7.2Hz), 1.41-2.50 (6H, m), 4.28 (2H, q, J = 7.2Hz), 4.29 (1H, d, J= 13.8Hz), 4.35 (1H, m), 4.55 (1H, d, J= 13.8Hz), 7.37-7.45 (4H, m), 7.50-7.55 (2H, m).
Elemental analysis: for C₁₆H₂₀O₄S 0.5H₂O
Calculated (%): C, 60.55; H, 6.67
Found (%): C, 60.98; H, 6.32.

### Reference Example D2

In the same manner as in Reference Example D1, ethyl 6-[(4-methoxybenzyl)sulfanyl]-1-cyclohexene-1-carboxylate (98 mg) obtained in Reference Example C2 was reacted to give ethyl 6-[(4-methoxybenzyl)sulfonyl]-1-cyclohexene-1-carboxylate (compound 2', 88 mg) as white crystals.
¹H-NMR (CDCl₃) δ: 1.34 (3H, t, J = 7.0Hz), 1.42-2.50 (6H, m), 3.82 (3H, s), 4.21 (1H, d, J = 13.6Hz), 4.28 (2H, q, J = 7.0Hz), 4.31 (1H, m), 4.50 (1H, d, J = 13.6Hz), 6.92 (2H, d, J = 8.8Hz), 7.41 (1H, t, J = 3.6Hz), 7.47 (2H, d, J = 8.8Hz). Elemental analysis: for C₁₇H₂₂O₅S
Calculated (%): C, 60.33; H, 6.55
Found (%): C, 60.42; H, 6.58.

### Reference Example D3

In the same manner as in Reference Example D1, ethyl 6-[(2,4-difluorobenzyl)sulfanyl]-1-cyclohexene-1-carboxylate (161 mg) obtained in Reference Example C3 was reacted to give ethyl 6-[(2,4-difluorobenzyl)sulfonyl]-1-cyclohexene-1-carboxylate (compound 3', 134 mg) as white crystals.
¹H-NMR (CDCl₃) δ: 1.32 (3H, t, J = 7.0Hz), 1.59-2.50 (6H, m), 4.27 (2H, q, J = 7.0Hz), 4.35 (1H, d, J = 14.0Hz), 4.39 (1H, m), 4.51 (1H, d, J = 14.0Hz), 6.83-6.96 (2H, m), 7.42 (1H, t, J = 4.0Hz), 7.49-7.61 (1H, m).
Elemental analysis: for C₁₆H₁₈F₂O₄S
Calculated (%): C, 55.80; H, 5.27
Found (%): C, 55.95; H, 5.40.

### Reference Example D4

In the same manner as in Reference Example D1, ethyl 6-[(2-chloro-4-fluorobenzyl)sulfanyl]-1-cyclohexene-1-carboxylate (509 mg) obtained in Reference Example C4 was reacted to give ethyl 6-[(2-chloro-4-fluorobenzyl)sulfonyl]-1-cyclohexene-1-carboxylate (compound 4', 422 mg) as white crystals.
¹H-NMR (CDCl₃) δ: 1.32 (3H, t, J = 7.0Hz), 1.55-2.52 (6H, m), 4.25 (2H, q, J = 7.0Hz), 4.41 (1H, d, J = 5.6Hz), 4.59 (2H, s), 7.03 (1H, td, J = 8.4Hz, 2.6Hz), 7.21 (1H, dd, J= 8.4Hz, 2.6Hz), 7.42 (1H, t, J = 4.0Hz), 7.62 (1H, dd, J = 8.4Hz, 6.2Hz).
Elemental analysis: for C₁₆H₁₈ClFO₄S
Calculated (%): C, 53.26; H, 5.03
Found (%): C, 53.08; H, 4.95.

### Reference Example D5

Ethyl 6-[(2-chloro-4-fluorobenzyl)sulfonyl]-1-cyclohexene-1-carboxylate (compound 4', 100 mg) obtained in Reference Example D4 was divided into two kinds of optical isomers by high performance liquid chromatography (CHIRALPAK AD; eluent: hexane/ethanol 8/2), passed through a 0.45 µm filter, concentrated and crystallized from hexane to give ethyl (-)-6-[(2-chloro-4-fluorobenzyl)sulfonyl]-1-cyclohexene-1-carboxylate (compound 5', 50 mg) and ethyl (+)-6-[(2-chloro-4-fluorobenzyl)sulfonyl]-1-cyclohexene-1-carboxylate (compound 6', 49 mg) respectively as white crystals.
Compound 5'
¹H-NMR (CDCl₃) δ: 1.32 (3H, t, J = 7.0Hz), 1.56-2.55 (6H, m), 4.26 (2H, q, J = 7.0Hz), 4.42 (1H, d, J = 5.6Hz), 4.59 (2H, s), 7.03 1H, td, J = 8.6Hz, 2.4Hz), 7.21 (1H, dd, J= 8.6Hz, 2.4Hz), 7.42 (1H, t, J = 4.2Hz), 7.61 (1H, dd, J = 8.6Hz, 6.0Hz).
Elemental analysis: for C₁₆H₁₈ClFO₄S
Calculated (%): C, 53.26; H, 5.03
Found (%): C, 53.24; H, 4.85.
[α]_{D}²⁰ -97.0° (c = 0.5, in methanol).
Compound 6'
¹H-NMR (CDCl₃) δ: 1.32 (3H, t, J = 7.0Hz), 1.56-2.55 (6H, m), 4.26 (2H, q, J = 7.0Hz), 4.42 (1H, d, J = 6.2Hz), 4.59 (2H, S), 7.03 (1H, td, J = 8.6Hz, 2.4Hz), 7.21 (1H, dd, J= 8.6Hz_{,} 2.4Hz), 7.42 (1H, t, J = 4.4Hz), 7.60 (1H, dd, J = 8.6Hz, 6.0Hz).
Elemental analysis: for C₁₆H₁₈ClFO₄S
Calculated (%): C, 53.26; H, 5.03
Found (%): C, 53.29; H, 4.82.
[α]D²⁰ +95.0° (c = 0.5, in methanol).

### Reference Example D6

2,4-Difluoroaniline (0.45g) was dissolved in ethyl acetate (10 ml), and triethylamine (0.55 mg) was added to the obtained solution under ice-cooling. Thereto was dropwise added a solution of ethyl 5-(chlorosulfonyl)-3,6-dihydro-2H-pyran-4-carboxylate (0.69 g) obtained in Reference Example C8 in ethyl acetate (4 ml). The reaction mixture was stirred at 0°C for 30 min and then at room temperature for 5.8 hr under a nitrogen stream. The reaction mixture was diluted with ethyl acetate and washed successively with water (50 ml), 0.5N hydrochloric acid (50 ml), water (50 ml x 2) and saturated brine (50 ml). The ethyl acetate layer was dried over magnesium sulfate. The solvent was evaporated under reduced pressure. The residue was subjected to silica gel column chromatography (eluent: ethyl acetate/hexane=1/2) for purification. The objective fraction was concentrated under reduced pressure and the residue was crystallized from a mixture of ethyl acetate and diisopropyl ether to give ethyl 3-[(2,4-difluorophenyl)sulfamoyl]-3,6-dihydro-2H-pyran-4-carboxylate (compound 7'; 0.57 g) as white crystals.
¹H-NMR(DMSO-d₆)δ: 1.14 (3H, t, J = 7.0 Hz), 3.69 (1H, dd, J = 12.8 Hz, 3.0 Hz), 4.08 (2H, q, J = 7.0 Hz), 4.25 (2H, s), 4.33 (1H, d, J = 1.8 Hz), 4.41-4.48 (1H ,m), 7.00-7.05 (1H, m), 7.12 (1H, br), 7.22-7.33 (1H, m), 7.43-7.55 (1H, m), 9.82(1H, S).
Elemental analysis: for C₁₄H₁₅F₂NO₅S
Calculated (%): C, 48.41; H,4.35; N,4.03.
Found (%): C,48.47; H,4.35; N,3.96.

### Reference Example D7

In the same manner as in Reference Example D6, ethyl 5-(chlorosulfonyl)-3,6-dihydro-2H-pyran-4-carboxylate (0.70 g) obtained in Reference Example C8 was reacted with 2-chloro-4-fluoroaniline (0.52 g) to give ethyl 3-[(2-chloro-4-fluorophenyl)sulfamoyl]-3,6-dihydro-2H-pyran-4-carboxylate (compound 8'; 0.54 g) as white crystals.
¹H-NMR(DMSO-d₆) δ: 1.11 (3H, t, J = 7.0 Hz), 3.72 (1H, dd, J = 12.8 Hz, 3.0 Hz), 4.07 (2H, q, J = 7.0 Hz), 4.15-4.25 (2H, m), 4.37 (1H, d, J = 2.2 Hz), 4.46-4.55 (1H, m), 7.15 (1H, br), 7.22-7.26 (1H, m), 7.46-7.59 (2H, m), 9.68 (1H, s).
elemental analysis: for C₁₄H₁₅ClFNO₅S
Calculated (%): C,46.22; H,4.16; N,3.85. Found (%): C,46.35; H,4.11; N,3.73.

Specific examples of compound (II) synthesizable in the same manner as in the aforementioned Reference Example D are shown in Table 6 and Table 7. The compound (II) is not limited to the compounds shown in Table 6 and Table 7.

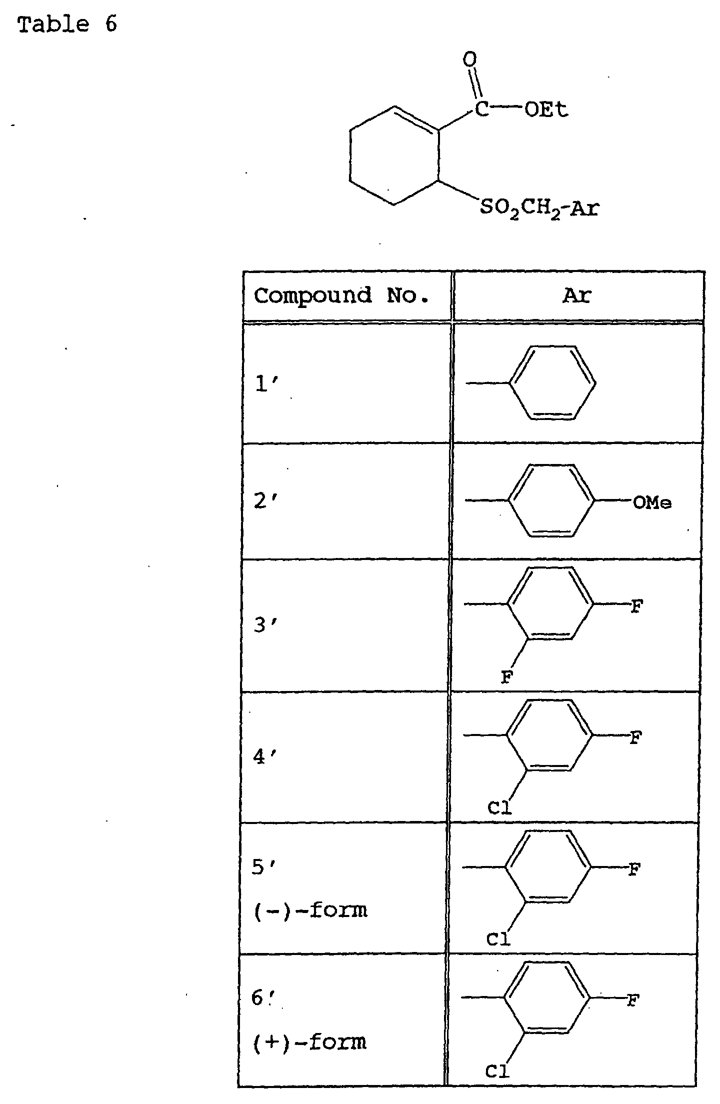

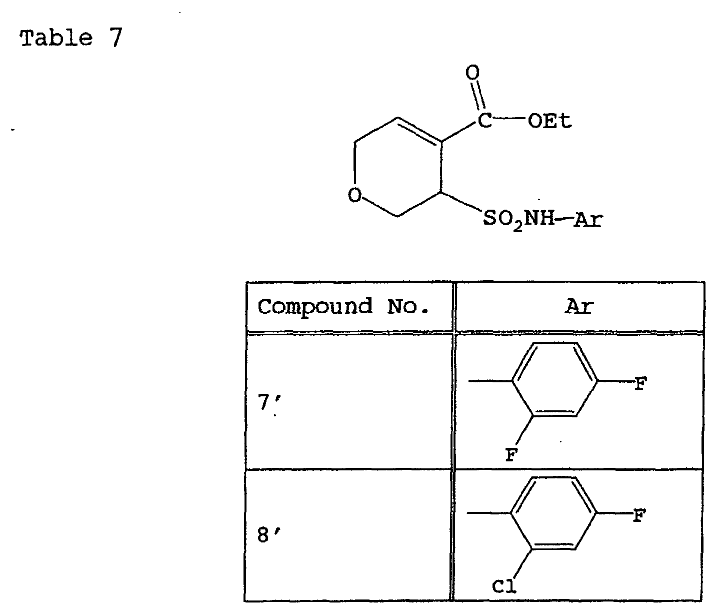

### Reference Example E1

| | |
|---|---|
| (1) compound 72 of Reference Example B66 | 10 mg |
| (2) lactose | 60 mg |
| (3) cornstarch | 35 mg |
| (4) gelatin | 3 mg |
| (5) magnesium stearate | 2 mg |

A mixture of compound 72 (10 mg) of Reference Example B66, lactose (60 mg) and cornstarch (35 mg) is granulated by the use of a 10% aqueous gelatin solution (0.03 ml) (3 mg as gelatin) and passing through a 1 mm mesh sieve, dried at 40°C and again passed through a sieve. The granules thus obtained are mixed with magnesium stearate (2 mg) and compressed. The obtained core tablets are sugar coated by applying an aqueous suspension of sucrose, titanium dioxide, talc and gum arabic and glazed with beeswax to give coated tablets.

### Reference Example E2

| | |
|---|---|
| (1) compound 72 of Reference Example B66 | 10 mg |
| (2) lactose | 70 mg |
| (3) cornstarch | 50 mg |
| (4) soluble starch | 7 mg |
| (5) magnesium stearate | 3 mg |

The compound 72 (10 mg) of Reference Example B66 and magnesium stearate (3 mg) are granulated using an aqueous solution (0.07 ml) of soluble starch (7 mg as soluble starch), dried and mixed with lactose (70 mg) and cornstarch (50 mg). The mixture is compressed to give tablets.

### Reference Example E3

| | |
|---|---|
| (1) compound 29 of Reference Example B65 | 10 mg |
| (2) lactose | 60 mg |
| (3) cornstarch | 35 mg |
| (4) gelatin | 3 mg |
| (5) magnesium stearate | 3 mg |

A mixture of compound 29 (10 mg) of Reference Example B65, lactose (60 mg) and cornstarch (35 mg) is granulated by the use of a 10% aqueous gelatin solution (0.03 ml) (3 mg as gelatin) and passing through a 1 mm mesh sieve, dried at 40°C and again passed through a sieve. The granules thus obtained are mixed with magnesium stearate (2 mg) and compressed. The obtained core tablets are sugar coated by applying an aqueous suspension of sucrose, titanium dioxide, talc and gum arabic and glazed with beeswax to give coated tablets.

### Reference Example E4

| | |
|---|---|
| (1) compound 29 of Reference Example B65 | 10 mg |
| (2) lactose | 70 mg |
| (3) cornstarch | 50 mg |
| (4) soluble starch | 7 mg |
| (5) magnesium stearate | 3 mg |

The compound 29 (10 mg) of Reference Example B65 and magnesium stearate (3 mg) are granulated using an aqueous solution (0.07 ml) of soluble starch (7 mg as soluble starch), dried and mixed with lactose (70 mg) and cornstarch (50 mg). The mixture is compressed to give tablets.

### Reference Example E5

| | |
|---|---|
| (1) ceftazidime | 5.0 mg |
| (2) salt | 20.0 mg |
| (3) distilled water to make total amount | 2 ml |

Ceftazidime (5.0 mg) and salt (20.0 mg) are dissolved in distilled water, and water was added to make the total amount 2.0 ml. The solution was filtrated and filled in a 2 ml ampoule under steric conditions. The ampoule is sterilized and sealed to give a solution for injection.

### Reference Example F1

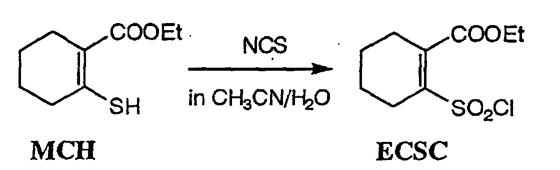

Acetonitrile (555 ml) and water (555 ml) were added to MCH (109.4 g, 0.59 mol), and the mixture was cooled to not higher than 0°C. N-Chlorosuccinimide (NCS) (255 g, 3.25-fold mol) was added to this mixture with thorough stirring, in such a manner that the internal temperature did not exceed 25°C. After the addition, the mixture was further stirred at 20±5°C for 1 hr. The reaction mixture was partitioned by adding isopropyl ether (IPE) (770 ml). The organic layer was washed 3 times with water (440 ml), and the solvent was evaporated under reduced pressure to give ECSC (149 g) as an oil.

### Reference Example F2

### d-ethyl 6-[N-(2-chloro-4-fluorophenyl)sulfamoyl]-1-cyclohexene-1-carboxylate (ethyl (6R)-6-[(2-chloro-4-fluoroanilino)sulfonyl]-1-cyclohexene-1-carboxylate)

d-Ethyl 6-[N-(2-chloro-4-fluorophenyl)sulfamoyl]-1-cyclohexene-1-carboxylate (ethyl (6R)-6-[(2-chloro-4-fluoroanilino)sulfonyl]-1-cyclohexene-1-carboxylate) (3.6 g) obtained according to Example 66 described in WO99/4624 was dissolved in ethanol (3 ml), and the mixture was concentrated under reduced pressure. A mixture of ethanol (1 ml) and hexane (3 ml) was added to the residue, and the precipitated crystals were collected by filtration. The crystals were washed with hexane and dried under reduced pressure to give the title compound (2.8 g) as colorless crystals having a melting point of 68-69°C. The powder X-ray diffraction pattern is shown in Fig. 2.
¹H-NMR (d₆-DMSO) δ 1.05 (3H, t, J=7.0Hz), 1.56-1.83 (2H, m), 1.97-2.43 (4H, m), 4.00 (2H, q, J=7.0 Hz), 4.29 (1H, d, J=5.0Hz), 7.10 (1H, br), 7.20-7.30 (1H, m), 7.50-7.58 (2H, m), 9.75 (1H, s).
Elemental analysis: calculated- for C₁₅H₁₇ClFNO₄S
Calculated: C, 49.79; H, 4.74; N, 3.87.
Experimented: C, 49.73; H, 4.69; N, 3.81.
[α]_{D}²⁰ +111.0°C (c=1.0, in methanol).

**Table 8.**

| data of powder X-ray diffraction | |
|---|---|
| angle of diffraction: 2θ(°) | lattice spacing: d (angstrom) |
| 8.54 | 10.3 |
| 9.52 | 9.28 |
| 13.2 | 6.72 |
| 15.0 | 5.89 |
| 17.2 | 5.16 |
| 19.5 | 4.54 |
| 20.2 | 4.38 |
| 24.8 | 3.59 |
| 25.3 | 3.52 |
| 25.8 | 3.45 |
| 26.3 | 3.39 |

### Example 1 (combination drug)

A preparation of any of Reference Example E1 - Reference Example E4 and the preparation of Reference Example E5 are combined.

### Experimental Example 1

The *E. coli* 0111 strain was suspended in 5% mucin, and 0.5 ml thereof was intraperitoneally inoculated to mice (BALB/c, male, 5-week-old) (9×10⁴ CFU per mouse). At 1 hr after inoculation, an antibacterial agent, ceftazidime (CAZ, 12.5 µg/kg) was administered subcutaneously, and compound 29 (test compound 30 mg/kg) of Reference Example B65 suspended in a 0.5% methyl cellulose (MC) solution was simultaneously administered orally. The survival of each mouse was monitored for a week, the results of which are shown in Fig. 1.

From Fig. 1, it was found that a combined use of an ineffective test compound or a test compound in an amount showing low effectiveness and ceftazidime remarkably increases the survival rate.

The combination drug of the present invention is useful as a prophylactic or therapeutic agent of diseases such as sepsis, septic shock, inflammatory disease, infectious disease and the like and other diseases.

This application is based on a patent application No. 2000-379787 filed in Japan, the contents of which are hereby incorporated by reference.

## Claims

1. A pharmaceutical agent comprising an anti-sepsis drug and at least one kind of drug selected from the group consisting of an antibacterial agent, an antifungal agent, a non-steroidal antiinflammatory drug, a steroid and an anticoagulant in combination.

2. The pharmaceutical agent of claim 1, wherein the anti-sepsis drug is a non-peptidic compound.

3. The pharmaceutical agent of claim 1, wherein the anti-sepsis drug is a cycloalkene compound.

4. The pharmaceutical agent of claim 1, wherein the anti-sepsis drug is a compound represented by the formula (I): 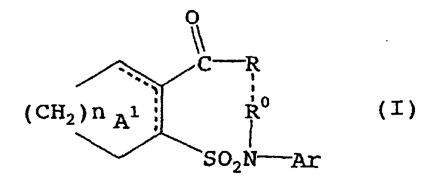 wherein
R is an aliphatic hydrocarbon group optionally having substituents, an aromatic hydrocarbon group optionally having substituents, a heterocyclic group optionally having substituents, a group represented by the formula: -OR¹ wherein R¹ is a hydrogen atom or an aliphatic hydrocarbon group optionally having substituents, or a group represented by the formula: 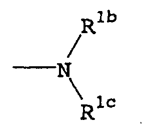 wherein
R^{1b} and R^{1c} are the same or different and each is a hydrogen atom or an aliphatic hydrocarbon group optionally having substituents,
R⁰ is a hydrogen atom or an aliphatic hydrocarbon group, or R and R⁰ in combination form a bond,
ring A¹ is a cycloalkene optionally substituted by 1 to 4 substituents selected from the group consisting of
(1) an aliphatic hydrocarbon group optionally having substituents,
(2) an aromatic hydrocarbon group optionally having substituents,
(3) a group represented by the formula: -OR¹¹ wherein R¹¹ is a hydrogen atom or an aliphatic hydrocarbon group optionally having substituents and
(4) a halogen atom,
Ar is an aromatic hydrocarbon group optionally having substituents,
a group represented by the formula: 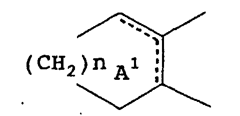 is a group represented by the formula: 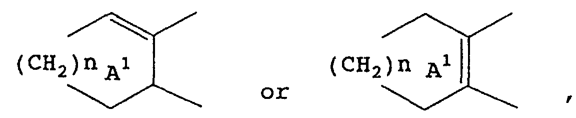 and
n is an integer of 1 to 4,
or a salt thereof or a prodrug thereof.

5. The pharmaceutical agent of claim 4, wherein, in the formula (I),
R is a group represented by the formula: -OR¹ wherein R¹ is as defined in claim 4,
R⁰ is a hydrogen atom or an aliphatic hydrocarbon group, ring A¹ is an unsubstituted cyclohexene,
Ar is an aromatic hydrocarbon group optionally having substituents, and
n is 2.

6. The pharmaceutical agent of claim 1, wherein the anti-sepsis drug is a compound represented by the formula (II): 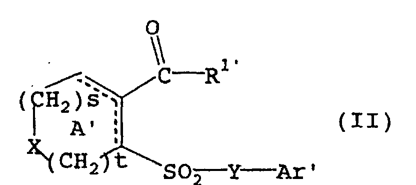 wherein R^{1'} is an aliphatic hydrocarbon group optionally having substituents, an aromatic hydrocarbon group optionally having substituents, a heterocyclic group optionally having substituents, a group represented by the formula: -OR^{1a'} wherein R^{1a'} is a hydrogen atom or an aliphatic hydrocarbon group optionally having substituents, or a group represented by the formula: 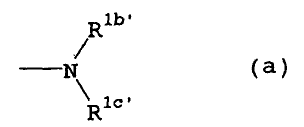 wherein R^{1b'} and R^{1c'} are the same or different and each is a hydrogen atom or an aliphatic hydrocarbon group optionally having substituents,
X is a methylene group, NH, a sulfur atom or an oxygen atom,
Y is a methylene group optionally having substituents or NH optionally having substituents,
ring A' is a 5- to 8-membered ring optionally having 1 to 4 substituents selected from the group consisting of (1) an aliphatic hydrocarbon group optionally having substituents, (2) an aromatic hydrocarbon group optionally having substituents, (3) a group represented by the formula: -OR^{2'} wherein R^{2'} is a hydrogen atom or an aliphatic hydrocarbon group optionally having substituents and (4) a halogen atom, Ar' is an aromatic hydrocarbon group optionally having substituents,
a group represented by the formula: 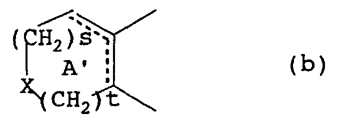 is a group represented by the formula: 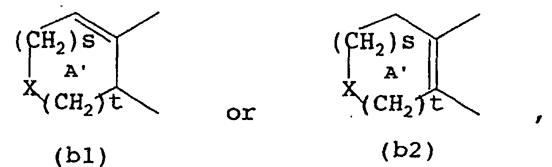 s is an integer of 0 to 2,
t is an integer of 1 to 3, and
the total of s and t is not more than 4;
provided that when X is a methylene group, Y is a methylene group optionally having substituents, or a salt thereof or a prodrug thereof.

7. The pharmaceutical agent of claim 6, wherein, in the formula (II),
R^{1'} is a group represented by the formula: -OR^{1a'} wherein R^{1a'} is C₁₋₆ alkyl,
X is a methylene group or an oxygen atom,
Y is a methylene group or NH,
Ar' is a phenyl group optionally having 1 or 2 substituents selected from the group consisting of halogen atoms and a C₁₋₆ alkoxy group,
a group represented by the formula: 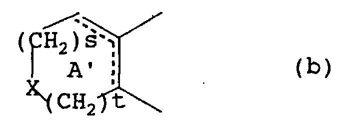 is a group represented by the formula: 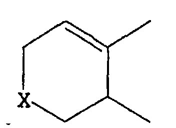 s is 1, and
t is 1.

8. The pharmaceutical agent of claim 1, comprising an anti-sepsis drug and one or more kinds of drugs selected from an antibacterial agent and an antifungal agent in combination.

9. The pharmaceutical agent of claim 1, comprising an anti-sepsis drug and one or more kinds of drugs selected from a non-steroidal antiinflammatory drug and a steroid in combination.

10. The pharmaceutical agent of claim 1, comprising an anticoagulant and an anti-sepsis drug in combination.

11. The pharmaceutical agent of claim 1 or 4, which is a prophylactic or therapeutic agent of sepsis.

12. The pharmaceutical agent of claim 1 or 4, which is a prophylactic or therapeutic agent of septic shock.

13. The pharmaceutical agent of claim 1 or 4, which is a prophylactic or therapeutic agent of an inflammatory disease or an infectious disease.

14. A method for the prophylaxis or treatment of sepsis, which comprises administration of an effective amount of an anti-sepsis drug and an effective amount of at least one kind of drug selected from an antibacterial agent, an antifungal agent, a non-steroidal antiinflammatory drug, a steroid and an anticoagulant in combination to a mammal.

15. A method for the prophylaxis or treatment of an inflammatory disease or an infectious disease, which comprises administration of an effective amount of an anti-sepsis drug and an effective amount of at least one kind of drug selected from the group consisting of an antibacterial agent, an antifungal agent, a non-steroidal antiinflammatory drug, a steroid and an anticoagulant in combination to a mammal.

16. Use of an anti-sepsis drug and at least one kind of drug selected from the group consisting of an antibacterial agent, an antifungal agent, a non-steroidal antiinflammatory drug, a steroid and an anticoagulant for the production of a prophylactic or therapeutic agent of sepsis.

17. Use of an anti-sepsis drug and at least one kind of drug selected from the group consisting of an antibacterial agent, an antifungal agent, a non-steroidal antiinflammatory drug, a steroid and an anticoagulant for the production of a prophylactic or therapeutic agent of an inflammatory disease or an infectious disease.
